# EUROPEAN PATENT APPLICATION

(11) **EP 2 033 576 A1**
(43) Date of publication of application: **11.03.2009**
(21) Application number: 07744727.4
(22) Date of filing: 05.06.2007
(51) Int. Cl.: A61B 5/151

(54) **LANCET ASSEMBLY AND PIERCING DEVICE**

(30) Priority: 13.06.2006 JP 2006163499
(71) Applicant: Izumi-Cosmo Company Limited, Osaka-shi, Osaka 530-0005 (JP)
(72) Inventor: KITAMURA, Yoritaka, Osaka-shi, Osaka 530-0005 (JP); ABE, Teruyuki, Chuo-ku, Tokyo 103-0022 (JP); SEKI, Kazuharu, Setagaya-ku, Tokyo 154-0001 (JP)
(74) Representative: Wiedemann, Peter
(86) International application number: PCT/JP2007/061375
(87) International publication number: WO 2007/145102

(57) **Abstract**

There is provided the pricking device that allows it to load the unspent lancet assembly into the injector, and that inhibits loading of the unspent lancet assembly into the injector. The pricking device has the notched portion formed on the edge of the opening end of the lancet holder, and the plate spring component is provided on the inner wall of the housing of the injector. One end of the plate spring component is secured to the injector housing, whereas the other end of plate spring component is free end, and the tip end portion of the free end being provided with the hook-shaped portion. In the spent lancet assembly, the notched portion engages with the hook-shaped portion so that the loading becomes impossible. In the unspent lancet assembly in which the lancet body is secured to the lancet holder such that the protrusion of the lancet body is located rearward with respect to the notched portion, the protrusion serves to expand the free end outwardly so that the notched portion does not engage with the hook-shaped portion. As a result, the unspent lancet assembly can be inserted into the injector.

## Description

### TECHNICAL FIELD

The present invention relates to a lancet assembly composed of a lancet and a lancet holder that houses the lancet. The present invention also relates to a pricking device composed of the lancet assembly and an injector used in combination with the lancet assembly. The pricking device is used for pricking a predetermined region of a human body with a sharp pricking component (e.g. a needle) so as to take a sample of body fluids (e.g. bloody.

### BACKGROUND OF THE INVENTION

In order to measure a blood sugar level of the patient, it is required to take a sample of the blood from the patient with diabetes. At present, various pricking devices have been used to collect a small amount of the blood sample. The pricking device is generally composed of a lancet and an injector wherein the lancet has a pricking component capable of pricking a predetermined region of the patient's body. The injector has a function of launching the lancet toward a predetermined region. The pricking device is set up for use by loading (or charging) the lancet into the injector. Due to an expansion of the compressed spring of the injector, the lancet is launched toward the predetermined region, and thereby the predetermined region is pricked.

Pricking operation by using the pricking device requires the following nine steps from the loading of the lancet into the injector until the taking out the used lancet from the injector:
(1) Removing an injector cap from the injector;
(2) Setting the lancet into the injector;
(3) Removing a lancet cap from the lancet so as to expose the tip of the pricking component;
(4) Putting the injector cap (which has been removed in step (1)) onto the injector;
(5) Cocking the injector to put the lancet in the loaded state (i.e. make the lancet ready to be launched);
(6) Applying the front end opening of the injector onto a region to be pricked, and then pressing a trigger button to launch the lancet;
(7) Removing the injector cap from the injector;
(8) Putting a protective cap on the exposed tip of the pricking component so as to shield the tip of the pricking component from its surroundings; and
(9) Discharging the lancet from the injector, and then disposing of the used lancet.

Carrying out the above nine steps sequentially is cumbersome and thus it is desired to reduce the number of the steps.

When taking a sample of the blood by using a pricking device, a particular attention must be paid to the handling of the used lancet. In the used lancet, the tip of the pricking component is exposed on the front end of a lancet body, and there may be the patient's blood adhered to the pricking component (particularly the tip of the pricking component) due to the pricking. If the body of a person other than the subject of the blood sampling (for example, a nurse or medical practitioner who collects the blood sample) accidentally should touch the tip of the pricking component, the body of such person may be pricked by the pricking component. This will result in a wound of the body through which the patient's blood may enter the body (i.e. the body of the nurse or medical practitioner), and thus posing a risk of the infection disease.

The presently-used pricking devices are not necessarily designed with due consideration with respect to the handling of the used lancet. It has been proposed to cover the exposed tip of the pricking component with a protective cap after the pricking component is used (see U.S. Patent No. 5,385,571). However, the covering the tip of the pricking component with the protective cap will in itself carry risk. That is, the lancet with the exposed tip of the pricking component has to be handled when the protective cap is placed thereon. Therefore the risk as described above is still not eliminated.

As will be understood from the above, the pricking device requires utmost attention in handling the used lancet. Due to this, there is a demand for a pricking device that allows it to be disposed of in safety after use (i.e. after the pricking operation).

### DISCLOSURE OF THE INVENTION

### PROBLEMS TO BE SOLVED BY THE INVENTION

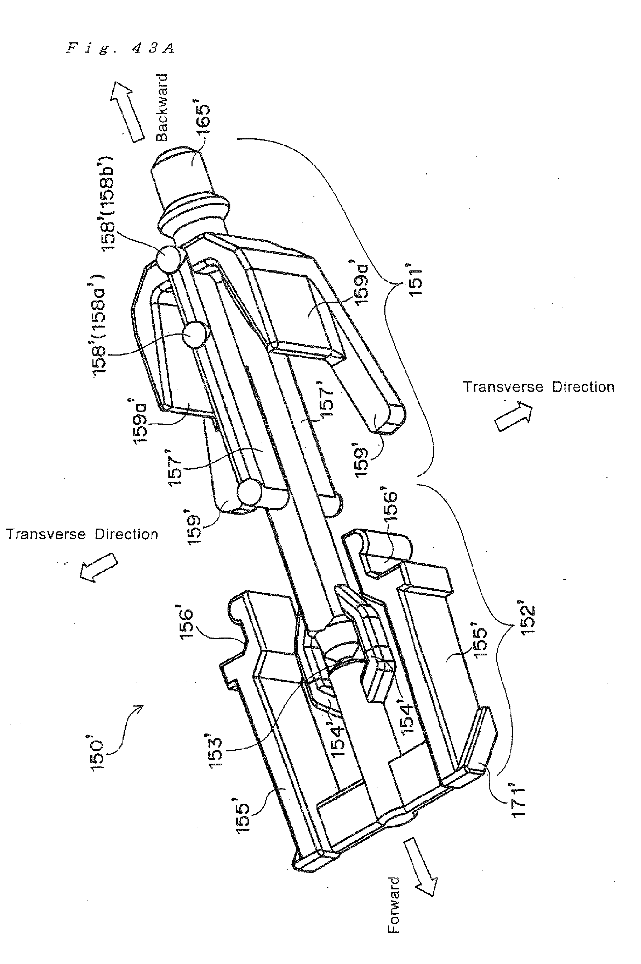

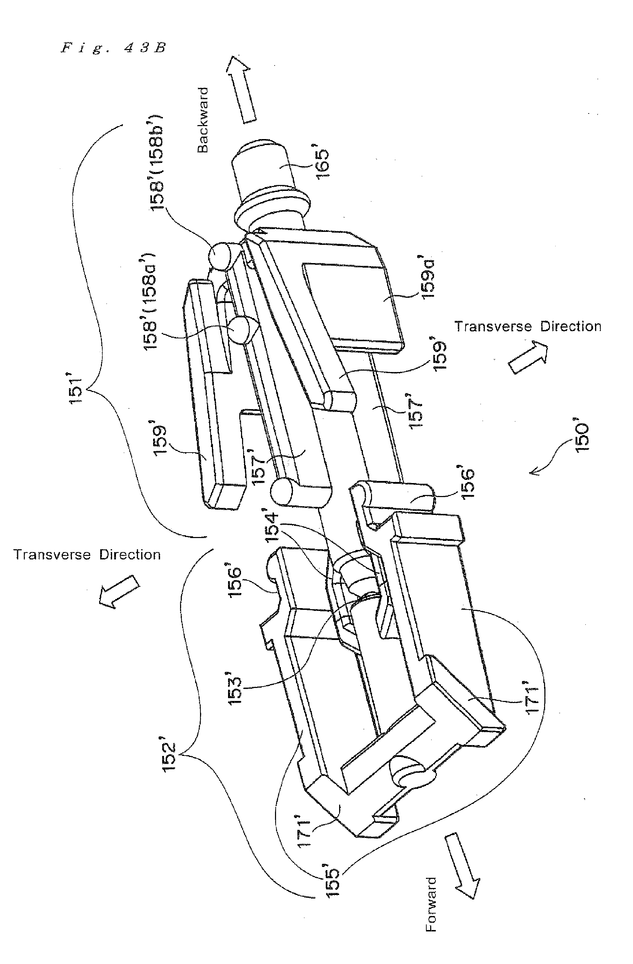

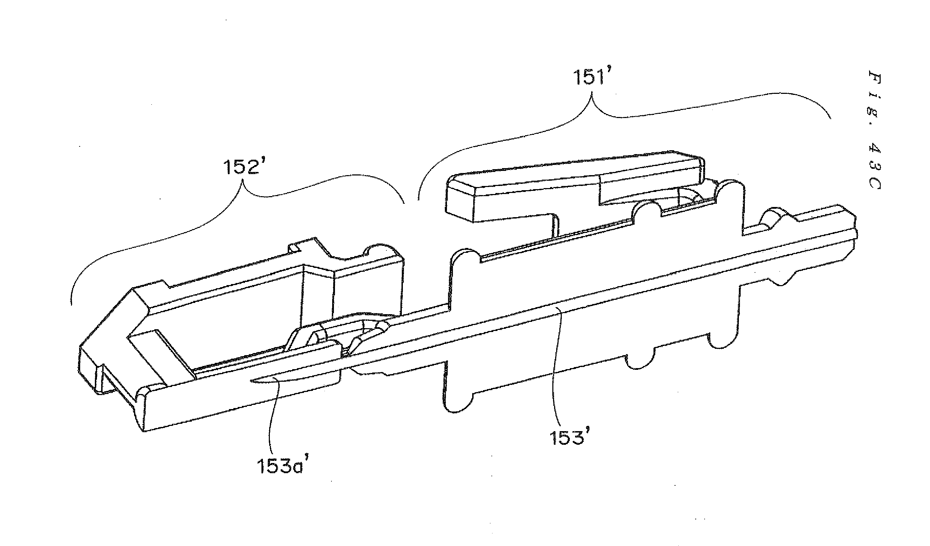

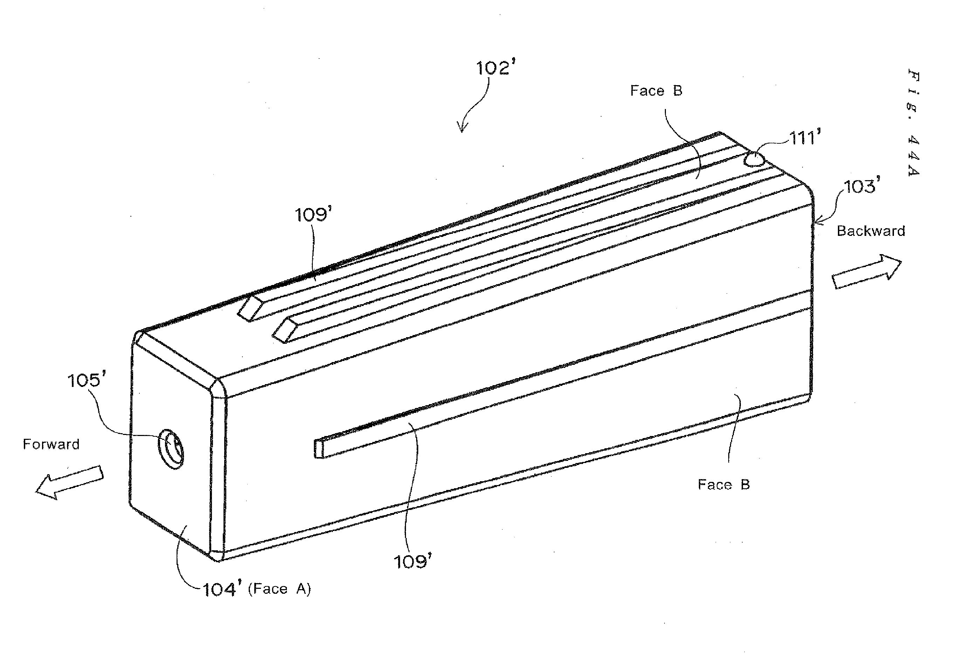

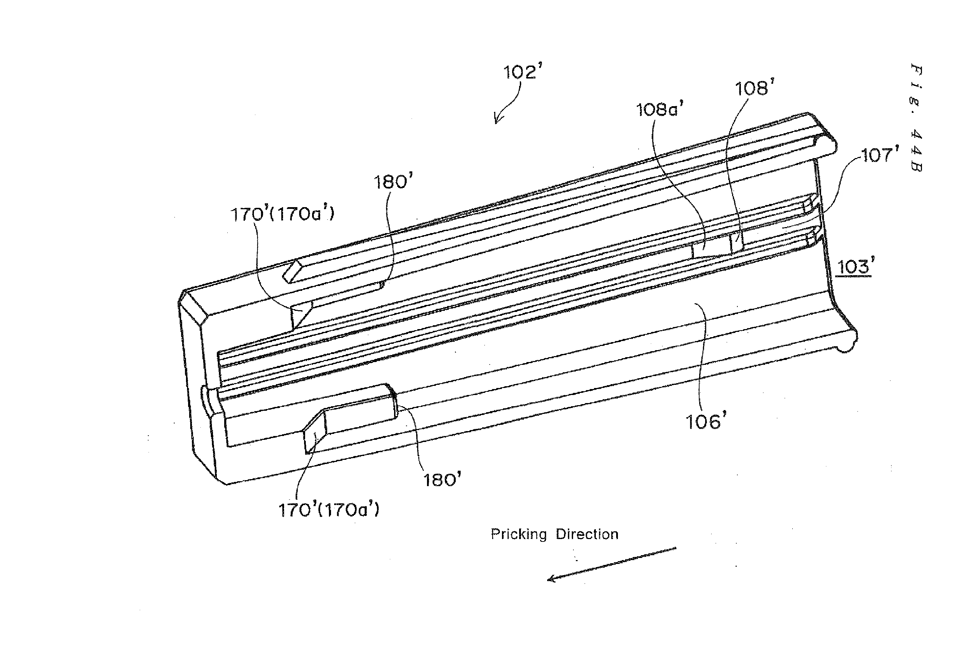

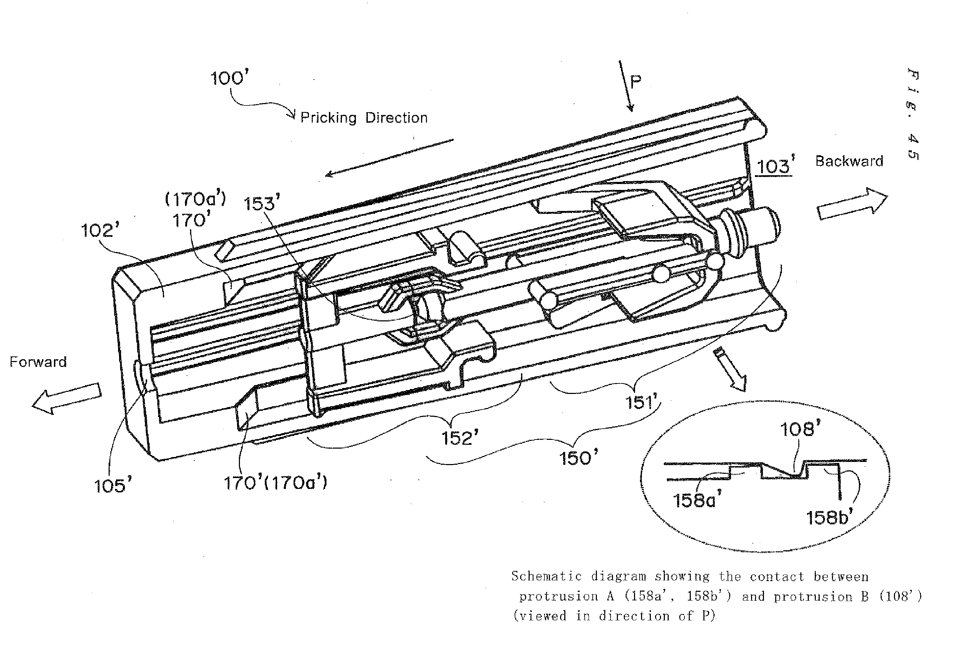

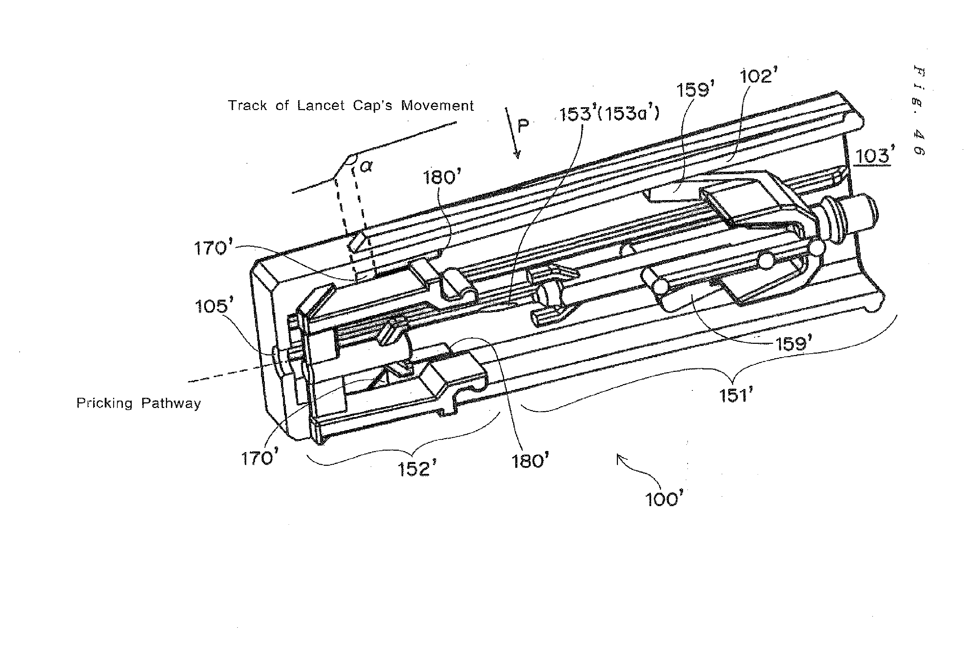

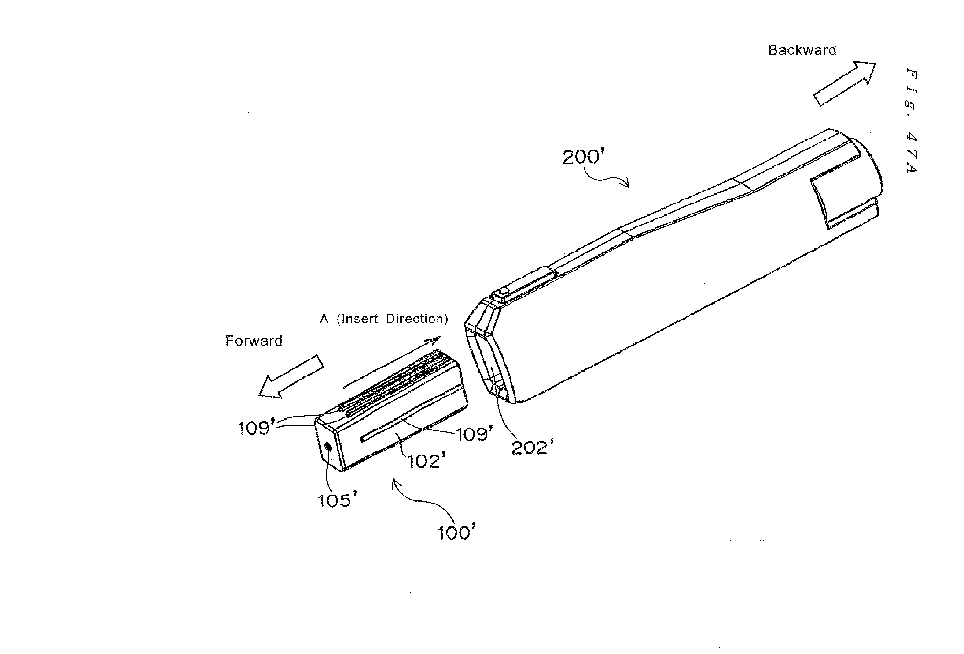

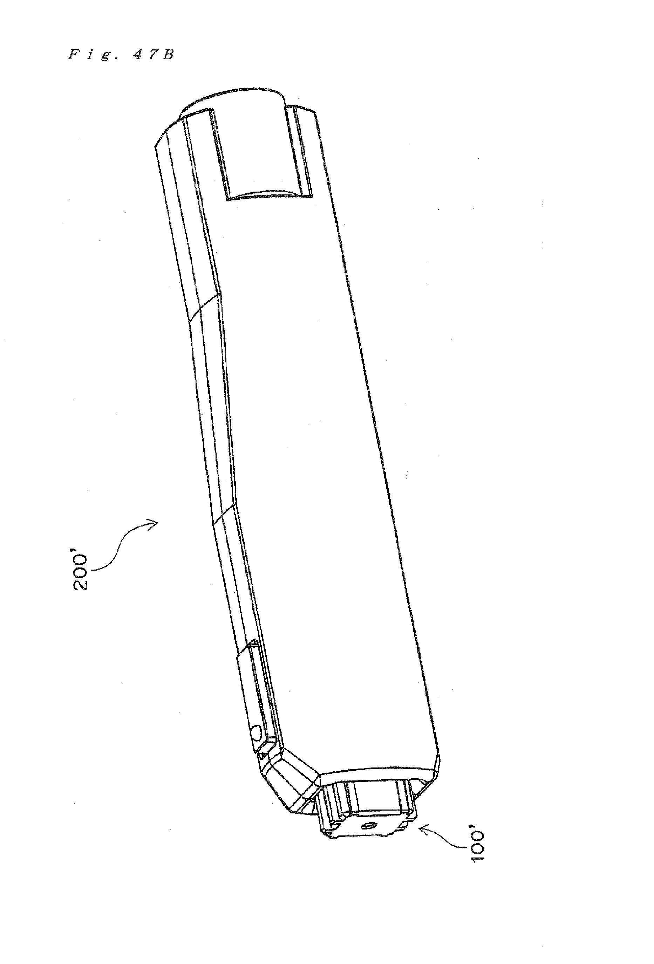

For address the problems described above, the applicant of the present invention previously filed patent applications for the invention of a pricking device (i.e. pricking device using a lancet) capable of collecting a blood sample with a reduced number of steps, and disposing of the used lancet more safely after pricking (Japanese Patent Application No. 2006-163499 filed on June 13, 2006, title of the invention "LANCET ASSEMBLY AND PRICKING DEVICE"). This lancet assembly and the pricking device will be briefly described with reference to accompanying drawings. The lancet assembly comprises a lancet 150' (see Fig. 43A to Fig. 43C) and a lancet holder 102' (see Fig. 44A and Fig. 44B) that houses the lancet. As shown in Fig. 43A to Fig. 43C, the lancet 150' comprises a lancet body 151', a lancet cap 152' and a pricking component 153' in which the lancet body 151' and the lancet cap 152' are made of resin and the pricking component 153' is made of metal, the pricking component 153' is situated in both of the lancet body 151' and the lancet cap 152', the tip of the pricking component 153' (i.e. distal end portion of the pricking component 153') is covered with the lancet cap 152', and the lancet cap 152' and the lancet body 151' are integrally connected together by a bridging component 154'. As shown in Fig. 44A and Fig. 44B, the lancet holder 102' has an opening end 103', and a pricking opening 105' in a face 104' opposed to the opening end. In the lancet assembly before use (i.e. the unspent lancet assembly), the lancet body 151' is secured to the lancet holder 102'. Specifically, the lancet holder 102' has a guide 107' formed on each of its opposing inner walls so as to guide the lancet body in the pricking direction (see Fig. 44B), whereas the lancet body 151' has a guided component 157' for slotting into the guide 107' (see Fig. 43A). The lancet body 151' is secured to the lancet holder 102' by making contact a protrusion 158' formed on the guided component 157' and a protrusion 108' formed within the guide with each other (see Fig. 44B). Particularly, as shown in a circled schematic drawing of Fig. 45, the lancet body 151' is secured to the lancet holder 102' by locating the one protrusion B 108'between the two protrusions A (158a', 158b'). When only the lancet cap 152' is pressed in the pricking direction (i.e. direction from the opening end 103' toward the pricking opening 105') while the lancet body 151' is still secured to the lancet holder 102', the bridging component 154' is broken so that the lancet cap 152' is separated from the lancet body 151', and thereby the tip of the pricking component 153' is exposed while the pricking component 153' remains situated in the lancet body 151'. When the separated lancet cap is pressed further in the pricking direction, the separated lancet cap 152' moves within the lancet holder 102' to a position that is off the pricking pathway of the pricking component 153' (see Fig. 45 and Fig. 46). Particularly, the loading of the lancet assembly into the injector 200' (see Fig. 47A and Fig. 47B) makes it possible not only for the lancet cap 152' to be separated from the lancet body 151', but also for the separated lancet cap 152' to move to the position that is off the pricking pathway.

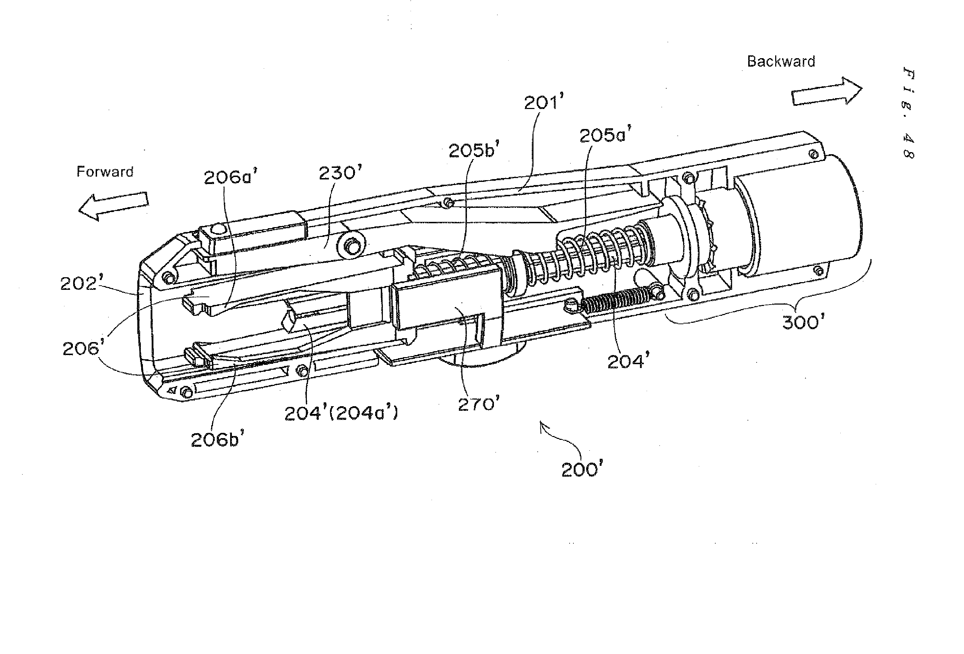

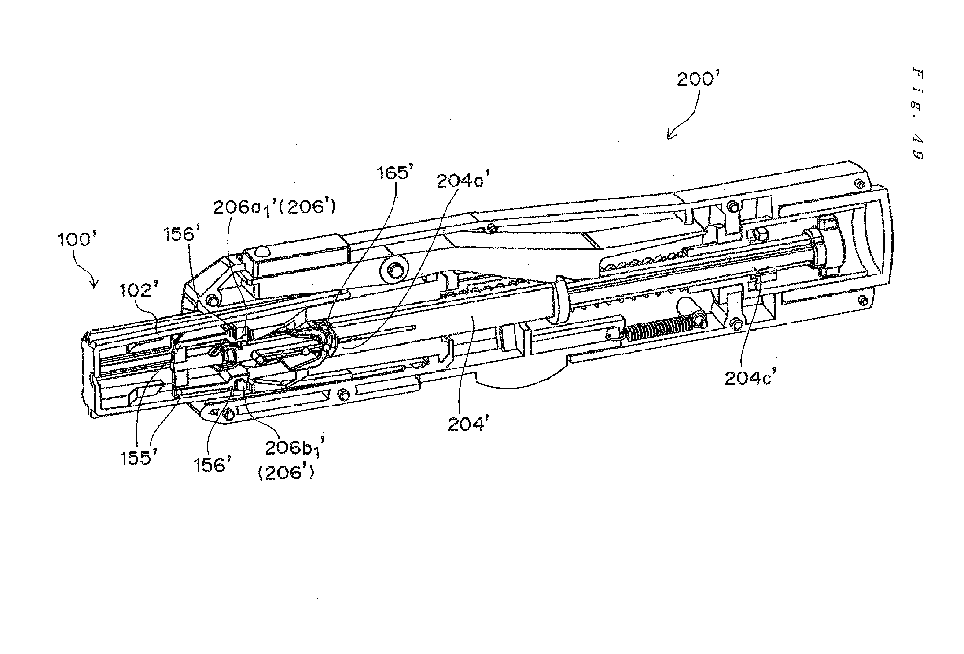

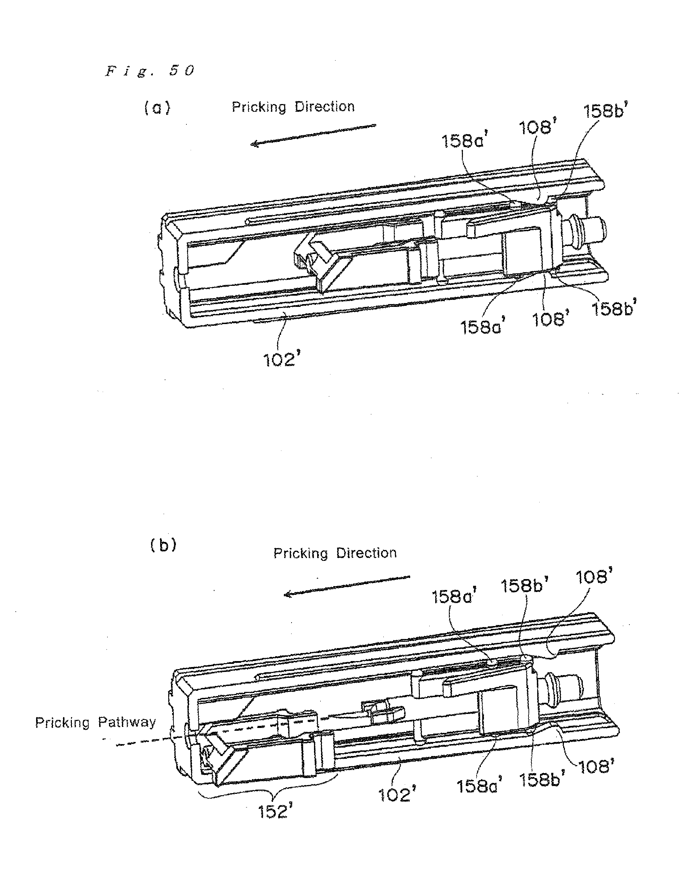

The injector 200' is a device that can be used in combination with the lancet assembly so as to launch the lancet body with the tip of the pricking component exposed. As shown in Fig. 48, the injector 200' comprises a plunger 204' and a lancet cap removing part 206'. The plunger 204' is capable of engaging with the rear end portion of the lancet body, and thus capable of launching the lancet body in the pricking direction. The lancet cap removing part 206' is capable of engaging with the lancet cap. Accordingly, as shown in Fig. 49, the lancet cap removing part 206' engages with the lancet cap when the lancet assembly is loaded into the injector 200' by inserting the lancet holder 102' into the injector through the front end opening 202' of the injector. When the lancet holder 102' to which the lancet body 151' is secured is further inserted while the lancet cap removing part 206' and the lancet cap 152' is still in engagement, the force for moving the lancet cap 152' and the lancet body 151' away from each other is generated so that the bridging component 154' interconnecting the lancet cap 152' and the lancet body 151' is broken. As a result, the lancet cap 152' is separated from the lancet body 151', and thereby the tip of the pricking component 153' is exposed while the pricking component 153' remains situated in the lancet body 151'. When the lancet holder 102' is further inserted into the injector after the breaking of the pricking component, the separated lancet cap 152' is pushed by the lancet cap removing part 206', and thereby the separated lancet cap 152' moves within the lancet holder 102' and finally reaches a position that is off the pricking pathway. The injector 200' of the present invention is configured such that the further retracting of the plunger 204' is prevented after the separation of the lancet cap 152' from the lancet body 151'. When the plunger 204' is prevented from being retracted further, the lancet body 151' being in engagement with the plunger 204' is prevented from being inserted further. Accordingly, when the lancet holder 102' is forced to move further in the direction of insertion after the prevention of the retracting of the plunger, the lancet holder 102' is forced to move in the direction of insertion whereas the force resisting it is exerted on the lancet body 151'. This causes the lancet holder 102' and the lancet body 151' to move away from each other, and thereby the lancet body 151' is no longer secured to the lancet holder 102'. More specifically, when there is generated the force causing the lancet holder 102' and the lancet body 151' to move away from each other, as shown in Fig. 50, the protrusion B 108' of lancet holder 102' finally rides over the protrusion A (i.e. 158b') of the lancet body 151' (in other words, the protrusion A 158b' of the lancet body can ride over the protrusion B 108' provided in the guide of the lancet holder). As a result, the contact between the protrusion A and the protrusion B ceases. The cease of the contact means that the lancet body 151' is no longer secured to the lancet holder 102'. Therefore, after the contact ceases, the lancet body 151' can move in the pricking direction along the guide.

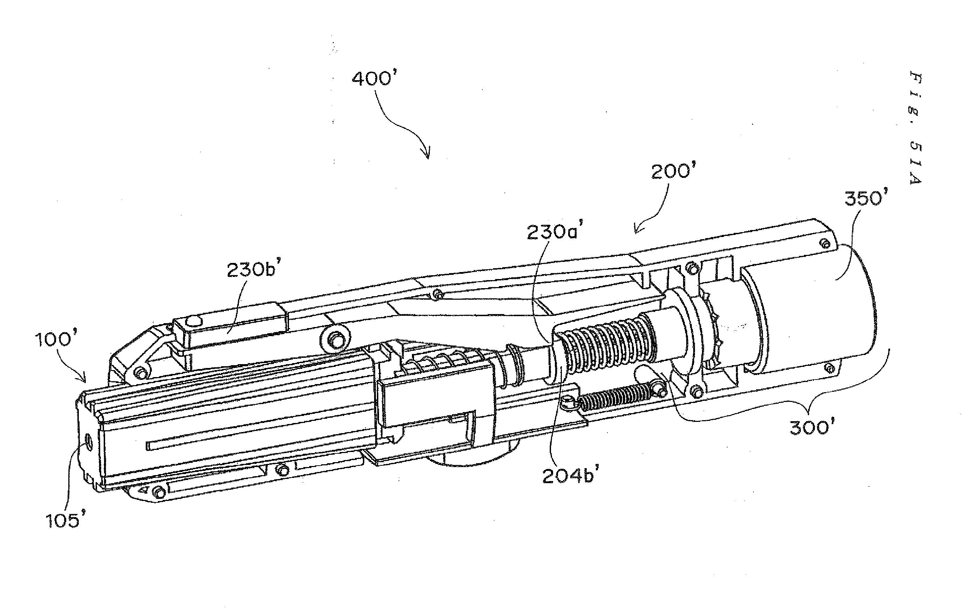

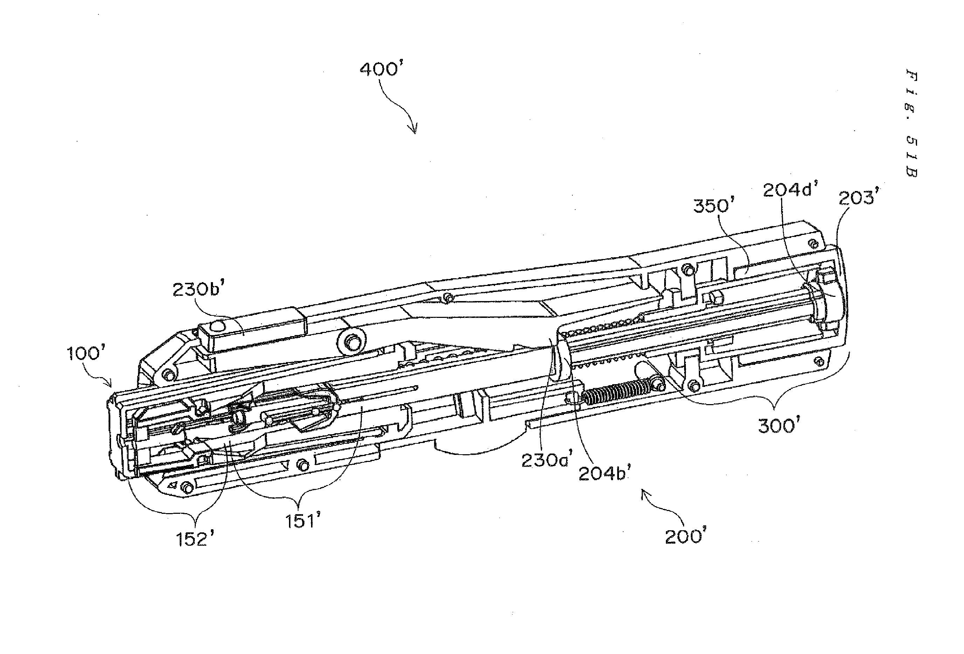

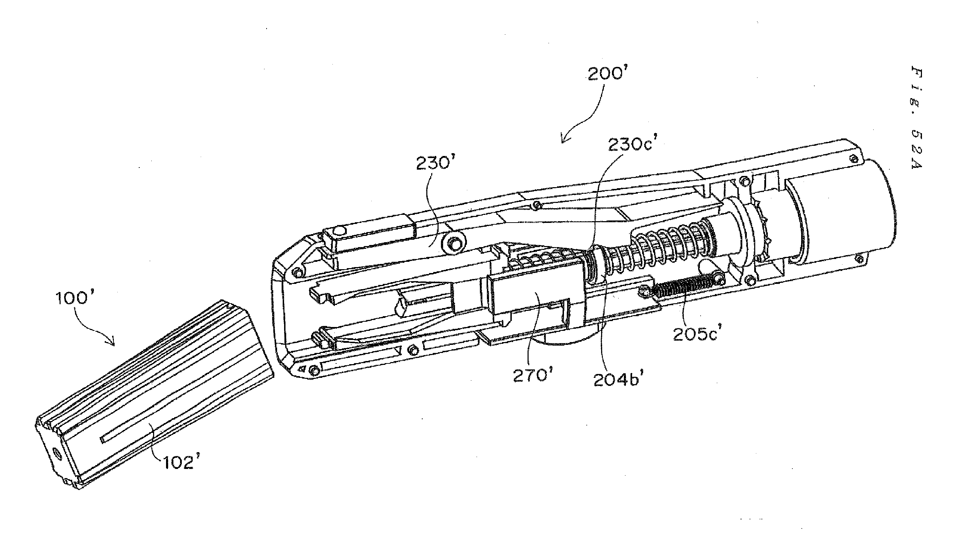

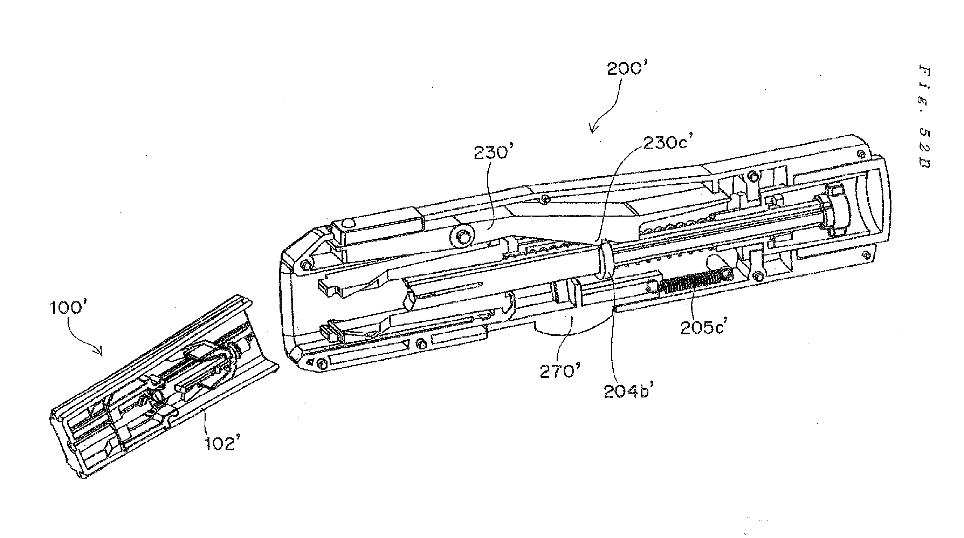

When the loading of the lancet assembly into the injector is completed, the injector is ready for pricking. The pricking is performed as follows: the pricking opening 105' is applied to a predetermined region to be pricked (for example, a finger tip). Subsequently, the front portion (indicated by reference numeral 230b' in Fig. 51A) of the trigger lever 230' is pressed toward the inside of the injector 200' so as to cease the engagement between the rear stepped portion 230a' of the trigger lever and the flange 204b' of the plunger 204'. This results in an instantaneous expansion of the compressed spring 205a', and thereby the lancet body 151' is launched in the pricking direction. Together with the lancet body 151', the pricking component 153' is launched forward, and thereafter it is retracted backward quickly. Finally, by sliding the ejector 270' forward, the spent lancet assembly 100' wherein the spent pricking component is housed in the lancet holder 102' is ejected from the injector 200' (see Fig. 52A and Fig. 52B). Specifically, the front part of the ejector 270' is pressed with a finger or the like so that the ejector slides forward. This causes the front part of the ejector 270' to press the edge of the opening end 103' of the lancet holder 102' so that the lancet holder 102' is discharged from the injector 200'. The spent lancet assembly that has been discharged from the injector is finally disposed of.

The used lancet assembly that has been discharged from the injector after pricking operation can hardly be distinguished from the lancet assembly that has not been used. That is to say, the spent lancet assembly and the unspent lancet assembly are similar in appearance. Accordingly, there is possibility that the spent lancet assembly can be accidentally loaded into the injector, and then can be used again.

There is another undesirable possibility upon the insertion of the lancet assembly into the injector. During the insertion of the lancet assembly, if an accidental force is exerted onto the lancet assembly in a direction opposite to the direction of the insertion, the lancet assembly can be come off the injector. For example, an unstable movement of the hand operating for the lancet assembly can cause the lancet assembly to be pulled, and thereby the injector can be come out of the injector. Even after the lancet assembly is inserted to a position where the lancet body can be launched, there may be occurred the accidental force exerted onto the lancet assembly in the direction opposite to the direction of the insertion, causing the lancet assembly with a ready-for-launch position to move adversely, and thereby the pricking operation is impaired.

Also there is another undesirable possibility upon the discharge of the lancet assembly from the injector by sliding the ejector forward. During the insertion of the lancet assembly, only the lancet holder is pressed, causing only the lancet holder to be discharged from the injector, and the lancet (particularly the lancet body) to remain situated in the injector.

Moreover, upon the pricking operation, the protrusion B provided in the guide of the lancet holder rides over the protrusion A of the guided portion of the lancet body as described above. During the riding of the protrusion B over the protrusion A, there is a possibility that the protrusion B and/or the protrusion A might be crushed and deformed. In the case of the crushed or deformed protrusion, the deformed protrusion A may come into frictional contact with the guide when the protrusion A moves along the guide of the lancet holder upon pricking. This results in the instability of pricking motion of the pricking component due to the sliding resistance (or contact resistance). Furthermore, in the case of the crushed or deformed protrusion, when the lancet body moves backward within the lancet holder of the lancet assembly after pricking (i.e. when the spent lancet body moves toward the opening end of the holder), there is a possibility that the lancet body (i.e. lancet body with the tip of the pricking component exposed) may come off the lancet holder due to the deformation of the protrusion A.

The present invention has been devised to address the problems described above. Therefore, an object of the present invention is to provide a pricking device capable of preventing the spent lancet assembly (i.e. the used lancet assembly that has been discharged from the injector after pricking) from being loaded or inserted again. Another object of the present invention is to provide a pricking device capable of holding the lancet assembly in place without allowing it to come off the injector, even when a force acting in the direction opposite to the direction of the insertion is exerted onto the lancet assembly upon the insertion thereof into the injector.

Further another object of the present invention is to provide a pricking device capable of preventing such a trouble that, upon the discharge of the lancet body after pricking, only the lancet holder is discharged from the injector with the lancet (particularly the lancet body) still remaining in the injector. Further another object of the present invention is to provide a lancet assembly capable of preventing such a trouble that, due to the protrusion for securing the lancet body, a pricking motion of the pricking component is hampered, or the spent lancet accidentally comes off the lancet holder.

### MEANS FOR SOLVING THE PROBLEMS

The present invention provides A pricking device comprising a lancet assembly and an injector, wherein
the lancet assembly comprising a lancet and a lancet holder that houses the lancet, wherein the lancet comprises a lancet body, a lancet cap and a pricking component in which the lancet body and the lancet cap are made of resin and the pricking component is made of metal, the pricking component is situated in both of the lancet body and the lancet cap, the tip of the pricking component is covered with the lancet cap, and the lancet cap and the lancet body are integrally connected together by a bridging component;
the injector used in combination with the lancet assembly is capable of launching the pricking component, the injector comprising a plunger that is capable of engaging with the rear end portion of the lancet body, and thus capable of launching the lancet body in the pricking direction; a lancet cap removing part that is capable of making contact with the lancet cap; and a trigger lever that is pressed to launch the pricking component, wherein the lancet assembly can be loaded into the injector by inserting the lancet holder into the injector through a front end opening of the injector;
the lancet holder has a notched portion (or cutout or incision) formed on an edge of the opening end of the lancet holder, whereas a plate spring component made of metal is provided on the inner wall of an injector housing at the front side of the injector,
the one end of the plate spring component is attached to the inner wall of the injector housing, whereas the other end of the plate spring component is a free end with a hook-shaped portion formed in the tip region thereof,
when the spent lancet assembly whose lancet body has been launched in the pricking direction is inserted through the front end opening of the injector, the notched portion of the lancet holder engages with the hook-shaped portion of the plate spring component, and thereby a further insertion of the lancet holder into the injector is prevented. The present invention is characterized in that the lancet holder and the injector are constituted such that the spent assembly that has been used for pricking cannot be again inserted into the injector.

The present invention is also characterized in that the pricking device is constituted such that the unspent lancet assembly that has not been used for pricking can be inserted into the injector, although the spent lancet assembly cannot be again inserted into the injector. Specifically, a rear portion of the lancet body is provided with a protrusion extending transversely;
when an unspent lancet assembly wherein the lancet body is secured to the lancet holder such that the protrusion of the lancet body is located rearward with respect to the notched portion of the lancet holder, is inserted through the front end opening of the injector, the protrusion of the lancet body outwardly presses and expands the free end of the plate spring component (namely, the protrusion of the lancet body is pressed in a direction opposite to the inside of the injector) so that the notched portion of the lancet holder does not engage with the hook-shaped portion of the plate spring component, which enables the unspent lancet assembly to be inserted into the injector.

In a preferred embodiment, the rear side of the protrusion of the lancet body is chamfered, in which case the surface of the chamfered rear side is capable of making contact with the tip end portion of the free end of the plate spring component, and thereby the free end of the plate spring component can expand outwardly upon being pressed. It is preferable that the main body of the plate spring component extends parallel to the inner wall surface of the injector housing, whereas the hook-shaped portion of the plate spring component extends in the direction perpendicular to the inner wall surface of the injector housing (in other words, the plate spring component is in a generally L-letter shape as a whole). It is more preferable that a pair of the plate spring components are provided on opposing inner walls of the injector housing, whereas a pair of the notched portions are provided on opposing edges of the opening end of the lancet holder. It is furthermore preferable that two hook-shaped portions are provided on opposing peripheral portions at a tip end region of the free end of the plate spring component, whereas two notched portions are formed in the edges of the opening end of the lancet holder such that the two notched portions correspond to the two hook-shaped portions.

The present invention also provides a pricking device in which the lancet assembly does not come off the injector upon the insertion of the lancet assembly into the injector. The pricking device of the present invention comprises a lancet assembly and an injector;
wherein the lancet assembly comprising a lancet and a lancet holder that houses the lancet wherein the lancet holder has a locking protrusion on its outer wall surface such that it is located adjacent to an opening end thereof, the lancet comprises a lancet body, a lancet cap and a pricking component in which the lancet body and the lancet cap are made of resin and the pricking component is made of metal, the pricking component is situated in both of the lancet body and the lancet cap, the tip of the pricking component is covered with the lancet cap, and the lancet cap and the lancet body are integrally connected together by a bridging component;
wherein the injector used in combination with the lancet assembly is capable of launching the pricking component, wherein the injector comprises a plunger that is capable of engaging with the rear end portion of the lancet body, and thus capable of launching the lancet body with the pricking component in the pricking direction; a lancet cap removing part that is capable of making contact with the lancet cap; and a trigger lever that is pressed to launch the pricking component, a holder locking component provided on the inner wall of an injector housing at the front side of the injector, and provided with a stopper A which protrudes from a surface S thereof toward the inside of the injector, wherein the lancet assembly can be loaded into the injector by inserting the lancet holder into the injector through a front end opening of the injector;
wherein a front end of the holder locking component is pivoted on the inner wall surface of the injector housing whereas a rear end of the holder locking component is a free end;
a spring is provided between the holder locking component and a side wall surface of the injector housing so that a inward force toward the inside of the injector is exerted on the holder locking component;
when the lancet assembly is inserted through the front end opening of the injector, the locking protrusion of the lancet holder slides on the holder locking component and then the locking protrusion rides over the stopper A, and thereby the locking protrusion and the stopper A can make contact with each other so that the inserted lancet holder does not come off the injector. The pricking device of the present invention is characterized in that the locking protrusion of the lancet holder and the stopper A of the holder locking component can come into contact with each other so that the lancet assembly is prevented from coming off the injector.

The present invention also provides a pricking device that is capable of holding the lancet assembly at a predetermined pricking-enabled position in the injector. In this pricking device, a stopper B protruding toward the inside of the injector is provided rearward with respect to the stopper A on the surface S of the holder locking component;
after the riding of the locking protrusion of the lancet holder over the stopper A upon the further insertion of the lancet holder (i.e. lancet assembly) into the injector, the locking protrusion rides over the stopper B so that the lancet holder moves (or the lancet holder is inserted) to a position where the lancet body is ready to be launched in the pricking direction (hereinafter referred to as "pricking-ready position"), and thereby the locking protrusion and the stopper B can make contact with each other. In case where the lancet assembly inserted in the injector is held at the predetermined pricking-ready position, the plunger of the injector is retracted to a predetermined position. This will be described in detail below. The rear end portion of the lancet body has an engagement portion that is capable of engaging with a plunger provided in the injector;
the rear end portion of the lancet body and the front end portion of the plunger engage with each other when the lancet assembly is loaded into the injector through the front end opening of the injector, and thereby the plunger is retracted so that the force required for launching the pricking component is stored in the plunger; and
while the lancet assembly is inserted until the force required for launching the pricking component is stored in the plunger, the locking protrusion of the lancet holder rides over the stopper B of the holder locking component and thereby the locking protrusion and the stopper B can make contact with each other. In a preferred embodiment, the locking protrusion of the lancet holder and the stopper A and/or the stopper B of the holder locking component have flat surfaces through which the locking protrusion respectively makes complementary contact with the stopper A and/or the stopper B upon the sliding of the locking protrusion on the stopper A and/or the stopper B.

In a preferred embodiment of the present invention, there is provided a an indicator mechanism capable of showing that the lancet assembly is held at the predetermined pricking-ready position in the injector. Specifically, the injector further comprises an indicator component in which the front end of the indicator component is pivoted on the trigger lever whereas the side of the rear end of the indicator component is provided with an indicating portion. In this case, when the lancet assembly is inserted to a position where the lancet body is ready to be launched in the pricking direction, the edge of the opening end of the lancet holder (i.e. the hem of the lancet holder) pushes a holder-contact region of the indicator component so that the indicator component swivels (i.e. rotates) around the front end thereof, causing the indicating portion to move to a position where a window is formed in the injector housing (such window hereinafter is referred to as "indicator window"). This makes it possible to determine whether or not the lancet assembly has been correctly loaded by checking whether or not the indicating portion can be seen through the indicator window.

The present invention also provides a pricking device capable of preventing an undesirable discharge where the only the lancet holder is discharged from the injector upon the discharge operation of the lancet assembly after the pricking. Specifically, the ejector presses the lancet holder and the lancet body forward when the ejector is caused to slide forward, and thereby the lancet assembly is ejected from the injector. In a particularly preferable embodiment, the ejector has a pair of pressing wing parts extending forward; each tip of the pressing wing parts makes contact with both of the edge of the opening end of the lancet holder and the rear end of the lancet body, and thereby both of the lancet holder and the lancet body are simultaneously pushed forward. It is preferable that the tips of the pair of pressing wing parts are respectively bent inwardly. In case where the tips of the pair of pressing wing parts are respectively bent inwardly, it is preferable that the plunger of the injector has a groove on its side; an edge portion of each of the pressing wing parts is guided along the groove when the ejector is caused to slide forward.

In case where the pricking device of the present invention has the holder locking component, the pricking device is preferably provided with a holder releasing mechanism. Specifically, the holder locking component further has a release part A and/or a release part B on its side; whereas the ejector further has a releasing part capable of making contact with the release part A and/or the release part B. In this case, when the ejector is caused to slide forward, the releasing part slides on the release part A and/or the release part B, causing the holder locking component to move outwardly while rotating around the front end thereof (i.e. rotate around the front end thereof). As a result, a contact-enabled state between the locking protrusion of the lancet holder and the stopper A and/or B of the holder locking component ceases. In a preferred embodiment, the release part A and the stopper A are located adjacent to each other in the holder locking component, and/or, the release part B and the stopper B are located adjacent to each other in the holder locking component.

Since the ejector of the pricking device of the present invention has the holder releasing mechanism, a forward sliding of the ejector not only presses the lancet holder and the lancet body simultaneously, but also cancels the contact-enabled state between the locking protrusion of the lancet holder and the stopper A and/or the stopper B of the holder locking component. This will lead to an achievement of a smooth discharge of the lancet assembly from the injector.

The present invention also provides a lancet assembly capable of preventing such a trouble that a pricking movement of the pricking component (pricking operation carried out by launching the lancet body with the tip of the pricking component exposed) would be hampered due to the protrusion for securing the lancet body to the lancet holder. Such lancet assembly comprises a lancet and a lancet holder that houses the lancet, wherein
the lancet comprises a lancet body, a lancet cap and a pricking component in which the lancet body and the lancet cap are made of resin and the pricking component is made of metal, the pricking component is situated in both of the lancet body and the lancet cap, the tip of the pricking component is covered with the lancet cap, and the lancet cap and the lancet body are integrally connected together by a bridging component;
when the lancet cap is pressed in the pricking direction with the lancet body attached to the lancet holder, the bridging component is broken so that the lancet cap is separated from the lancet body, and thereby the tip of the pricking component is exposed while the pricking component remains situated in the lancet body;
when the separated lancet cap is pressed further in the pricking direction, the separated lancet cap moves to a position that is off the pricking pathway of the pricking component;
wherein the lancet holder has two securing bosses formed within a securing groove formed on the inner wall thereof whereas the lancet body has a securing protrusion that slots into the securing grooves;
the lancet body is secured to the lancet holder by locating one securing protrusion between the two securing bosses;
wherein the lancet holder has a guide (preferably guide channel) on its inner wall, and the lancet body has a guided protrusion; and
when a contact between the securing bosses and the securing protrusion ceases by pressing the lancet body , the guided protrusion can move along the guide so that the lancet body is guided in the pricking direction. The present invention is characterized in that the protrusion for securing the lancet body to the lancet holder and the protrusion for guiding the lancet body within the lancet holder are provided separately. In a preferred embodiment, the securing protrusion extends in the transverse direction of the lancet body along flat surface A that includes the center axis of the lancet, and the guided protrusion extends in the transverse direction of the lancet body along flat surface B (excluding the plane A) that is parallel to the flat surface A. While the guided protrusion moves along the guide of the lancet holder when the lancet body is launched in the pricking direction, the guided protrusion may also have a function of expanding the plate spring component outwardly upon the loading of the lancet assembly. In other words, "guided protrusion" may be identical with "transversely-extending protrusion that is provided in the rear portion of the lancet body", the transversely-extending protrusion making it possible to insert the unspent lancet assembly into the injector.

Moreover, the present invention provides a lancet assembly that ensures that the used lancet does not come off the inside of the lancet holder. The lancet assembly has such a constitution that the securing boss of the lancet holder forms a flat surface facing forward. Specifically, with respect to the two securing bosses of the lancet holder, a front side of boss A located forward with respect to the other boss forms a flat surface facing forward. In other words, the front-sided face of the protrusion A is formed so as to bulge to the inside straight in the direction perpendicular to the pricking direction. Accordingly, once the lancet body is ready to be launched in the pricking direction, the securing protrusion of the lancet body can make contact with the front side of the boss A of the lancet holder, and thereby the lancet body does not come off the lancet holder. Now, *"the lancet body is ready to be launched in the pricking, direction"* will be described in more detail. After the lancet cap is separated from the lancet body upon the loading of the lancet assembly into the injector, the plunger is prevented from being retracted further, so that the lancet body being in engagement with the plunger is prevented from being further inserted. Subsequently, when the lancet holder is forced to move further in the direction of insertion, the contact between the securing boss of the lancet holder and the securing protrusion ceases, and thereby the lancet body can move in the pricking direction. Once the lancet body is ready to be launched, the securing protrusion of the lancet body can make contact with the front side of the securing boss A of the lancet holder, and thereby the lancet body does not come off the lancet holder.

In a preferred embodiment, the lancet cap is capable of making contact with a lancet cap removing part that is provided in the injector for launching the pricking component. Accordingly, the lancet cap removing part makes contact with the lancet cap when the lancet holder with the lancet body secured thereto is inserted into the front end opening of the injector (i.e. when the lancet assembly in which the lancet body is secured to the lancet holder is loaded into the injector). When the lancet holder is further inserted while the lancet cap removing part and the lancet cap are still in contact with each other, the force for pressing the lancet cap in the pricking direction is generated so that the bridging component is broken and the lancet cap is separated from the lancet body, and thereby the tip of the pricking component is exposed while the pricking component remains situated in the lancet body. When the lancet holder is furthermore inserted, the separated lancet cap moves to the position that is off the pricking pathway within the lancet holder. In the preferred embodiment, as described above, the front side face of the forward-sided boss among the two securing bosses of the lancet holder forms a flat surface that faces forward. As a result, the securing boss of the lancet body becomes capable of making contact with the front side face of the securing boss of the lancet holder, which will lead to achievement of preventing the lancet body from coming off the lancet holder.

In a further preferred embodiment, the lancet cap has an indicating part that protrudes in the transverse direction thereof, and the lancet holder has, in its wall, a slit extending in the longitudinal direction thereof. In this case, when the separated lancet cap moves to a position that is off the pricking pathway within the lancet holder, the indicating part moves along the slit. Accordingly, a position of the indicator part can be observed from the outside. In this embodiment, it is preferable that the securing protrusion of the lancet body moves along the slit of the lancet holder when the separated lancet cap moves to a position that is off the pricking pathway within the lancet holder. This will lead to an achievement of preventing the securing protrusion to make frictional contact with the inner wall of the lancet holder, and thereby the lancet body can be launched stably in the pricking direction. The lancet assembly of the present invention has such a constitution that the pair of wing parts provided on the lancet body can make contact with a stopper surface provided within the lancet holder, and thereby the tip of the pricking component is prevented from protruding from the pricking opening after the pricking (as will be described in detail later). Each of wing parts extends backward such that it gradually expands outwardly so as not to impede the pricking motion of the lancet body. It should be noted that the tip of the wing part is provided with "contact portion". This configuration of the wing parts extending backward also makes it possible to launch the lancet body stably in the pricking direction.

The pricking device of the present invention enables it to load the lancet assembly more safely into the injector. There may be such a case that the lancet assembly is loaded into the injector while the trigger lever is inadvertently kept depressed, in which case the pricking component may be accidentally launched in the pricking direction at the instant the charging is completed. In the pricking device of the present invention, however, the trigger lever locking mechanism is provided to prevent such an accidental launching from occurring. Specifically, the injector has a trigger locking component provided at the front end of the trigger lever. A part of the trigger locking component extends through an opening of the injector housing, and the trigger locking component is capable of sliding on the trigger lever forward or backward within the opening of the injector housing. The trigger locking component is provided with a spring for forcing the trigger locking component to move forward so that the trigger locking component is located at the front position of the opening of the injector housing, as long as the force is not applied to the trigger locking component. In this state, a locking part (or engaging part) provided at the bottom of the trigger locking component can make contact (or engagement) with a lock part (or engaged part) provided on the inner wall of the injector housing so that the trigger locking component cannot be pressed into the injector, and thereby the trigger lever cannot be pressed into the injector. In a case where the force is applied to the trigger locking component against the forward force, causing the trigger locking component to move backward within the opening of the injector housing, a contact-enabled state (or engagement-enable state) between the locking part (or engaging part) of the trigger locking component and the lock part (or engaged part) provided on the inner wall of the injector housing ceases, enabling the trigger locking component to be pressed into the injector, and thereby the trigger lever can be pressed into the injector.

### EFFECT OF THE INVENTION

In the pricking device of the present invention, the spent lancet assembly that has been discharged from the injector after the pricking operation cannot be loaded again in the injector. As a result, there is eliminated such an accident that the lancet assembly that has been used to take blood sample and thus poses health hazard would be used again. Also, since the injector has the holder locking mechanism, the lancet assembly is prevented from accidentally coming off the injector when the lancet assembly is loaded into the injector, thus enabling it to load the lancet assembly more safely and carry out pricking operation more safely.

Also in the pricking device of the present invention, both of the lancet holder and the lancet body are pushed and then discharged from the injector when the spent lancet assembly is discharged from the injector after the pricking operation. As a result, there is eliminated such an accident that only the lancet body is left in the injector upon the discharge of the lancet assembly. This will ensures that the lancet assembly can be discharged more safely. Particularly in accordance to the present invention, both the operation of pressing the lancet holder and the lancet body and the operation of unlocking the lancet holder and the holder locking component can be done at the same time by simply pressing the ejector to move forward. This ensures an easier and more smooth discharge of the lancet assembly.

Moreover, in the lancet assembly of the present invention, the protrusion for securing the lancet body to the lancet holder and the protrusion for guiding the lancet body within the lancet holder are provided separately. As a result, there is avoided such a problem that the securing protrusion makes frictional contact with the guide for guiding the lancet body with the pricking component exposed in the pricking direction. This ensures a more stable pricking motion of the pricking component. Moreover, in the spent lancet assembly, the securing protrusion of the lancet body can reliably make contact with the securing boss A of the lancet holder (the front-sided face of the securing protrusion A that forms a flat surface facing forward). As a result, a handling after use is improved in terms of safety without possibility of the spent lancet coming off the lancet holder.

According to the present invention, the injector has the indicator mechanism, and also the lancet assembly has the indicator mechanism. As a result, not only the user is given the visual means for identifying the stage of operation but also it is made easier to determine whether the lancet assembly has been used or not. Specifically, state of the indicator window of the injector changes when the pricking device becomes ready for pricking, thus enabling it to indirectly know whether it is ready for pricking. Also, because the injecting part provided on the lancet cap can be seen through the slit of the lancet holder, it can be indirectly known by the position of the injecting part that the lancet cap has been separated.
This means that it can be determined by the position of the indicating part whether the lancet assembly has been used or not. This will result in a reduction such a danger that the user accidentally uses the spent lancet assembly.

Based on the effects of the present invention described above, it is concluded that the pricking device and the lancet assembly of the present invention is capable of safer and more hygienic pricking operation with simpler operation.

### BRIEF DESCRIPTION OF THE DRAWINGS

Fig. 1A is a schematic perspective view of the lancet holder.
Fig. 1B is a schematic perspective view of the lancet holder cut away in half along the longitudinal direction thereof.
Fig. 1C shows longitudinal sectional views of the lancet holder (taken along lines Z1-Z1 and Z2-Z2).
Fig. 1D shows side views and end views of the lancet holder (Figs. 1D(a) to 1D(d) are side views of the lancet holder, Fig. 1D(e) is a front end view of the lancet holder, and Fig. 1D(f) is a rear end view of the lancet holder.
Fig. 2A is a schematic perspective view of the lancet.
Fig. 2B shows side views and end views of the lancet (Figs. 2B(a) to 2B(d) are side views of the lancet, Fig.2B(e) is a front end view of the lancet, Fig. 2B(f) is a rear end view of the lancet, and Fig. 2B(g) schematically shows flat surfaces A and B.
Fig. 2C is a schematic perspective view of the lancet as cut away in half along the longitudinal direction thereof.
Fig. 3 is a schematic perspective view of the lancet assembly wherein the lancet holder has been cut away in half along the longitudinal direction thereof.
Fig. 4 is a schematic perspective view of the state immediately before inserting the lancet into the lancet holder.
Fig. 5A is a schematic perspective view of the lancet assembly, showing the state before the lancet cap is separated (i.e. the state of the unspent lancet assembly).
Fig. 5B is a schematic perspective view of the lancet assembly, showing that the lancet cap has been separated.
Fig. 5C is a schematic perspective view of the lancet assembly, showing that the separated lancet cap has moved to a position that is off the pricking pathway of the pricking component.
Fig. 5D is a schematic perspective view of the lancet assembly, showing that the lancet body with the tip of the pricking component exposed has been launched.
Fig. 5E is a schematic perspective view of the lancet assembly, showing the state after pricking.
Fig. 6A is a schematic perspective view of the state immediately before the lancet assembly is loaded into the injector.
Fig. 6B is a schematic perspective view of the state where the lancet assembly has been completely loaded into the injector.
Fig. 7A is a schematic perspective view of the external appearance of the injector.
Fig. 7B shows side views and end views of the injector (Figs. 7B(a) to 7B(d) are side views of the injector, Fig. 7B(e) is a front end view of the injector, and Fig. 7B(f) is a rear end view of the injector).
Fig. 7C is a schematic perspective view of the inner structure of the injector.
Fig. 7D is a schematic perspective view of the inner structure of the injector, with a part of thereof shown in Fig. 7C cut away.
Fig. 8(a) is a schematic perspective view of the plunger.
Fig. 8(b) is a schematic perspective view of the lancet cap removing part.
Fig. 8(c) is a schematic perspective view of the trigger lever.
Fig. 8(d) is a schematic perspective view of the ejector.
Fig. 8(e) is a schematic perspective view of the holder locking component.
Fig. 8(f) is a schematic perspective view of the trigger locking component.
Fig. 9A is a schematic perspective view of a half member that constitutes the injector housing.
Fig. 9B is a schematic perspective view of a half member that constitutes the injector housing.
Fig. 10 is a schematic perspective view of the state where the ejector presses both of the lancet holder and the lancet body.
Fig. 11 is a schematic perspective view of the state where the plate spring component is provided on the injector housing.
Fig. 12(a) shows the state immediately before the spent lancet assembly is inserted into the injector.
Fig. 12(b) shows the state where the insertion of the lancet assembly is prevented.
Fig. 13(a) shows the state immediately before the unspent lancet assembly is inserted into the injector.
Fig. 13(b) shows the state where the unspent lancet assembly 100 is being inserted into the injector, showing that the plate spring component is pressed and expanded outwardly.
Fig. 13(c) shows the state where the unspent lancet assembly is further inserted from the state shown in Fig. 13(b), showing that the outwardly-expanded plate spring component is sliding on the outer wall of the lancet holder.
Fig. 14(a) is a schematic perspective view of the pressing wing parts of the ejector.
Fig. 14(b) is a schematic perspective view of the main body of the ejector.
Fig. 15A is a schematic perspective view of the indicator component.
Fig. 15B is a schematic perspective view of spring provided on the indicator component.
Fig. 16 shows a schematic perspective view of the trigger locking component (Fig. 16(a) shows the state where the trigger locking component is provided such that it can slide on the front end of the trigger lever. Fig. 16(b) shows the embodiment of Fig. 16(a) cut away in half along the longitudinal direction thereof.)
Fig. 17A is a schematic perspective view of the state before the lancet assembly is inserted into the injector.
Fig. 17B is a schematic perspective view of the state before the lancet assembly is inserted into the injector.
Fig. 18A is a schematic perspective view of the state around the time when the lancet assembly is beginning to be inserted into the injector.
Fig. 18B is a schematic perspective view of the state around the time when the lancet assembly is beginning to be inserted into the injector, showing that the lancet cap removing part has been inserted into the lancet holder.
Fig. 19A is a schematic perspective view of the state where the lancet assembly is being inserted into the injector.
Fig. 19B is a schematic perspective view of the state where the lancet assembly is being inserted into the injector, showing that the lancet cap and the lancet cap removing part are in contact with each other.
Fig. 20A is a schematic perspective view the state where the lancet assembly has been completely loaded into the injector.
Fig. 20B is a schematic perspective view the state where the lancet assembly has been completely loaded into the injector.
Fig. 21A is a schematic perspective view of the pricking process, showing the state at the point when the trigger lever is pressed to carry out the pricking.
Fig. 21B is a schematic perspective view of the pricking process, showing the state at the point when the trigger lever is pressed to carry out the pricking.
Fig. 22A is a schematic perspective view of the pricking process, showing that the tip of the pricking component is protruding from the pricking opening.
Fig. 22B is a schematic perspective view of the pricking process, showing that the tip of the pricking component is protruding from the pricking opening.
Fig. 23A is a schematic perspective view of the state after pricking.
Fig. 23B is a schematic perspective view of the state after pricking.
Fig. 24A is a schematic perspective view of the state after pricking, especially showing the state around the time when discharging of the lancet assembly from the injector is commenced by the ejector.
Fig. 24B is a schematic perspective view of the state after pricking, especially showing the state around the time when discharging of the lancet assembly from the injector is commenced by the ejector.
Fig. 25A is a schematic perspective view of the state after pricking, especially showing that the mid stage of the eject operation for discharging the lancet assembly from the injector.
Fig. 25B is a schematic perspective view of the state after pricking, especially showing the mid stage of the eject operation for discharging the lancet assembly from the injector.
Fig. 26A is a schematic perspective view of the state after pricking, especially showing the later stage of the eject operation for discharging the lancet assembly from the injector.
Fig. 26B is a schematic perspective view of the state after pricking, especially showing the later stage of the eject operation for discharging the lancet assembly from the injector.
Fig. 27A is a schematic perspective view of the state where the lancet assembly has been completely discharged from the injector.
Fig. 27B is a schematic perspective view of the state where the lancet assembly has been completely discharged from the injector.
Fig. 28 shows the state before the unspent lancet assembly is loaded into the injector.
Fig. 29 shows the early stage of the operation for loading the unspent lancet assembly, especially showing that the locking protrusion of the lancet holder slides on the holder locking component.
Fig. 30 shows the state immediately before the locking protrusion rides over the stopper A.
Fig. 31 shows the state where the guided portion of the lancet body makes contact with the tip end portion of the free end of the plate spring component.
Fig. 32 shows the state where the free end of the plate spring component is outwardly deformed by the guided portion of the lancet body.
Fig. 33 shows the state where the tip end portion of the plate spring component is beginning to slide on the outer wall of the lancet holder.
Fig. 34 shows the state immediately before the locking protrusion rides over the stopper B.
Fig. 35 shows the state where the opening end of the lancet holder makes contact with and also presses the holder contact region of the indicator component.
Fig. 36 shows the state after the pricking of the pricking device.
Fig. 37 shows the state where the ejector is beginning to slide forward, especially showing that the releasing part of the ejector is beginning to slide on the release part B.
Fig. 38 shows the state where the ejector is slid further forward from the state shown in Fig. 37.
Fig. 39 shows the state where the ejector is slid further forward from the state shown in Fig. 38, showing that the releasing part of the ejector is beginning to slide on the release A of the holder locking component.
Fig. 40 shows the state where the ejector is slid further forward from the state shown in Fig. 39, showing that the plate spring component has returned to its original shape from its outwardly-deformed shape.
Fig. 41 shows the state where the spent lancet assembly has been completely discharged from the injector.
Fig. 42 shows the state where the insertion of the spent lancet assembly into the injector is prevented.
Fig. 43A is a schematic perspective view of lancet.
Fig. 43B is a schematic perspective view of the
lancet, viewed sideways in a direction different from that of Fig. 43A.
Fig. 43C is a schematic perspective view of the lancet, cut away in half along the longitudinal direction of the lancet.
Fig. 44A is a schematic perspective view of lancet holder.
Fig. 44B is a schematic perspective view of the lancet holder, cut away in half along the longitudinal direction of the lancet holder.
Fig. 45 is a schematic perspective view of the lancet assembly, showing the state before the lancet cap is separated. The enclosure by dashed line schematically shows that protrusions A (158a', 158b') and protrusion B (108') are in contact with each other, viewed in direction P.
Fig. 46 is a schematic perspective view of the lancet assembly, showing that the separated lancet cap has moved to a position off from the pricking pathway of the pricking component.
Fig. 47A is a schematic perspective view of the state immediately before the lancet assembly is loaded into the injector.
Fig. 47B is a schematic perspective view of the state where the lancet assembly has been completely loaded into the injector.
Fig. 48 is a schematic perspective view of the inner structure of the injector.
Fig. 49 is a schematic perspective view of the state where the lancet assembly is being inserted into the injector, and the lancet cap and the lancet removing part are in engagement with each other.
Fig. 50 is a schematic perspective view showing the state of the lancet being housed in the lancet holder, viewed from above in direction P indicated in Fig. 45 or Fig. 46 wherein the upper half of the lancet holder of Fig. 45 or Fig. 46 is cut away. Fig. 50(a) shows that the lancet body is secured to the lancet holder, whereas Fig. 50(b) shows that the lancet body is no longer secured to the lancet holder.
Fig. 51A is a schematic perspective view of the state where the lancet assembly has been completely loaded into the injector.
Fig. 51B is a schematic perspective view (type A) of the state where the lancet assembly has been completely loaded into the injector.
Fig. 52A is a schematic perspective view (type A) of the state where the lancet assembly has been completely discharged from the injector.
Fig. 52B is a schematic perspective view (type A) of the state where the lancet assembly has been completely discharged from the injector.

### Reference Numerals

- 100: Lancet assembly
- 102: Lancet holder
- 102a: Outer wall of lancet holder
- 103: Opening end of lancet holder
- 104: Face which is opposed to the opening end
- 105: Pricking opening
- 106: Inner wall of lancet holder
- 107: Guide or guide channel
- 109: Continuous protrusion formed on external surface of
- lancet: holder
- 113: Notched portion
- 113a: Space region between two notched portions
- 116: Locking protrusion provided on external surface of lancet holder
- 118: Slit
- 120: Securing groove
- 121a: Front securing boss (securing boss A)
- 121a₁: Front side of front securing boss
- 121a₂: Rear side of front securing boss
- 121b: Rear securing boss
- 150: Lancet
- 151: Lancet body
- 152: Lancet cap
- 152a: Indicating part of lancet cap
- 153: Pricking component
- 153a: Tip of pricking component (i.e. distal end portion of pricking component)
- 154: Bridging component
- 155: First wing part
- 155a: Plate-shaped portion of lancet cap
- 156: Backward-protruding portion of lancet cap
- 157a: Upper guided protrusion
- 157a₁: Rear portion of upper guided protrusion
- 157b: Lower guided protrusion
- 158: Securing protrusion
- 159: Second wing part (wing for preventing the spent lancet from coming off the lancet holder)
- 159a: Contact portion provided at distal end of second wing part
- 165: Engagement portion of rear end of lancet body
- 165a: Rear end of lancet body
- 170: Slope component provided in lancet holder
- 171: Sloped portion provided in the lancet cap
- 180: Stopper surface provided in lancet holder
- 200: Injector
- 201: (201a, 201b) Injector housing
- 202: Front end opening of injector
- 204: Plunger
- 204a: Frond end of the plunger
- 204b: Flange provided on the plunger
- 204d: Rear end of the plunger
- 204f: Groove provided in the plunger
- 205a: Release spring provided on the plunger
- 205b: Return spring provided on the plunger
- 205c: Ejector spring
- 206: Lancet cap removing part
- 206a, 206b: A pair of elongated members of the lancet cap removing part
- 206a₁,: 206b₁ End portions of the lancet cap removing part
- 206c: Base portion of the lancet cap removing part
- 206d: Bow-shaped groove of the lancet cap removing part
- 206d₁: Bow-shaped groove located in background of the drawing

- 209: Partition provided in the injector housing
- 210: Inner wall of housing
- 211a, 211b: Lock part provided on inner wall of housing
- 212: Side wall of housing
- 214: Opening of housing
- 216: Window of housing
- 220: Plate spring component
- 220a: Main body of plate spring component
- 222: Fixed end of the plate spring component
- 224: Free end of the plate spring component
- 225: Hook-shaped portion of plate spring component
- 226: Opposing peripheral portions at the tip end region of the free end of the plate spring component
- 227: Tip end portion of the free end of the plate spring component
- 230: Trigger lever
- 230a: Rear stepped portion of the trigger lever
- 230c: Engagement portion (front stepped portion) of the trigger lever
- 230d: Leaf spring portion of trigger lever
- 270: Ejector
- 270a: Sliding portion or main body of the ejector
- 270a₁: A part of the sliding portion
- 272: Releasing part of the ejector
- 274: Pressing wing parts of ejector
- 275: Tip of pressing wing part
- 275a: Inside tip of pressing wing part
- 275b: Outside tip of pressing wing part
- 280: Holder locking component
- 280a: Spring used in holder locking component
- 280b: Front end portion of holder locking component
- 282: Stopper A
- 284: Stopper B
- 286: Release part A
- 288: Release part B
- 290: Trigger locking component
- 290a,: 290b Locking part of trigger locking component
- 290d: Spring used in trigger locking component
- 300: Pricking depth adjusting mechanism
- 350: Outer drum
- 400: Pricking device
- 500: Indicator component
- 500a: Holder-contact region provided on indicator component
- 500b: Front end portion of indicator component
- 500c: Rear end portion of indicator component
- 500d: Indicating portion
- 510: Spring used in indicator component
- 100': Lancet assembly
- 102': Lancet holder
- 103': Opening end of lancet holder
- 104': Face which is opposed to the opening end
- 105': Pricking opening
- 106': Inner wall of lancet holder
- 107': Guide or guide channel
- 108': Protrusion B formed in guide channel
- 108a': Forward face of protrusion B
- 109': Continuous protrusion formed on external surface of lancet holder
- 111': Semispherical protrusion provided on external surface of lancet holder
- 150': Lancet
- 151': Lancet body
- 152': Lancet cap
- 153': Pricking component
- 153a': Tip of pricking component
- 154': Bridging component
- 155': First wing part
- 156': Engagement portion of first wing portion
- 157': Guided component
- 158' (158a', 158b'): Protrusion A provided on the guided component
- 159': (159a') Second wing part
- 165': Engagement portion of rear end of the lancet body
- 170': Slope component provided in lancet holder
- 170a': Sloped surface
- 171': Sloped portion provided in the first wing part
- 180': Stopper surface provided in the lancet holder
- 200': Injector
- 201': Injector housing
- 202': Front end opening of injector
- 204': Plunger
- 204a': Front end of the plunger
- 204b': Flange provided on the plunger
- 204c': Rear end portion of the plunger
- 204d': Rear end of the plunger
- 205a': Release spring provided on the plunger
- 205b': Return spring provided on the plunger
- 205c': Ejector spring
- 206': Lancet cap removing part
- 206a', 206b': A pair of elongated members of the lancet cap removing part
- 206a₁', 206b₁': End portions of the lancet cap removing part
- 230': Trigger lever
- 230a': Rear stepped portion of the trigger lever
- 230b': Front portion of the trigger lever
- 230c': Engagement portion (front stepped portion) of the trigger lever
- 270': Ejector
- 300': Pricking depth adjusting mechanism
- 350': Outer drum

### BEST MODES FOR CARRYING OUT THE INVENTION

With reference to the accompanying drawings, the pricking device of the present invention and the lancet assembly and the injector that constitute the same will be described in detail. First, basic configurations and basic functions of the lancet assembly, the injector and the pricking device according to the present invention will be described, followed by description of the features of the present invention.

The word "forward" used herein substantially means the pricking direction that is the direction of launching the pricking component (or the lancet body with the tip of the pricking component exposed). The word "backward" or "rear" substantially means the direction opposite to the forward direction. In other words, throughout the claims, the description and the abstract, the word "forward" means the pricking direction in which the lancet moves for pricking (namely the direction in which the pricking component moves for pricking), and the word "backward" or "rear" means the direction opposite to "forward". It should be noted that "pricking direction" corresponds to a direction from the opening end of the lancet holder toward the pricking opening of the lancet holder. These directions and the transverse direction are indicated in the drawings.

Also the phrase "off the pricking pathway of the pricking component" used herein substantially implies that the separated lancet cap does not impede the forward launching of the lancet body (more specifically, the lancet body having the exposed tip of the pricking component) and the pricking of the pricking component in the pricking direction. Since the launching and the pricking are not impeded by the separated lancet cap, the pricking component can puncture the predetermined region. Preferably, the phrase "off the pricking pathway of the pricking component" implies that the lancet body does not touch the separated lancet cap at all.

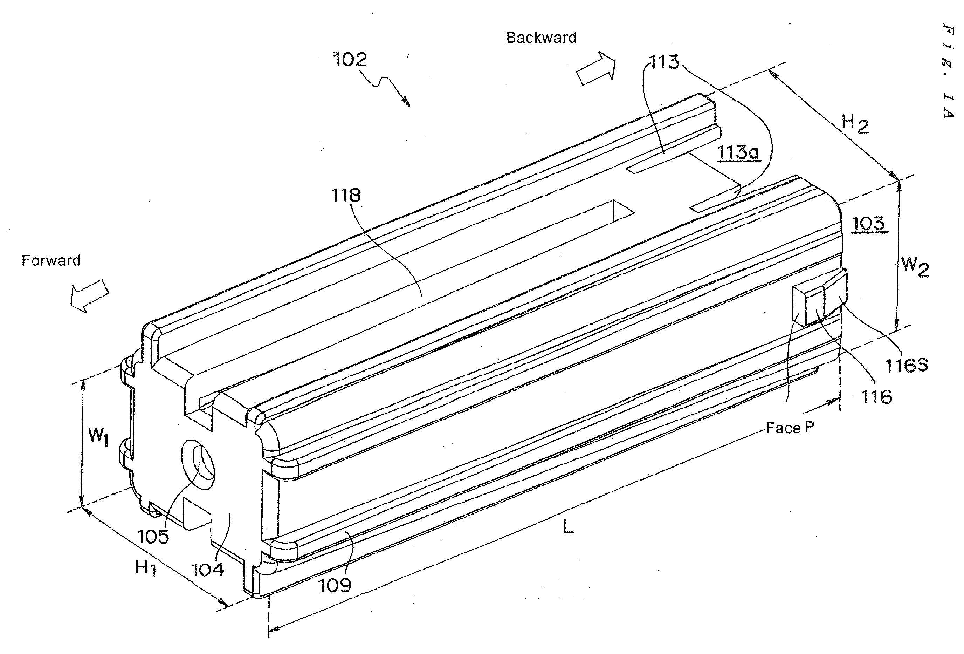

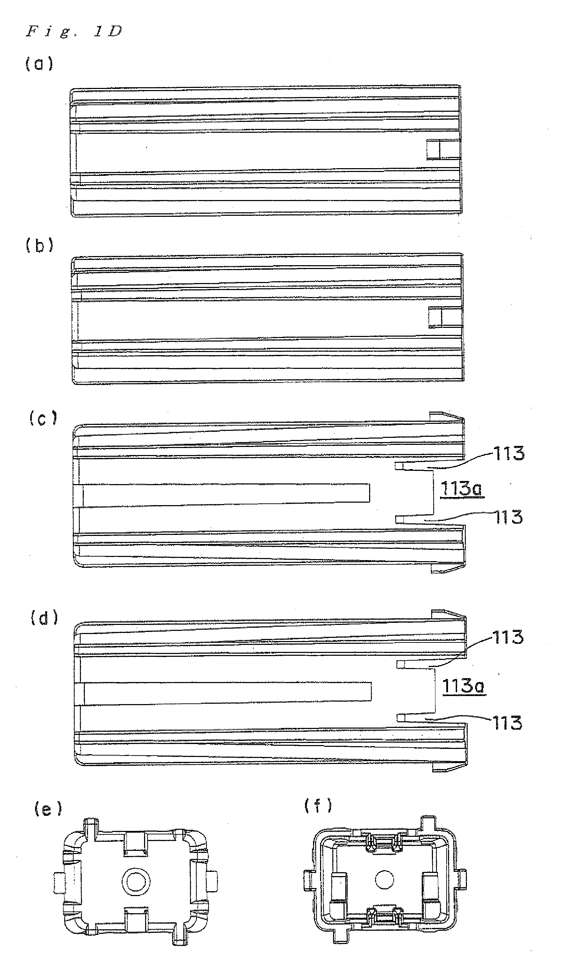

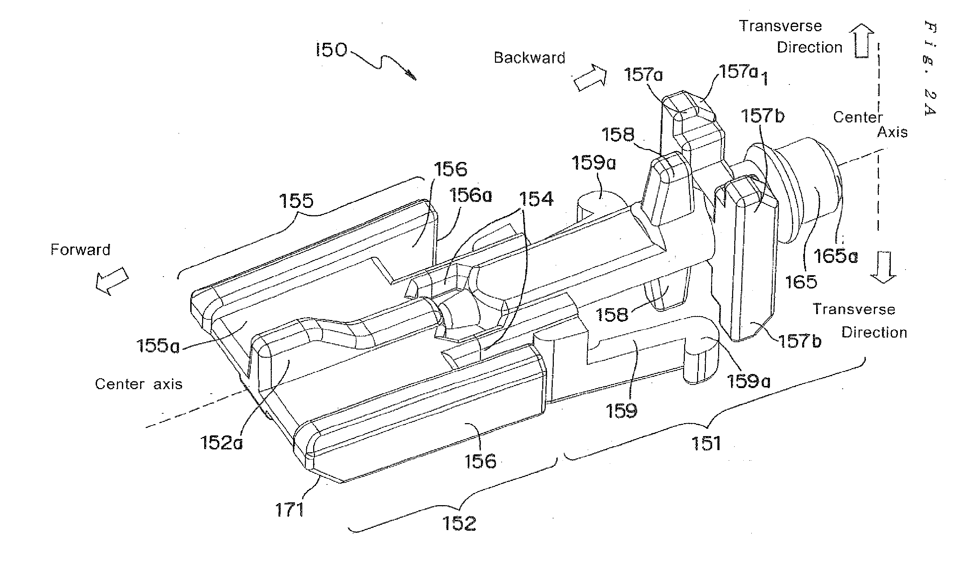

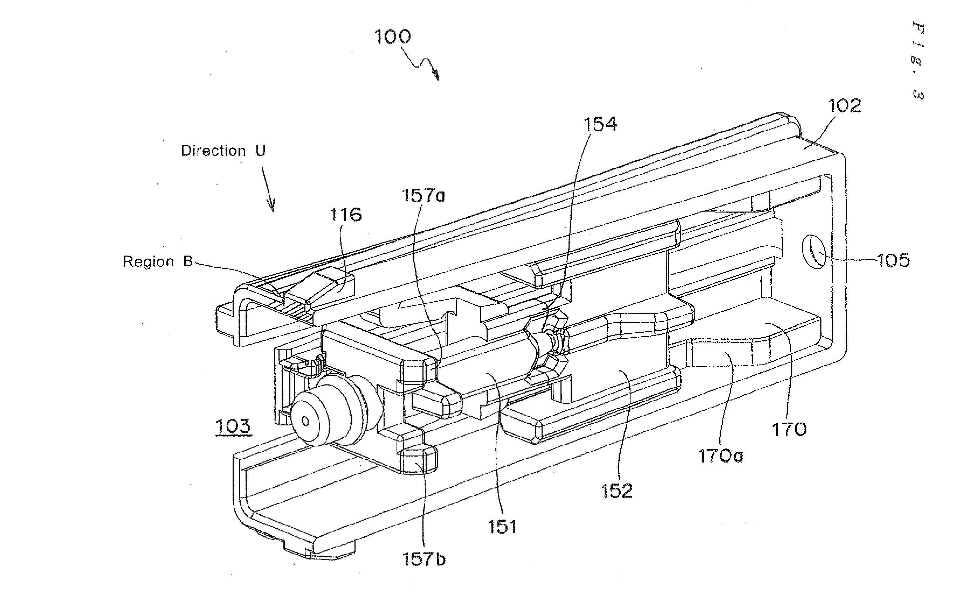

The lancet assembly according to the present invention is used in such a state that a lancet 150 shown in Fig. 2A is secured to a lancet holder 102 shown in Fig. 1A. Fig. 3 schematically shows a lancet assembly 100. It will be noted that, for the ease of understanding the structure of the lancet assembly 100, the lancet holder 102 is shown in Fig.3 with a half thereof cut away along the longitudinal direction.

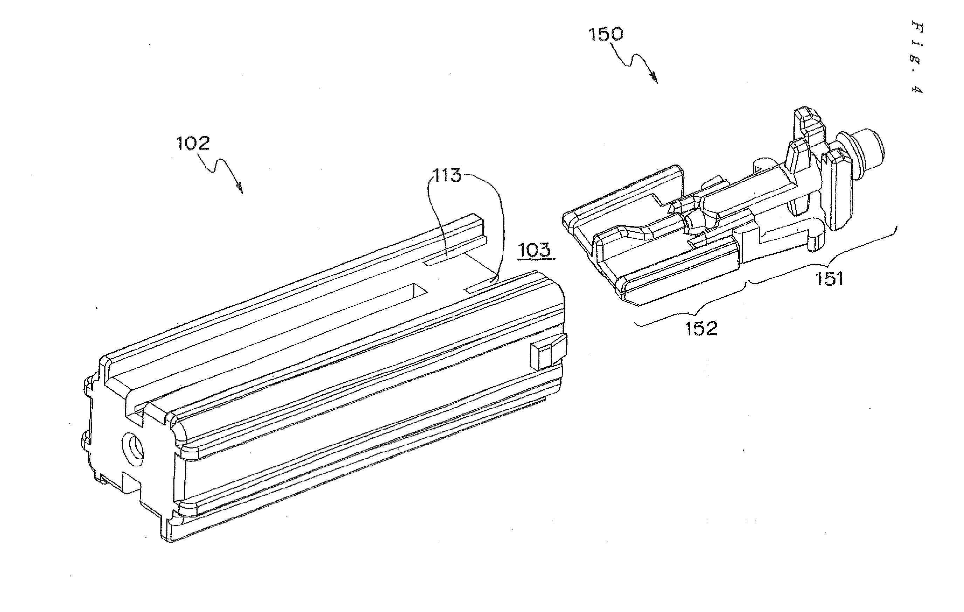

The lancet holder 102 has, as a whole, a shape of square box or square tube as shown in Fig. 1A, for example. The lancet holder 102 has small dimensions. For example, length-height-width (L, H₁, H₂, W₁, W₂) shown in Fig. 1A may be as follows: "L" is in the range from 23.4 to 31.4 mm, "H₁" is in the range from 6.9 to 10.4 mm, "H₂" is in the range from 8.6 to 12.4 mm, "W₁" is in the range from 4.2 to 7.9 mm and "W₂" is in the range from 4.7 to 8.5 mm. However, the shape of the lancet holder 102 is not limited to the square box or square tube, and may be a cylinder. As shown in the drawings, a continuous protrusion 109 is provided on the outer surface of the lancet holder 102. This continuous protrusion promotes a smooth insertion of the lancet assembly 100 into the injector 200. In addition, the continuous protrusion 109 has the function of increasing the strength of the lancet holder 102. Moreover, in case where two continuous protrusions 109 are formed substantially parallel to each other on the same surface as shown in the drawing, there is provided an advantage that the continuous protrusions 109 can serve as "foot", when the lancet holder is placed such that the continuous protrusions 109 face downward. The lancet holder 102 may be formed of any kind of resin material which is used for lancets in general. The lancet holder 102 has an opening end 103 and a pricking opening 105 provided in a face 104 opposed to "opening end 103". The pricking opening 105 is a portion applied to the region (e.g. finger) to be pricked. The lancet assembly 100 of the present invention is assembled by inserting the lancet 150 through the opening end 103 into the lancet holder 102, and then securing the lancet 150 to the lancet holder 102. Particularly, the lancet holder of the lancet assembly has notched portions 113 formed at the edge of the opening end 103 of the lancet holder 102 (namely on the holder wall at the opening end). Each of the notched portions has a slit-like configuration in the longitudinal direction of the holder. As illustrated, it is preferable that two notched portions 113 are formed in parallel to each other and the wall 113a of the holder is partially cut off, the wall 113a being located between the two notched portions 113. A locking protrusion 116 is formed on the outer wall surface that adjoins the opening end 103 of the lancet holder 102. A slit 118 is formed to extend in the longitudinal direction of the holder near the center of the side of the lancet holder. Fig. 4 schematically shows an embodiment of constituting the lancet assembly 100. As shown in this drawing, the lancet 150 is inserted through the opening end 103 into the lancet holder 102 with the lancet cap 152 at the lead.

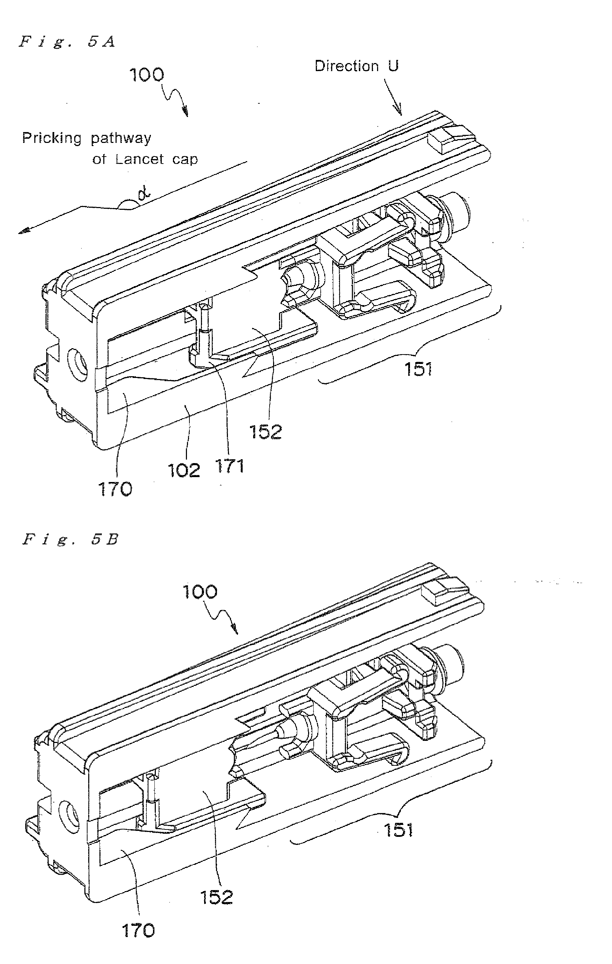

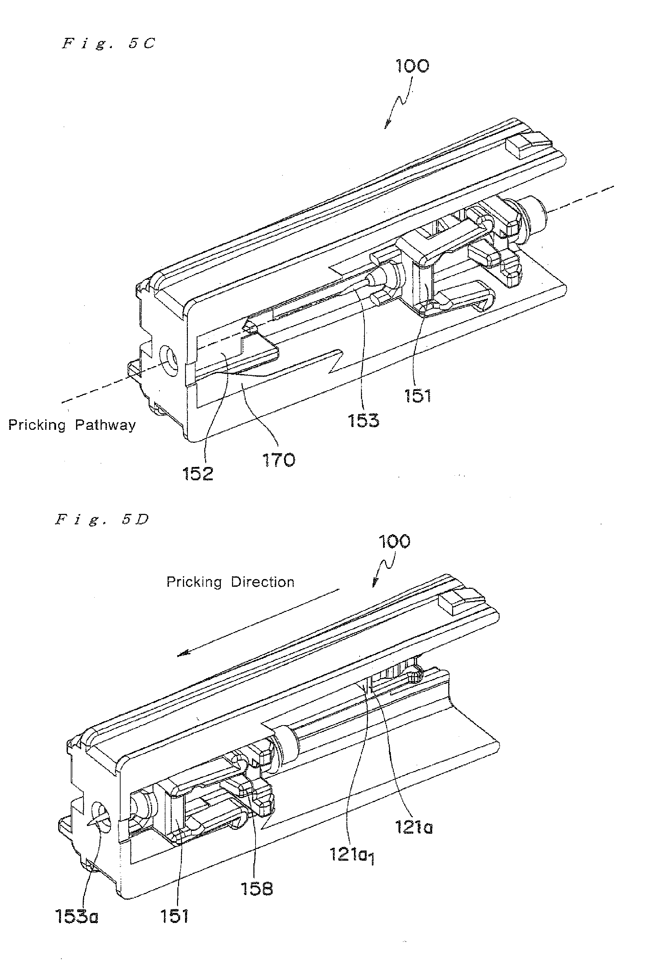

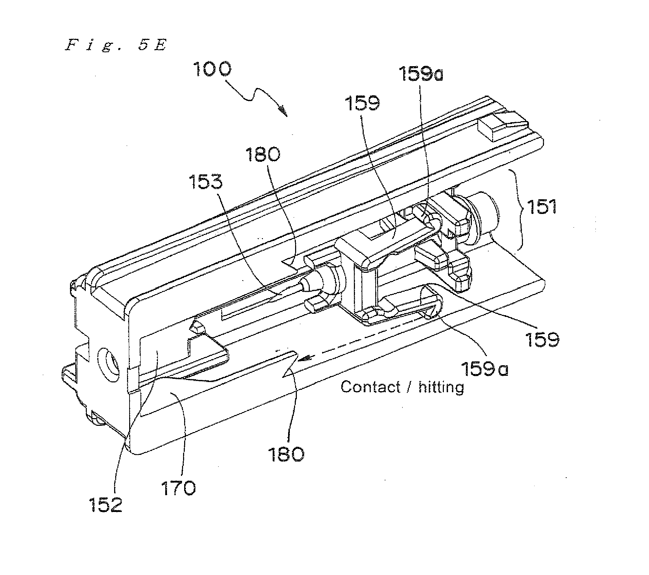

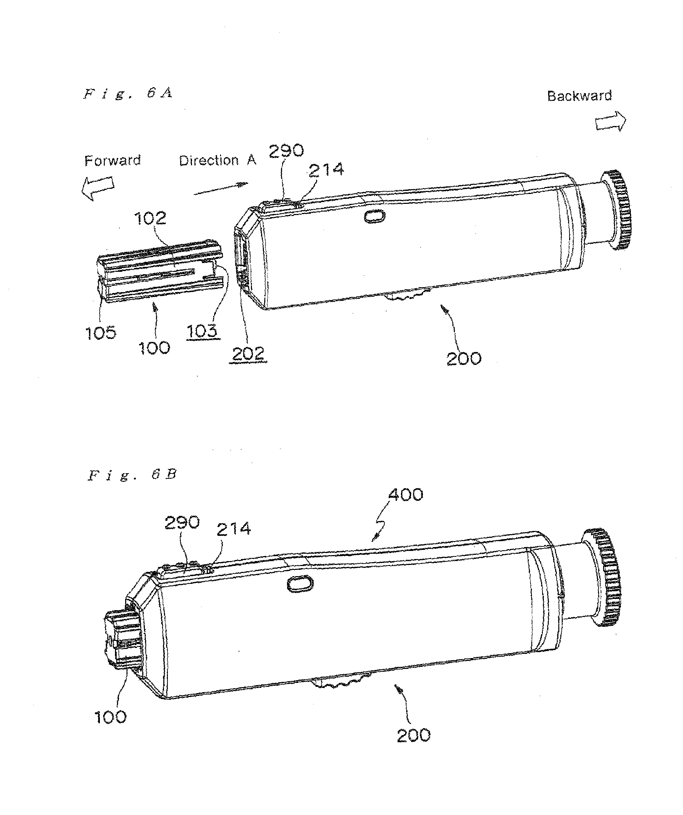

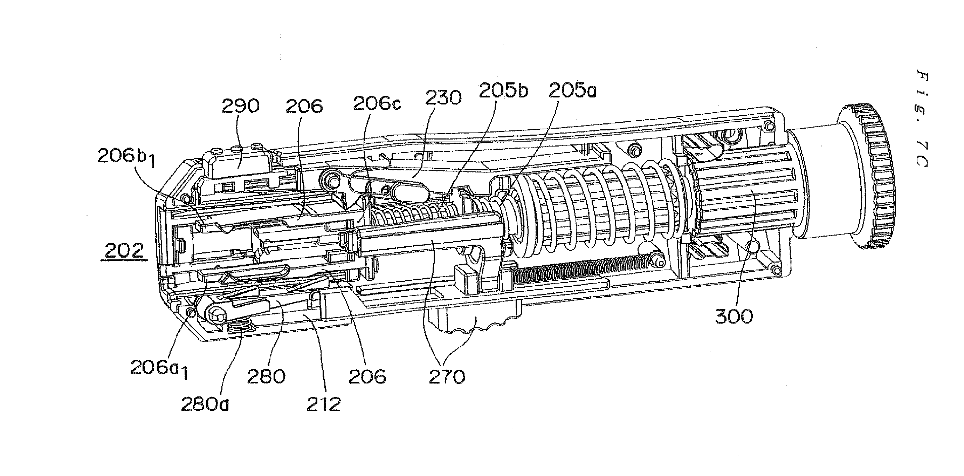

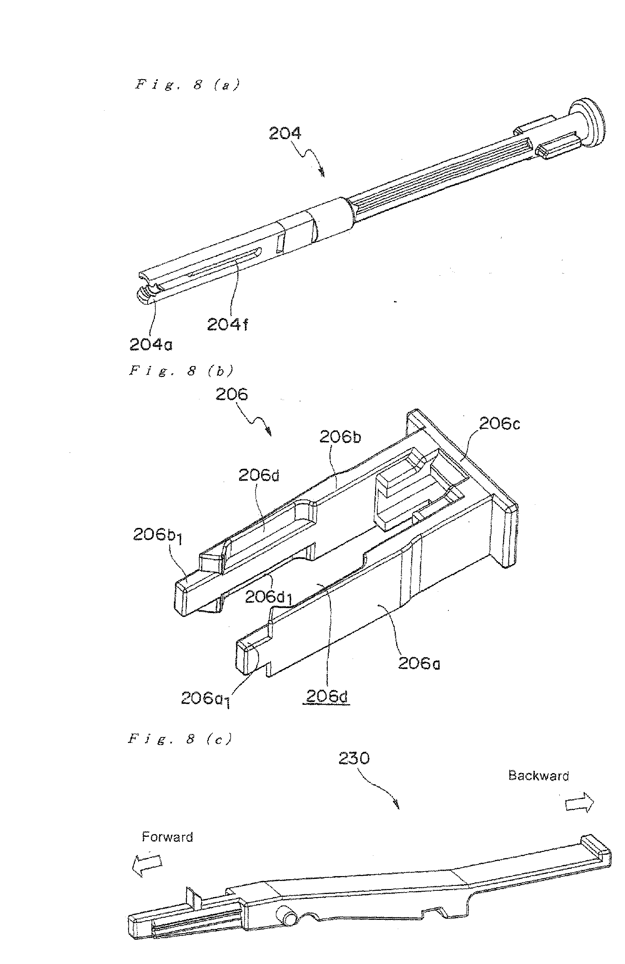

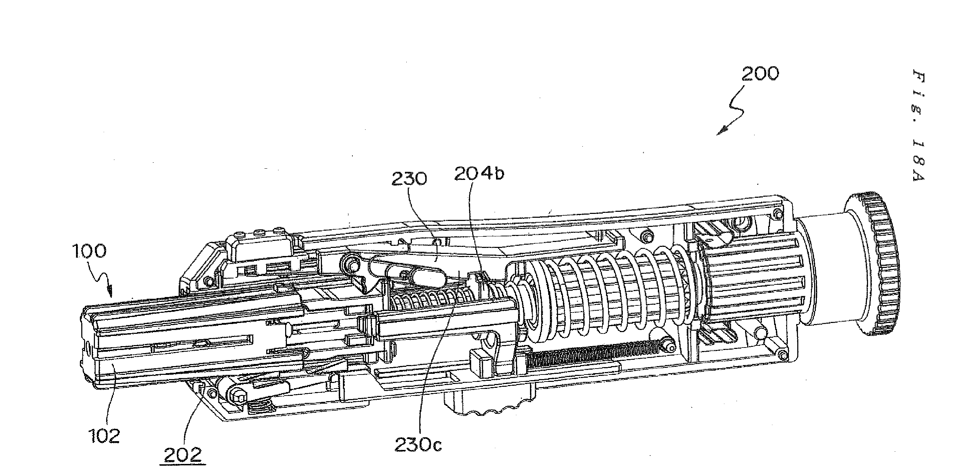

Use of the lancet assembly 100 will now be described. In use of the lancet assembly 100, a lancet cap removing part (such part is provided in an injector 200 for launching the pricking component, and denoted with reference numeral 206 in Fig. 7C and Fig. 8(b) is inserted into the lancet holder 102 through the opening end 103 of the lancet holder 102. When the lancet assembly 100 is loaded (i.e. charged) in the injector, the lancet holder 102 is inserted through the front end opening 202 of the injector 200 in such an orientation that the pricking opening 105 faces forward and the opening end 103 faces backward as shown in Fig. 6A. By inserting the lancet holder 102 into the injector 200, the lancet cap removing part 206 can enter the lancet holder 102 through the opening end 103 of the lancet holder 102 (see Fig. 18(a) for example). As will be understood from Figs. 5A to 5E showing that the state of the lancet assembly 100 varies with time, a pricking direction (in which the pricking component 153 or the lancet body 151 moves for pricking) corresponds to a direction from the opening end 103 to the pricking opening 105. Fig. 5A shows the state of the unspent lancet assembly 100 prior to pricking. Fig. 5B shows the state after the lancet cap 152 is separated from the lancet body 151. Fig. 5C shows the state where the separated lancet cap 152 has moved to the position that is off the pricking pathway. Fig. 5D shows the state in which the lancet body 151 with the tip of the pricking component 153 exposed is launched. Fig. 5E shows the lancet assembly 100 after pricking.

As previously mentioned, the pricking opening 105 of the lancet holder 102 is applied to the region (e.g. a finger tip) to be pricked upon pricking. It should be noted that the pricking opening 105 of the lancet assembly 100 according to the present invention is formed with a small size so that the pricking depth is kept constant, regardless of the strength of the force with which the lancet holder 102 is applied to the region (e.g. finger tip) to be pricked. Small size of the pricking opening 105 makes it less likely that a part of the finger to which the pricking opening 105 is applied swells into the lancet holder 102, even if the lancet holder is pressed strongly. This makes it possible to keep the depth of pricking substantially constant, virtually without the influence of the pressing force. For example, diameter of the pricking opening 105 is preferably from 0.5 to 2.0 mm, and more preferably from 1.0 to 1.5 mm. The term "diameter" is used with respect to the pricking opening having a circular shape. However, shape of the pricking opening 105 is not limited to the circle. In a case where the pricking opening has a shape other than the circle, it is preferable that the pricking opening 105 has an equivalent diameter of preferably from 0.5 to 2.0 mm, and more preferably from 1.0 to 1.5 mm. The term "equivalent diameter" used herein means the diameter of a circle having the same area as that of the intended pricking opening (in this case "area" is the area of the pricking opening in a plane perpendicular to the pricking direction).

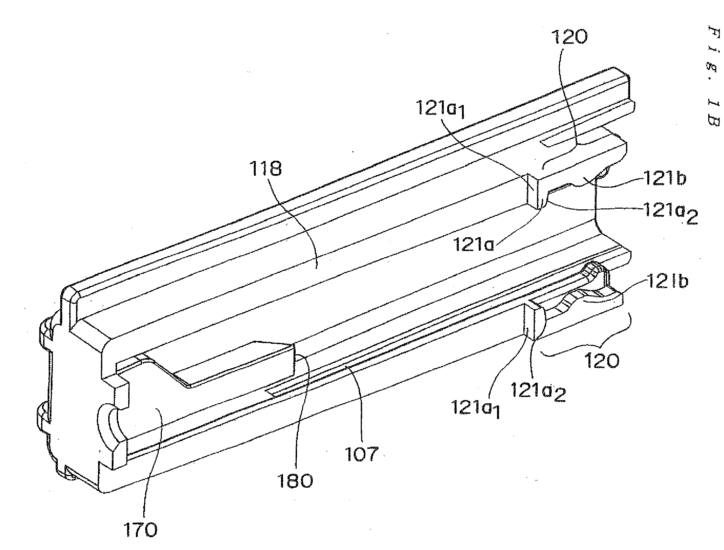

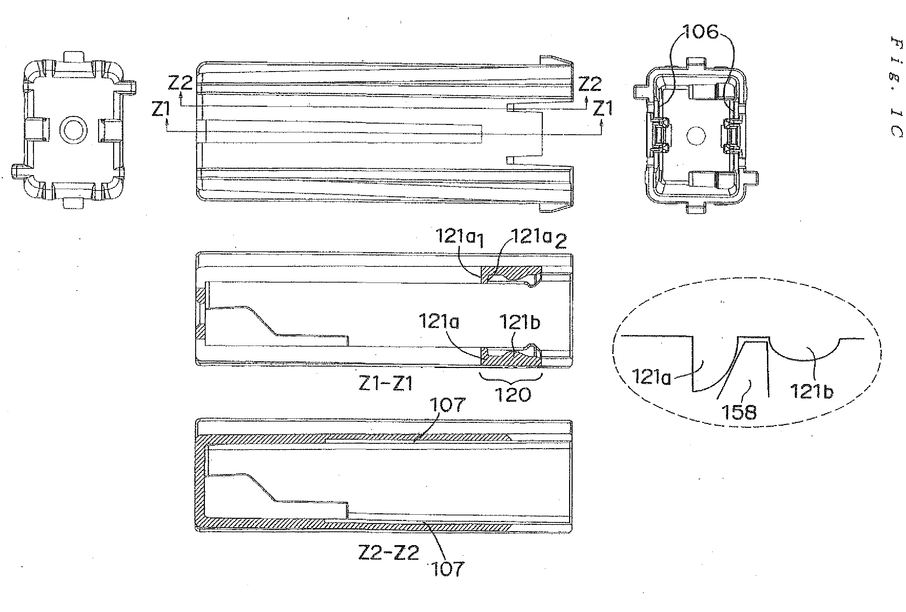

In order to help understand the internal structure of the lancet holder, Fig. 1B shows the lancet holder 102 as cut away in half along the longitudinal direction, and Fig. 1C shows the longitudinal sectional view of the lancet holder 102. The lancet holder 102 has two bosses 121a, 121b provided in the securing groove 120 on the opposing inner walls 106 thereof (specifically, the inner walls that oppose each other in a direction perpendicular to the direction in which the opening end 103 and the pricking opening 105 oppose each other). On the other hand, the lancet body 151 of the lancet 150 has a securing protrusion 158 that slots into the securing groove 120 (see Fig. 2A for the securing protrusion 158). The lancet body 151 is secured to the lancet holder 102, by bringing the securing bosses 121a, 121b and the securing protrusion 158 in contact with each other in such that the two securing bosses 121a, 121b of the lancet holder 102 sandwich the securing protrusion 158 of the lancet body 151. See the schematic diagram circled by dashed line in Fig. 1C (the diagram circled by dashed line is a view in direction U (a view from above the lancet assembly) and shows the contact between the securing bosses 121a, 121b and the securing protrusion 158). The lancet holder 102 also has guides 107, preferably two guide channels 107 for guiding the lancet body 151 of the lancet 150 in the pricking direction, on the opposing inner walls 106 thereof. On the other hand, the lancet body 151 has a pair of guided protrusions 157a and a pair of guided protrusions 157b (only one of each pair of guided protrusions is shown in Fig. 2A) that can fit into the guide channels 107 as shown in Fig. 2A. As the pair of guided portions 157a and the pair of guided portions 157b of the lancet body 151 are configured to slot into the guide channels 107 of the lancet holder 102, the lancet body 151 can move in the pricking direction upon the pricking. Thus, in the lancet assembly 100 of the present invention, the protrusion (namely the protrusion 158) for securing the lancet body 151 to the lancet holder 102 and the protrusions 157a, 157b for guiding the lancet body are provided separately from each other.

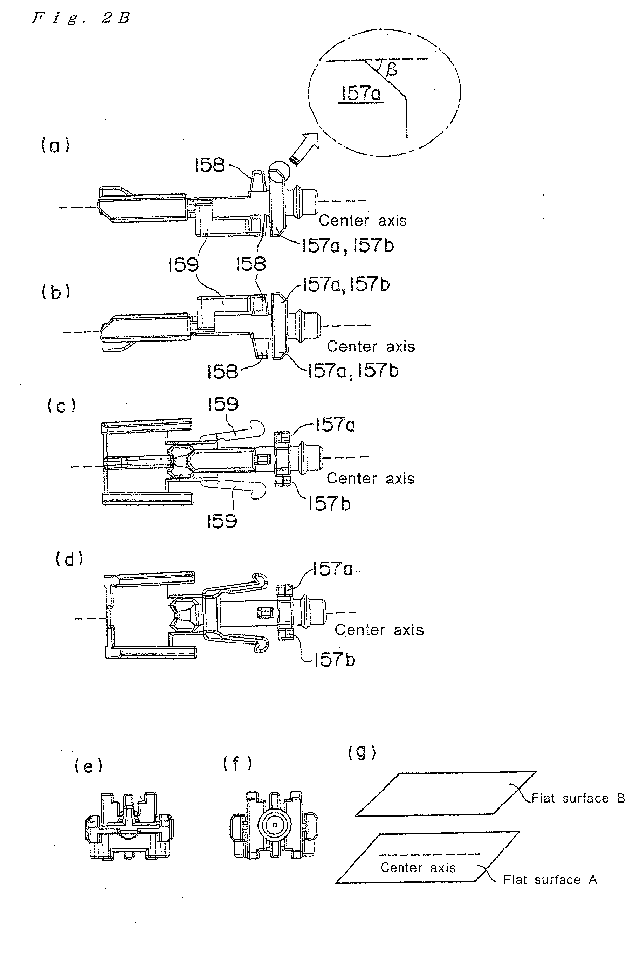

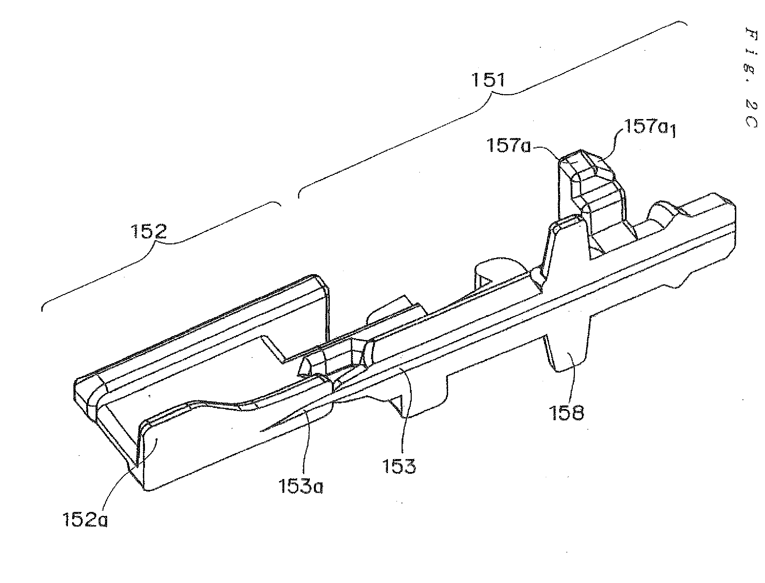

The perspective view of the lancet 150 is shown in Fig. 2A. Fig. 2B shows the lancet 150 as viewed sideways in different directions. Similarly to the lancet holder 102, the lancet 150 is also small. That is, the lancet 150 is small in size so as to be accommodated in the lancet holder 102. The lancet 150 comprises the lancet body 151, the lancet cap 152 and the pricking component 153 as shown in the drawings (see Fig. 2C for the pricking component 153). The pricking component 153 is a metal needle, for example. As shown in Fig. 2C, the pricking component 153 is situated in both of the lancet body 151 and the lancet cap 152 made of resin wherein the tip 153a of the pricking component 153 is covered with the lancet cap 152. Since the pricking component 153 is situated within the lancet body 151 and the lancet cap 152, the pricking component 153 is not shown in Fig. 2A. As shown in Fig. 2A, the lancet cap 152 and the lancet body 151 are integrally connected via the bridging component 154. The lancet 150 can be formed of resin (such as polyethylene or polypropylene) by inserting the pricking component 153 into a die, in a so-called insert molding process. In this case, the bridging component 154 can be formed upon carrying out the insert molding process. Accordingly, the bridging component 154 can be formed of the same resin as that of the lancet cap 152 and the lancet body 151. It is preferable that the bridging component has such a form or shape that it is easily broken (therefore, the bridging component may also be called "weakened portion" or "easily-broken portion"). For example, as shown in Fig.2, the bridging component 154 may be a rod-like component with small diameter. Such rod-like component is provided preferably in such a configuration that it bridges between the lancet cap 152 and the lancet body 151. It is particularly preferable that two bridging component 154 are disposed symmetrically with respect to the center axis of the lancet 150 (or the pricking component 153), as shown in Fig. 2A. The bridging component 154 may have a notch (for example, a V-shaped notch) so that the bridging component 154 can be easily broken. Moreover, the bridging component 154 may be cut off in advance. No bridging component 154 may be also provided in the lancet 150. This means that any configuration of the bridging component may be possible as long as, when the lancet cap 152 is pressed as will be described later, the lancet cap 152 and the lancet body 151 move away from each other, and thereby the tip of the pricking component 153 is exposed while the pricking component 153 remains situated in the lancet body 151.

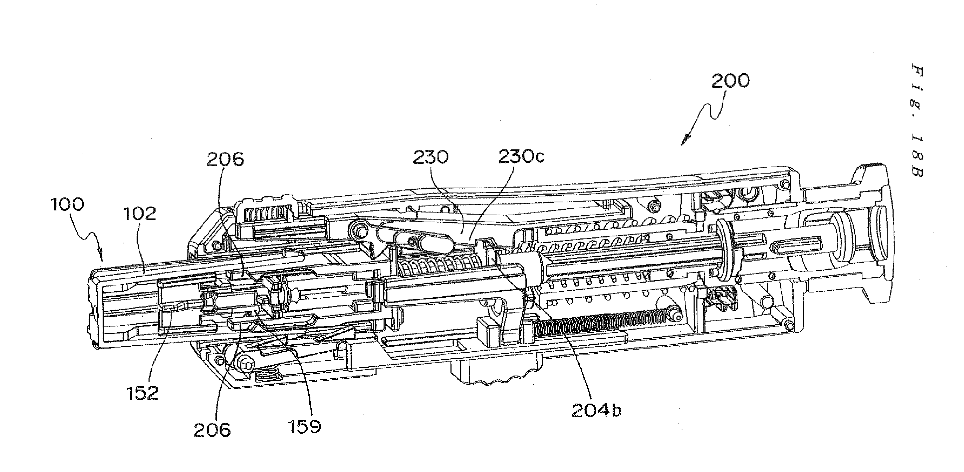

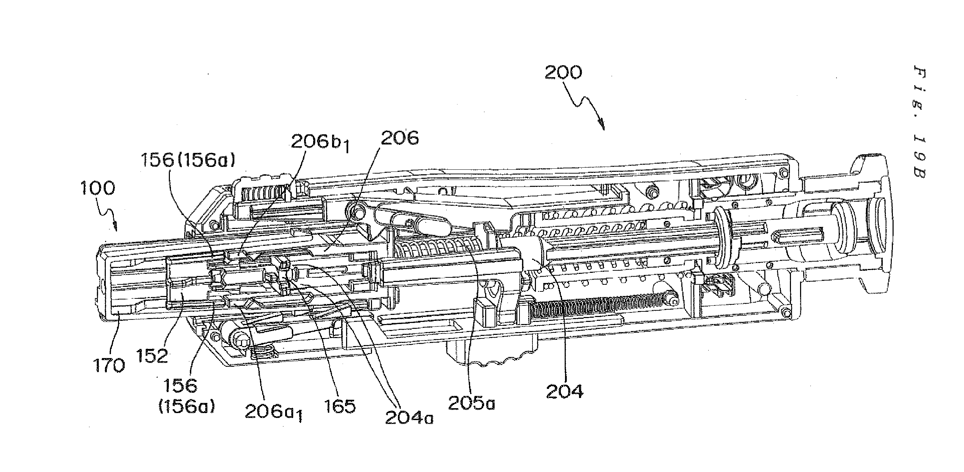

The lancet cap 152 has a pair of first wing parts 155. As shown in Fig. 2A, it is preferable that the pair of first wing parts 155 have such a configuration that side portion of plate-shaped portion 155a protrudes backward such that the plate-shaped portion 155a extends symmetrically with respect to center axis of the lancet 150 (namely with respect to the center axis in the longitudinal direction of the lancet 150, or with respect to the pricking component 153). In particular, the portion 156 that protrudes backward is preferably substantially parallel to the center axis of the lancet as illustrated. The lancet cap 152 makes it possible to engage with a lancet cap removing part 206. When the lancet cap removing part 206 enters the lancet holder 102 upon loading the lancet assembly 100 into the injector 200(see Fig. 18B), the lancet cap removing part 206 and the lancet cap 152 engage with each other. (see Fig. 19B for the state of contact between the protruding portion 156 of the lancet cap 152 and the lancet cap removing part 206).

The lancet body 151 has the pair of guided protrusions 157a and the pair of guided protrusions 157b, both of which are capable of slotting or fitting into the guide channels 107 of the lancet holder 102 as described previously. As shown in Fig. 2B(a) and 2B(b), the pair of guided protrusions 157a, 157b protrude outwardly symmetrically with respect to the center axis of the lancet. The pair of guided protrusions 157a, 157b preferably extend in a direction perpendicular to the direction in which the pair of protruding portions 156 of the first wing parts 155 oppose each other as shown in Fig. 2A. It is preferable that the pair of guided protrusions 157a and the pair of guided protrusions 157b oppose each other on both sides of the center axis of the lancet as shown in Fig. 2A and Fig.2B(c), 2B(d). The lancet body 151 is secured to the lancet holder 102 as the securing bosses 121a, 121b and the securing protrusion 158 make contact with each other such that the securing bosses 121a, 121b sandwich the securing protrusion 158. When the force acting between the lancet holder 102 and the lancet body 151 exceeds a predetermined threshold, the securing boss 121a provided at the front side of the lancet holder 102 (namely securing boss A) rides over the securing protrusion 158 of the lancet body 151 (in other words, the securing protrusion 158 rides over the securing boss 121a), which causes the cease of the contact between the securing protrusion 158 and the securing bosses 121a, 121b. As a result, the lancet body 151 can move in the pricking direction and the opposite direction thereto (in this case, the pair of guided protrusions 157a and the pair of guided protrusions 157b can respectively move along the guide channels 107). For example, when the lancet holder 102 is further inserted while the lancet body 151 is still prevented from being inserted further, there is generated the force causing the lancet holder 102 and the lancet body 151 to move away from each other, and thereby the front-side securing boss 121a of the lancet holder 102 rides over the securing protrusion 158 of the lancet body 151 (in other words, the securing protrusion 158 rides over the securing boss 121a). When this situation is viewed as the securing protrusion 158 riding over the securing boss 121a, the rear side 121a₂ of the securing boss 121a is formed in a smooth slope in order for the protrusion 158 to easily ride over the boss 121a as shown in Fig. 1B and Fig. 1C. On the other hand, the front side 121a₁ of the securing boss 121a forms a flat surface that extends straight in the lateral direction of the holder (specifically a flat surface 121a₁ that extends toward the interior of the holder in a direction perpendicular to the pricking direction), and thereby the lancet 150 does not come off the lancet holder 102 after the securing protrusion 158 has ridden over the securing boss 121a (in other words, the rear-sided face of the securing boss 121a is a curved surface whereas the front-sided face of the securing boss 121a is a flat surface). In case where the front side 121a₁ of the securing boss 121a is a flat surface, even when the used lancet body 151 moves backward within the lancet holder 102, the securing protrusion 158 engages reliably with the flat surface 121a₁, and thereby the used lancet body 151 does not come off the lancet holder 102.

As shown in Fig. 2A, Fig. 2B(c) and Fig. 2B(d), the lancet body 151 has a pair of second wing parts 159. The pair of second wing parts 159 backward expands gradually toward the outside in substantially symmetrical configuration with respect to the center axis of the lancet 150 (i.e. symmetrical with respect to the longitudinal axis of the lancet 150 or the pricking component 153). The pair of second wing parts 159 prevents the tip 153a of the pricking component 153 from protruding from the pricking opening 105 after pricking. That is to say, the pair of second wing parts 159 makes it impossible for the operator to touch the tip 153a of the pricking component 153 through the pricking opening 105 of the lancet holder 102. Specifically in the spent lancet assembly 100 that has been removed from the injector 200 after pricking, the pair of second wing parts 159 is capable of making contact with (i.e. abutting on) or hitting a stopper surface (indicated by reference numeral 180 in Fig. 1B) provided in the lancet holder 102 so as to restrict the movement of the pricking component 153 in the pricking direction (see Fig. 5E). Due to the restricted movement of the pricking component 153, the tip 153a of the pricking component 153 does not protrude from the pricking opening 105. The pair of second wing parts 159 have contact portions 159a provided at the distal end thereof. By the contact portions 159a, the pair of second wing parts 159 and the stopper 180 can reliably make contact with each other.

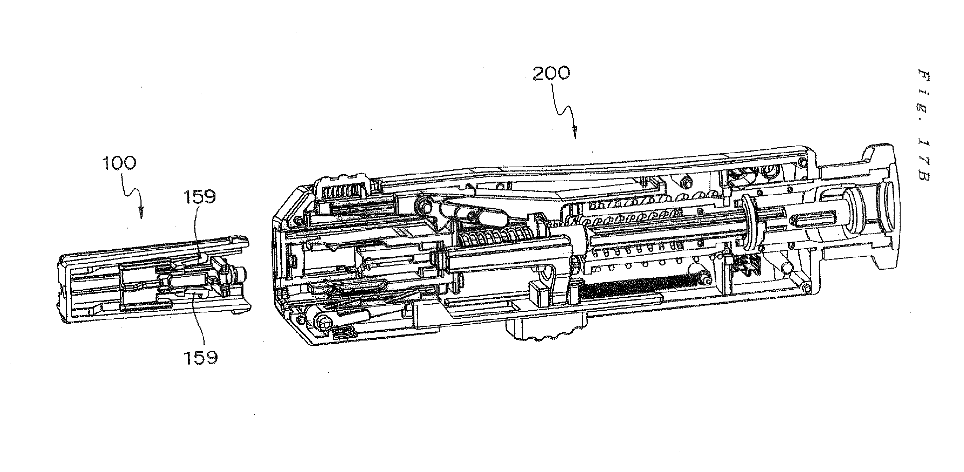

In the lancet cap removing part 206, the bow-shaped groove is provided. As the bow-shaped groove, there is provided a continuous recess or a continuous notch 206d having bow-shape as shown in Fig. 8(b). The bow-shaped groove serves to accommodate the pair of second wing parts 159. Consequently, the pair of second wing parts 159 can be placed in the bow-shaped groove 206d while preventing the second wing parts from being significantly deformed, when the lancet cap removing part 206 enters the lancet holder 102 upon loading the lancet assembly 100 into the injector 200. In other words, when the pricking component 153 is ready to be launched, the second wing parts 159 lie in the bow-shaped groove 206d without being virtually deformed, preferably with being not deformed at all (namely, there is occurred no deformation attributable to the creeping effect). Since the pair of second wing parts 159 is kept in a non-deformed state as described above, the pair of the second wing parts 159 retains its original shape after the lancet cap removing part 206 is pulled out of the lancet holder 102. This makes it possible for the pair of the second wing parts 159 to surely make contact with or hit the stopper surface 180 of the lancet holder 102. The bow-shaped groove 206d is shown on the foreground in Fig. 8(b) for the ease of understanding, although the bow-shaped groove is actually disposed on the background of the drawing (see bow-shaped groove 206d₁ in Fig. 8(b)). It will be understood that the bow-shaped groove 206d for accommodating the second wing parts 159 is actually disposed on the background of the drawing, by making reference to Fig. 17B or Fig. 18B. Fig. 17B or Fig. 18B show that the second wing parts 159 being within the lancet holder 102 are located on the background of the drawing.

Fig. 3 shows the state where the lancet 150 is housed in the lancet holder 102 and the lancet 150 is secured to the lancet holder 102 (for the ease of understanding, the lancet holder 102 is shown with one half thereof being cut away along the longitudinal direction). The lancet cap 152 is positioned on the side of the pricking opening 105 of the lancet holder 102, whereas the lancet body 151 is positioned on the side of the opening end 103 of the lancet holder 102. As described previously, the securing bosses 121a, 121b and the securing protrusion 158 are in contact with each other such that the two securing bosses 121a, 121b sandwich the securing protrusion 158 of the lancet body 151 (see the diagram circled by dashed line in Fig. 1C). That is to say, the lancet body 151 is secured to the lancet holder 102, while on the other hand the lancet cap 152 is not secured to the lancet holder 102. As a result, pressing only the lancet cap 152 in the pricking direction generates the force for departing the lancet cap 152 and the lancet body 151 from each other.
Due to this force, the bridging component 154 is broken so that the lancet cap 152 and the lancet body 151 are separated from each other and the tip of the pricking component 153 is exposed (see Fig. 5B).

Subsequently, when the separated lancet cap 152 is pressed in the pricking direction, the cap 152 moves forward. In the inside of the lancet holder 102 and near the pricking opening 105 thereof, there is provided a slope component 170 for guiding the separated lancet cap 152 to a position that is off the pricking pathway. While on the other hand, the lancet cap 152 is provided with a sloped portion 171 having a shape corresponding to the slope component 170. Specifically, the slope component 170 and the sloped portion 171 respectively have flat surfaces that allow them to make complementary contact with each other (i.e. abut against each other). The slope component 170 is clearly shown not only in Fig. 3 but also in Figs. 5A to 5C. The sloped portion 171 is clearly shown in Fig. 2A and Fig. 5A. Due to the slope component 170 and the sloped portion 171, when the separated lancet cap 152 is forced to move further forward, the sloped portion 171 of the lancet cap 152 moves while abutting on the slope component 170 of the lancet holder 102 (in other words, the sloped portion 171 slides on the slope component 170), thus allowing the lancet cap 152 to move to the position that is off the pricking pathway. Figs. 5D and 5E show the state where the separated lancet cap 152 has deviated from the pricking pathway.

The movement of the lancet cap 152 will now be described in more detail. The slope component 170 of the lancet holder 102 is provided with a sloped surface 170a (see Fig. 3) disposed at an angle to the pricking pathway. Whereas and the sloped portion 171 of the lancet cap 152 has a surface capable of making complementary contact with the sloped surface 170a. Therefore, when the separated lancet cap 152 is pushed in the pricking direction, the lancet cap 152 slides forward while surface 170a of the lancet holder and the surface of the lancet cap are in contact with each other, which results in a forward and oblique movement of the lancet cap. For example, Fig. 5A and Fig. 5C show the states before and after the movement of the separated lancet cap 152. As the lance cap 152 moves from the position shown in Fig. 5A to the position shown in Fig. 5C, it will be understood that the lancet cap 152 moves to the position that is off the pricking pathway. Track or route of the lancet cap's movement is shown in the upper portion of Fig. 5A wherein the track or route is viewed in the direction of U (i.e. from above). This track or route of the movement also indicates that the lancet cap 152 eventually moves and deviates from the pricking pathway of the pricking component 153. The angle of the sloped surface 170a with respect to the pricking pathway (namely, angle α of the track or route of the movement as shown in Fig. 5A) is preferably in the range from 30° to 60°, more preferably in the range from 40° to 50°.

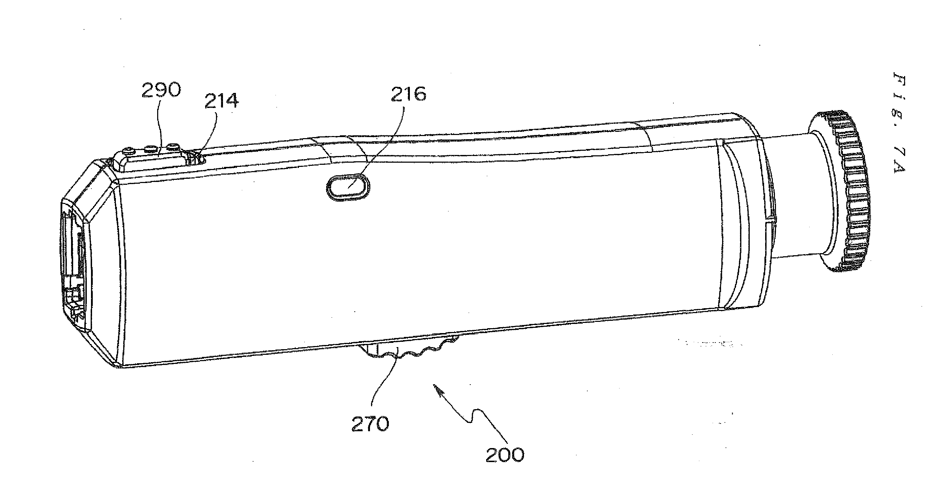

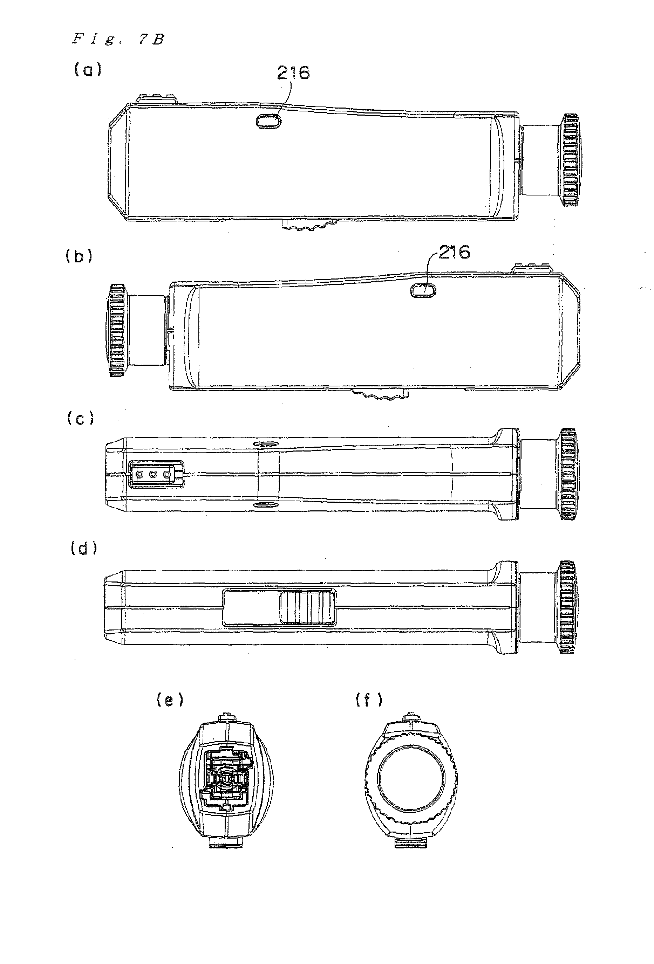

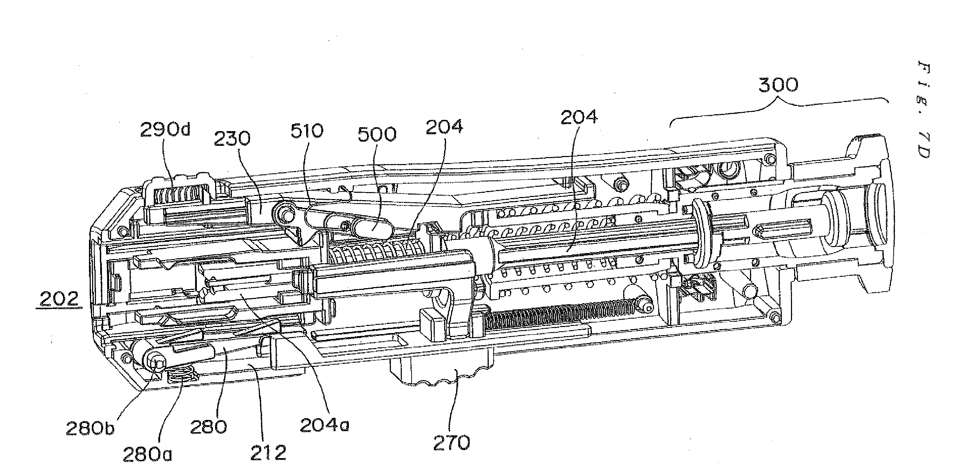

Since the lancet cap 152 moves forward in an oblique direction to deviate from the pricking pathway as described above, the pricking component 153 (more specifically, the lancet body 151 with the pricking component 153 exposed) can be launched forward without being obstructed by the lancet cap 152. Fig. 7 schematically shows the lancet assembly 100 upon pricking (namely at the time when the pricking component is being launched). As will be seen from Fig. 5D, the tip 153a of the pricking component 153 is protruding from the pricking opening 105 without being obstructed by the lancet cap 152.

When the lancet assembly 100 is loaded into the injector 200 for use, the assembly 100 is inserted through the front end opening 202 of the injector 200 backward (in the direction indicated by arrow A) as shown in Fig. 6A. This loading operation is carried out by holding the lancet holder 102 with one hand and then inserting the lancet holder 102 through the front end opening 202 of the injector 200 while holding the injector 200 with the other hand. The loading is complete when the lancet holder 102 cannot be inserted further by the force exerted by an ordinary person. The state when loading has been completed is shown in Fig. 6B. By carrying out the loading operation, the plunger of the injector 200 is retracted and thereby the force required for launching the pricking component 153 is stored in the plunger. The retracting of the plunger is accompanied by the following actions:
(1) The bridging component 154 is broken so that the lancet cap 152 is separated from the lancet body 151; and then
(2) The separated lancet cap 152 moves and deviates from the pathway of the pricking.

Figs. 7A to 7D show the injector 200 of the present invention. Fig. 7A and Fig. 7B show an external appearance of the injector 200, and Figs. 7C and 7D show the inside of the injector 200. As shown in Fig. 7C and Fig. 7D, the injector 200 has the plunger 204, the lancet cap removing part 206, the trigger lever 230, the ejector 270, a holder locking component 280, spring 280a for the holder locking component, a trigger locking component 290, spring 290d for the trigger locking component, a release spring 205a, a return spring 205b and a pricking depth adjusting mechanism 300. The plunger 204, the lancet cap removing part 206, the trigger lever 230, the ejector 270, the holder locking component 280 and the trigger locking component 290 are respectively shown in Fig. 8(a) to Fig. 8(f).

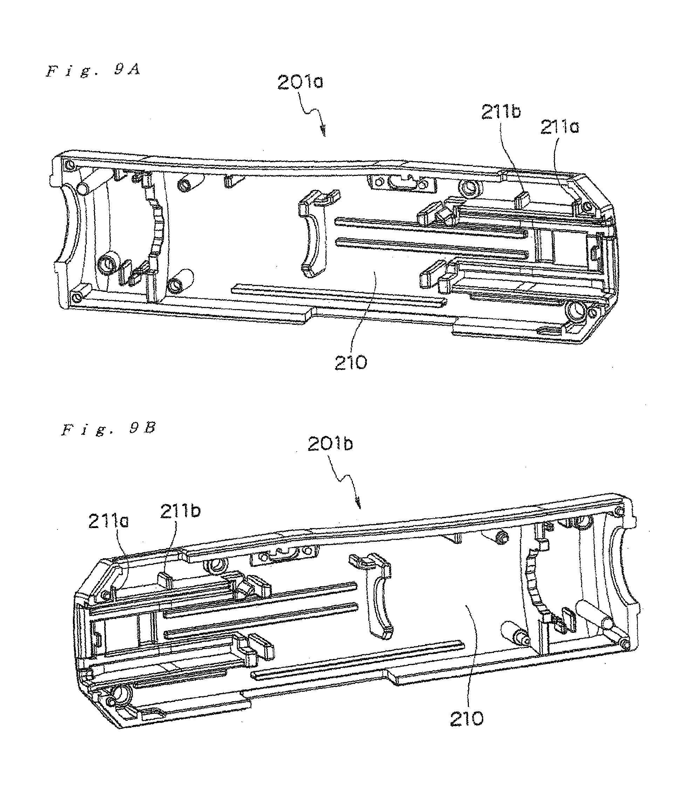

The housing 201 of the injector 200 is preferably constituted from the halved members. For example, a member 201a shown in Fig. 9A and a member 201b shown in Fig. 9B are integrally combined to form the housing 201.

The plunger 204 is disposed along the longitudinal direction of the injector 200. The plunger 204 has a function of launching the lancet body 151 in the pricking direction. As shown in Fig. 7D and Fig. 8(a), the front end portion 204a of the plunger 204 is configured to engage with the rear end of the lancet body 151 (i.e. the engaging portion of the rear end of the lancet body is indicated by reference numeral 165 in Fig. 2A). By inserting the lancet body 151 into the injector 200 upon loading the lancet assembly 100 into the injector 200, the front end portion 204a of the plunger 204 engages with the rear end 165 of the lancet body 151. Subsequently, as the lancet body 151 is inserted further backward, the plunger 204 is thrust backward. As a result, the spring 205a provided on the plunger 204 is compressed, and the force required for launching the pricking component 153 can be stored in the plunger 204.

The lancet cap removing part 206 can engage with the lancet cap 152, and thus it functions to remove the lancet cap 152 upon loading the lancet assembly 100 in the injector 200. As shown in Fig. 8(b), the lancet cap removing part 206 has such a configuration that two long members 206a and 206b protrude forward (hence the lancet cap removing part 206 may be called "protruding rod"). The edge portions 206a₁, 206b₁ of the long members 206a and 206b are capable of making contact with the distal ends 156a of the rear protruding portions 156 of the first wing parts 155 of the lancet cap 152. The lancet cap removing part 206 is installed in the injector 200 in such a manner that the edge portions 206a₁, 206b₁ of the lancet cap removing part 206 are positioned near the front end opening 202 of the injector 200 whereas the base portion 206c of the lancet cap removing part 206 is positioned on the rear end side of the injector 200 (see Figs. 7C and 8(b)). Due to this, the lancet cap 152 and the lancet cap removing part 206 can make contact with each other upon loading the lancet assembly 100 into the injector 200. Each of the edge portions 206a₁, 206b₁ of the lancet cap removing part 206 may have such a shape that is complementary with each of the rear protruding portions 156 of the lancet cap 152 so as to achieve a more reliable engagement therebetween.

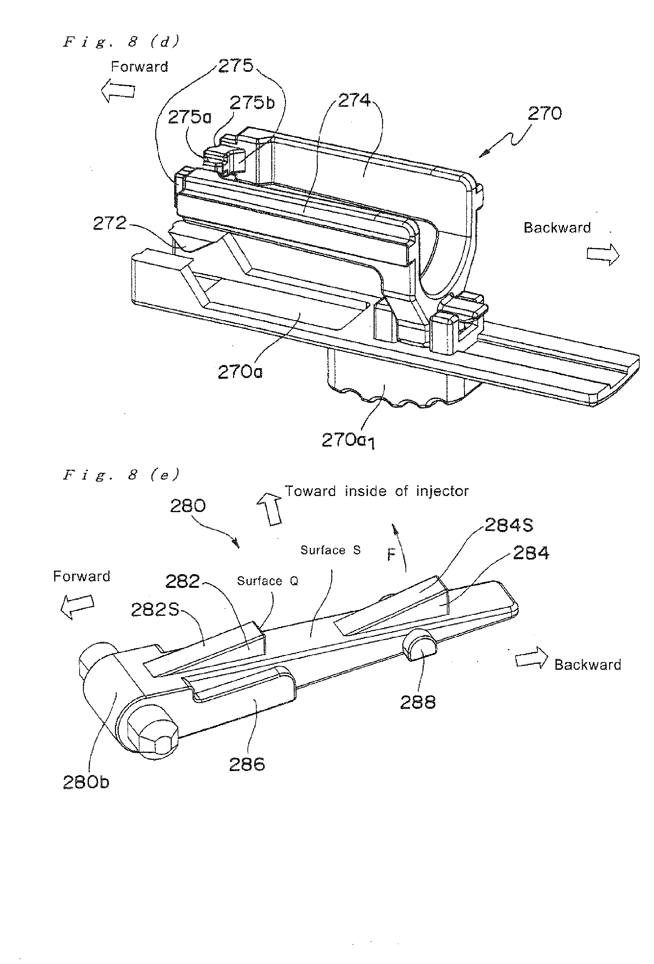

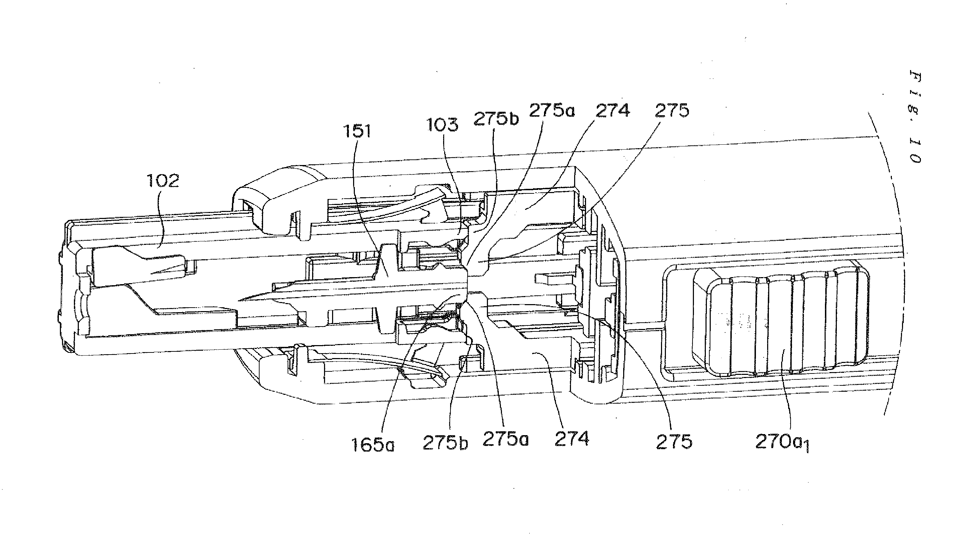

The injector 200 has the ejector 270 for removing the lancet holder 102 from the injector 200 after pricking. The ejector 270 as shown in Fig. 8(d) is provided in the injector 200 in such a manner that only a part 270a₁ of the sliding portion 270a protrudes from the injector housing 201. When the loading of the lancet holder 102 into the injector 200 is completed, the tip 275 of the pressing wing parts 274 of the ejector 270 makes it possible to contact with the edge of the opening end 103 of the lancet holder 102 and the rear end 165a of the lancet body 151 (see Fig. 10). Thus, as the part 270a₁ is pressed to slide forward, the pressing wing parts 274 of the ejector 270 acts to push the lancet holder 102 and the lancet body 151 so that the used lancet assembly 100 is ejected from the injector 200.

In the spent lancet holder 102 which has been ejected from the injector 200 by the ejector 270 after pricking, the separated lancet cap 152 and the lancet body 151 with the tip of the pricking component 153 exposed are contained. Since the pair of second wing parts 159 of the lancet body 151 is capable to make contact with (i.e. abut against) or hit the stopper surface 180 of the lancet holder 102 as described previously, the pricking component 153 will not protrude from the pricking opening 105. As a result, the spent lancet assembly 100 can be safely disposed of.

Now features of the present invention will be described in detail. The pricking device of the present invention has a function of "recharging prevention". That is, the unspent lancet assembly 100 that is not yet used can be inserted into the injector 200, while on the other hand, the spent lancet assembly that has been used for pricking and discharged from the injector cannot be inserted into the injector 200.

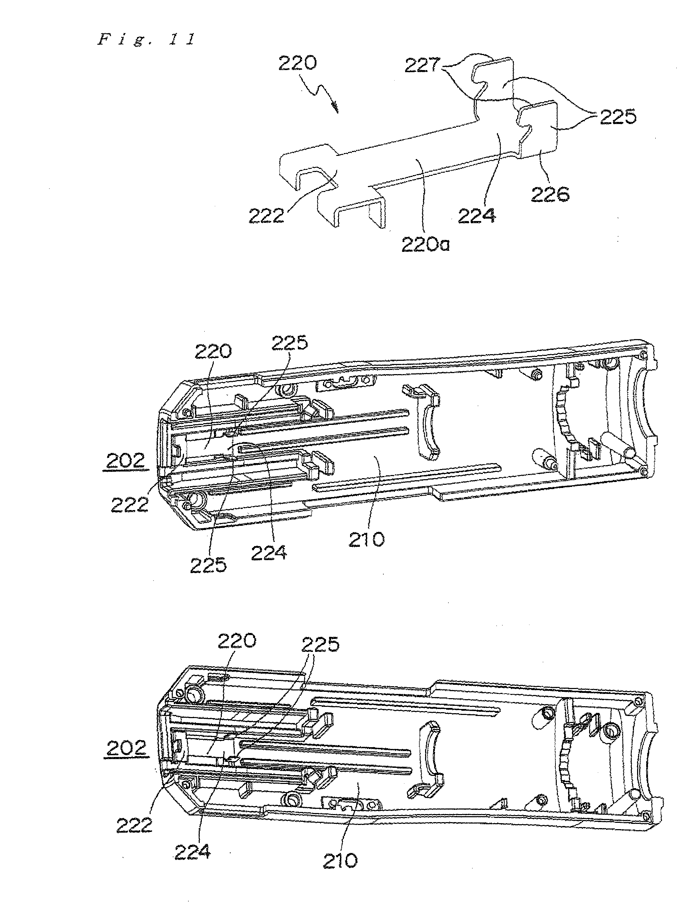

For the recharging prevention, there are mainly provided "plate spring component" of the injector and "notched portion" formed in the lancet holder. As shown in Fig. 11, the plate spring component 220 is provided on the inner wall 210 of the injector housing. Particularly the plate spring component 220 is provided adjacent to the front end opening 202 of the injector 200. One end 222 of the plate spring component 220 is fixed onto the inner wall 210 of the housing of the injector 200, whereas the other end 224 of the plate spring component 220 is a free end. It is preferable that the plate spring component 220 is disposed on the injector such that the fixed end 222 is located forward with respect to the free end 224. The free end 224 has a hook-shaped portion 225 formed in the tip end portion. The plate spring component 220 is preferably made of metal such as stainless steel. Main body of the plate spring component 220 preferably has thickness of about 0.15 to 0.20 mm. Due to this, the plate spring component 220 fixed to the inner wall 210 of the housing can be deformed outwardly (that is, expanded toward the inner wall of the housing), when the plate spring component 220 is subjected to a force acting toward the outside (a force acting from the inside of the housing toward the outside).

The notched portion 113 is formed on the edge of opening end 103 of the lancet holder 102 as shown in Fig. 1A. The notched portion 113 is preferably in a slit-like form extending along the longitudinal direction of the lancet holder.

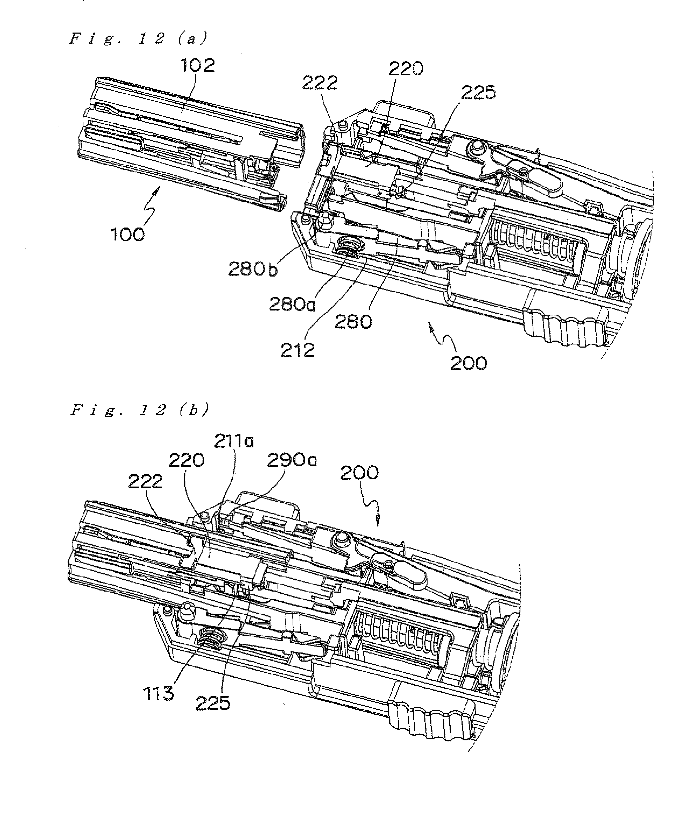

The plate spring component and the notched portion are disposed in such positional relationships that, when the lancet holder 102 is inserted into the injector 200 through the front end opening 202 thereof, the notched portion 113 of the lancet holder 102 engages with the hook-shaped portion 225 of the plate spring component 220 provided in the injector 200. Accordingly, when the spent lancet assembly 100 is inserted into the injector 200, the notched portion 113 of the lancet holder 102 engages with the hook-shaped portion 225 of the plate spring component 220 of the injector 200, and thereby the lancet holder 102 cannot be inserted further into the injector, as shown in Fig. 12(a) and Fig. 12(b). Fig. 12(a) shows the state immediately before the spent lancet assembly 100 is inserted into the injector 200, and Fig. 12(b) shows the state where the spent lancet assembly 100 is being inserted into the injector 200. As will be seen from Fig. 12(b), the hook-shaped portion 225 of the plate spring component 220 is in engagement with the notched portion 113 of the lancet holder 102 so that the lancet holder 102 cannot be inserted further into the injector. More specifically, the plate spring component 220 provided on the injector housing is preferably disposed such that the main body 220a thereof extends parallel to the inner wall surface 210 of the injector housing and the hook-shaped portion 225 extends in the direction perpendicular to the inner wall surface 210 as shown in Fig. 11 (in other words, the plate spring component is in a generally L-letter shape as a whole). In the injector, the hook-shaped portion 225 is disposed on the route of the notched portion 113 of the lancet holder 102 at the time of insertion. As a result, when the lancet holder 102 is inserted, the hook-shaped portion 225 and the notched portion 113 engage with each other such that the hook-shaped portion 225 catches the notched portion 113.

In a preferred embodiment, two plate spring components 220 are provided in a pair on the inner wall surfaces 210 of the injector housing, whereas two notched portions are also formed in a pair in the edges on the front end opening of the lancet holder. In case where only one hook-shaped portion is provided, insertion of the lancet holder is prevented at one point only, and thus the lancet holder may be tipped by the preventing force, with a possibility of the hook-shaped portion 225 and the notched portion 113 being released from the engagement. Such trouble is eliminated by provided the opposed hook-shaped portions 225 and also the opposed notched portions 113. For the same reason (namely for the purpose of reliably preventing the insertion of the lancet holder), it is preferable that two hook-shaped portions are provided on the opposing peripheral portions 226 at the tip end region of the free end 224 of the plate spring component 220, whereas the two notched portions 113 are formed in the edges on the opening end of the lancet holder in correspondence to the two hook-shaped portions (see Fig. 11). This will lead to an achievement of reliably preventing the insertion of the lancet holder 102 into the injector. With such a constitution that the opposed hook-shaped portions and the opposed notched portions are respectively provided by two not one, the insertion of the lancet holder can be prevented at four points, thus improving the stability of preventing the insertion of the lancet holder. It should be noted that the drawings all show the state of preventing the insertion of the lancet holder at four points.

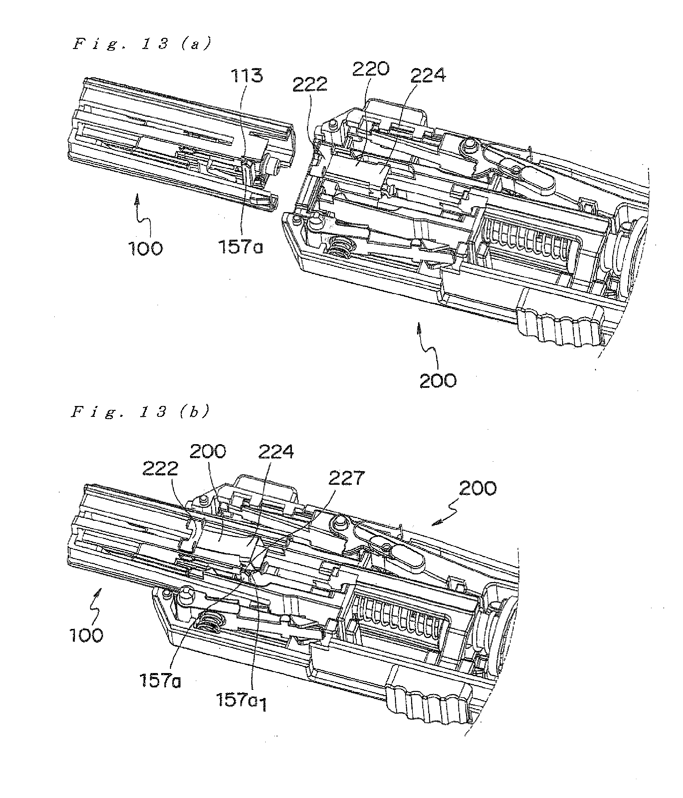

As described above, it is made impossible to insert the spent lancet holder (hence spent lancet assembly) into the injector. While on the other hand, the unspent lancet holder (hence the unspent lancet assembly) can be inserted into the injector. Such difference will be now described. The unspent lancet assembly 100 is shown in Fig. 3. As illustrated, the lancet body 151 is secured to the lancet holder 102 in the unspent lancet assembly 100. In this case, the guided protrusion 157a provided on the lancet body is located rearward with respect to the notched portion 113 of the lancet holder (see Fig. 13(a) as well as Fig. 3). Therefore, when the lancet assembly 100 in this state is inserted into the injector 200, the rear portion 157a₁ of the guided protrusion a of the lancet body 151 (see Fig.2A and Fig. 2C) makes contact with the tip end portion 227 of the free end 224 of the plate spring component 220, so that the guided protrusion 157a (specifically, the rear portion 157a₁ of the guided protrusion 157a) presses and expands the free end 224 of the plate spring component 220 toward the outside. Subsequently, when the lancet assembly 100 is further inserted, the tip end portion 227 of the plate spring component 220 comes onto the outer wall of the lancet holder 102 while the plate spring component 220 remains expanded outwardly. Thereafter, the tip end portion 227 of the plate spring component 220 moves sliding on the outer wall. As a result, the notched portion 113 of the lancet holder 102 does not engage with the hook-shaped portion 225 of the plate spring component 220 of the injector 200, and thereby the unspent lancet assembly 100 can be inserted into the injector.

It should be noted that the unspent lancet assembly 100 can be inserted into the injector 200 because the lancet body 151 is secured to the lancet holder such that the guided protrusion 157a is located rearward with respect to the notched portion 113. In order to make it possible for the guided protrusion 157a to be located rearward with respect to the notched portion 113, it is preferable that the holder wall 113a between the two notched portions 113 is further cut out in part thereof (see Fig. 1A), the two notched portions 113 being in a slit-like form extending in the longitudinal direction of the holder. Accordingly, the phrase "the protrusion of the lancet body is located rearward with respect to the notched portion of the lancet holder" used in this specification substantially means a state where the hook-shaped portion of the plate spring component and the protrusion of the lancet body can make contact with each other so that the hook-shaped portion does not engage with the notched portion of the lancet holder upon the insertion of the lancet assembly into the injector.

As shown in Fig. 2A and Fig. 3, the guided protrusion 157a is preferably chamfered on the rear side thereof. For, example refer to Fig. 2A, Fig. 2C and Fig. 3 (surface formed by chamfering is indicated by reference numeral 157a₁). This makes it easier for the rearward-chamfered surface 157a₁ of the guided protrusion 157a being moving in the inserting direction to outwardly expand the tip end portion 227 of the free end 224 of the plate spring component 220. The angle β of chamfering shown in Fig. 2B(a) is preferably from 25° to 65°, and more preferably from 35° to 55°, for example 45°.

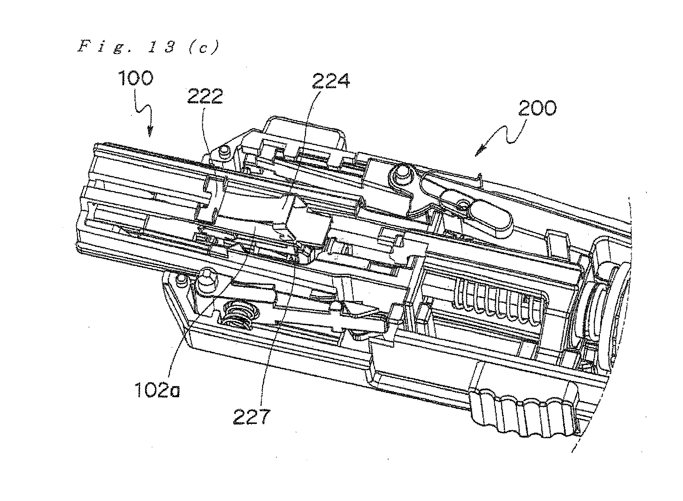

With reference to Fig. 13(a) to Fig. 13(c), the unspent lancet assembly being not inserted into the injector 200 will be now described. Fig. 13(a) to Fig. 13(c) show the state of the pricking device varies with time. Fig. 13(a) shows the state before inserting the unspent lancet assembly 100 into the injector, Fig. 13(b) shows the state during inserting the unspent lancet assembly 100 into the injector, and Fig. 13(c) shows the state where the insertion has proceeded from the state shown in Fig. 13(b). In the state shown in Fig. 13(b), the rear portion 157a₁ of the guided protrusion 157a of the lancet body is in contact with the tip end portion 227 of the free end 224 of the plate spring component 220, and thereby the guided protrusion 157a begins to outwardly expand the free end 224 of the plate spring component 220. In the state shown in Fig. 13(c), the free end 224 of the plate spring component 220 has been completely expanded outwardly, and the tip end portion 227 of the plate spring component 220 has ridden on the outer wall 102a of the lancet holder. It will be understood from these changes that the notched portion 113 of the lancet holder 102 does not engage with the hook-shaped portion 225 of the plate spring component 220 of the injector 200, and thus the unspent lancet assembly 100 can be inserted into the injector.

In the embodiment described above, the guided protrusion 157a serves to expanding the free end 224 of the plate spring component 220 outwardly. However, the present invention is not limited to this embodiment, and a protrusion that serves to expand the free end 224 of the plate spring component 220 outwardly may be provided on the lancet body separately from the guided protrusion 157a.

Now, "holder locking function", which is another main feature of the present invention, will be described. This function holds the lancet assembly in the injector upon the insertion of the lancet assembly into the injector. Namely, the holder locking function prevents the lancet assembly from coming off the injector, even when a force is exerted onto the lancet assembly in the direction opposite to the insertion of the lancet assembly. For such function, there are mainly provided holder locking component" of the injector and "locking protrusion" of the lancet holder. As shown in Fig. 7C and Fig. 7D, the holder locking component 280 is provided on the inner wall of the housing such that it is located adjacent to the front end opening 202 of the injector 200. As shown in Fig. 1A, the locking protrusion 116 is provided on the outer wall of the holder such that it is located adjacent to the opening end 103 of the lancet holder 102. The holder locking component 280 is shown in Fig. 8(e). As shown in Fig. 8(e), the holder locking component 280 has a stopper A 282 and a stopper B 284 on the surface S thereof.

As shown in Fig. 7D and Fig. 12(a), the front end 280b of the holder locking component 280 is pivotally attached onto the inner wall of the injector housing (that is, the holder locking component is fastened so as to be capable of swiveling). As shown in Fig. 12(a), the spring 280a is provided between the holder locking component 280 and the side wall surface 212 of the injector housing, and thereby the holder locking component 280 is subjected to a force acting toward the inside of the injector (i.e. the direction indicated by arrow F in Fig. 8(e)).

When the lancet assembly 100 is inserted into the injector 200 that has the holder locking component 280, the locking protrusion 116 of the lancet holder 102 slides on the holder locking component 280. As the insertion is continued, the locking protrusion 116 rides over the stopper A 282. Since the holder locking component 280 is subjected to the inward force exerted by the spring 280a, the holder locking component 280 is caused by the locking protrusion 116 to move outwardly in a circular movement with a center located at the front end upon the riding over the stopper A. However, once the locking protrusion 116 has ridden over the stopper A, the holder locking component 280 is returned to the initial position. As a result, after the locking protrusion 116 rides over the stopper A 282, the locking protrusion 116 and the stopper A 282 can make contact with each other. In other words, even when the lancet holder is pulled in the direction opposite to the inserting direction, surface P (see Fig. 1A) of the locking protrusion 116 and surface Q (see Fig. 8(e)) of the stopper A 282 can make contact with each other, and thereby the lancet holder that has been inserted to the position where the locking protrusion has ridden over the stopper A 282 does not come off the injector.

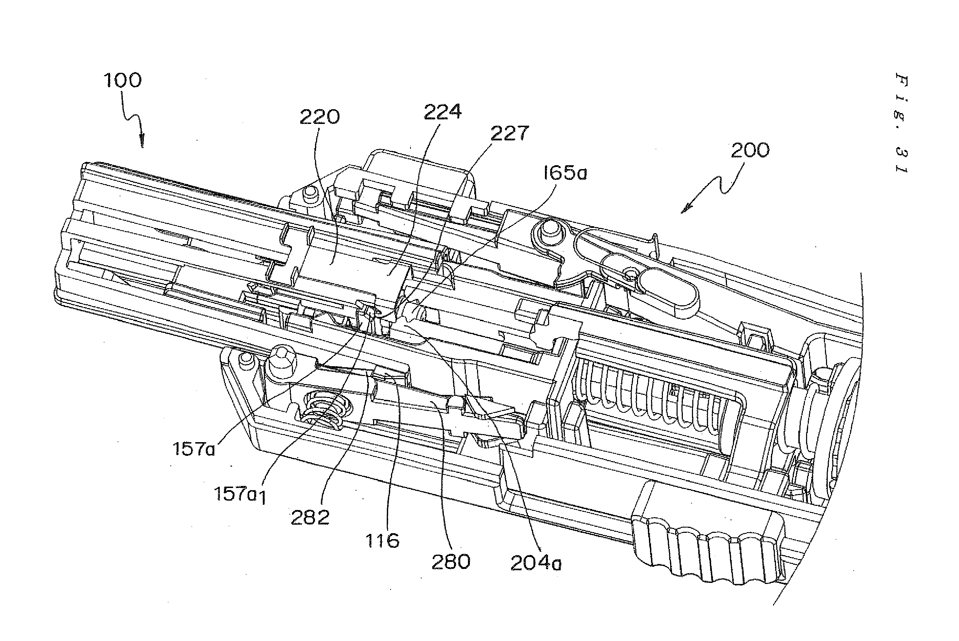

When the lancet assembly receives a force acting in the direction opposite to the inserting direction at a time point where the rear end of the lancet body and the front end portion of the plunger engage with each other so that the lancet and the plunger are integrated, there is a possibility that only the lancet holder is discharged from the injector while leaving the lancet on the front end portion of the plunger. In this case, the engagement between the lancet and the lancet holder is canceled, the lancet assembly loses its function. In case where only the lancet holder comes off the injector after the lancet cap is separated from the lancet body, the pricking component is exposed (more specifically, there is provided the lancet body with the tip of the pricking component exposed, the lancet body being attached to the plunger of the injector), which is very dangerous. With this regard, in the present invention, the locking protrusion 116 and the stopper A 282 can make contact with each other at the time point where the lancet and the plunger are integrated (see Fig. 31, for example). In the present invention, there is therefore eliminated such a trouble that only the lancet holder comes off. This means that not only the function of the lancet assembly will not be lost during the loading operation, but also the danger of the exposed pricking component is eliminated.

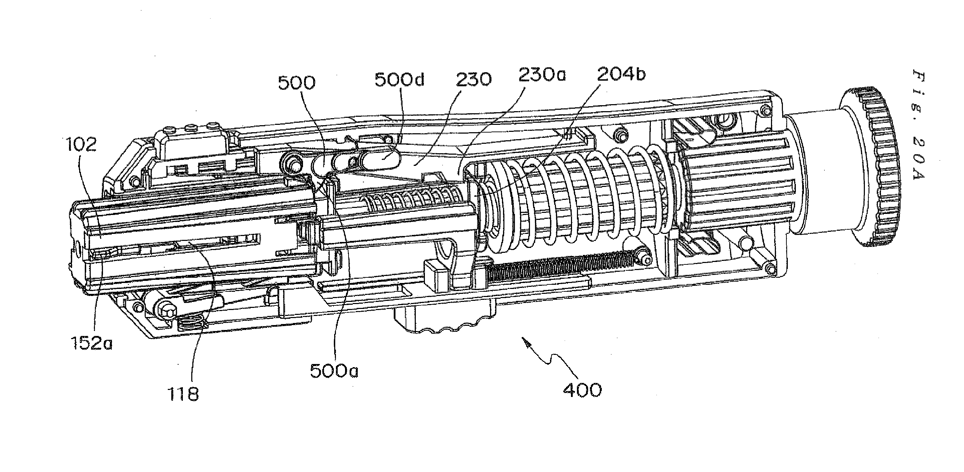

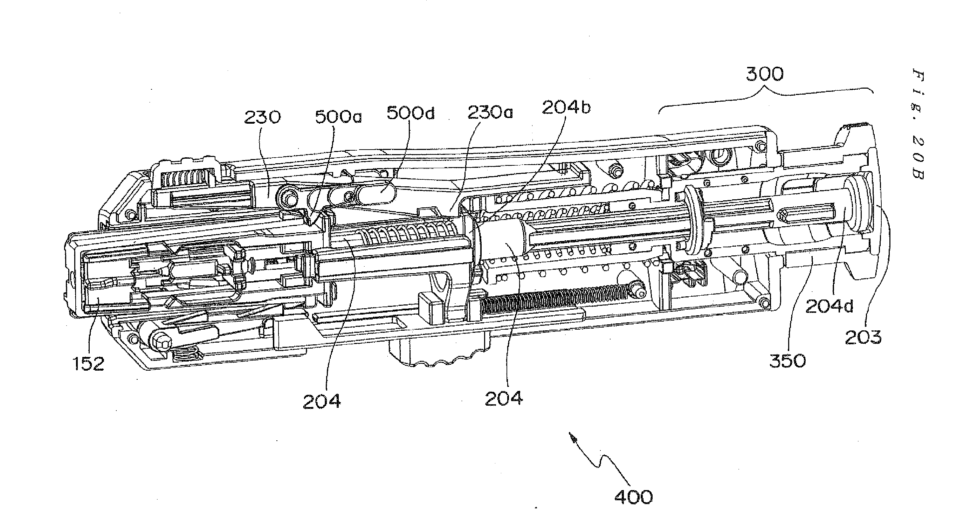

In addition to the stopper A, a stopper B is provided on the surface S of the holder locking component wherein the stopper B is located reward with respect to the stopper A (see Fig. 8(e)). Accordingly, when the insertion is continued after the locking protrusion 116 rides over the stopper A 282, it rides over the stopper B 284. Since the holder locking component 280 is subjected to the inward force exerted by the spring 280a similarly to the case of the stopper A, the holder locking component 280 is caused by the locking protrusion 116 to move outwardly in a circular movement with a center located at the front end upon the riding of the locking protrusion over the stopper B. After the holder locking component 280 rides over the stopper B, the holder locking component 280 is returned to the initial position. After that, the locking protrusion 116 and the stopper B 284 can make contact with each other, similarly to the case of the stopper A. The time point where the locking protrusion 116 has ridden over the stopper B 284 is substantially the same as the time point where the lancet assembly has been inserted to a position where the lancet body can be launched in the pricking direction. Thus, the loading of the lancet assembly 100 into the injector 200 is completed when the locking protrusion 116 has ridden over the stopper B. In other words, when the lancet assembly has been inserted enough for the plunger to store the force required to launch the pricking component, the locking protrusion 116 has ridden over the stopper B 284 of the holder locking component 280 so that the locking protrusion 116 and the stopper B 284 can make contact with each other. Also as shown in Fig. 20A and Fig. 20B, when the lancet assembly has been inserted enough to store the force required to launch the pricking component in the plunger, the rear stepped portion 230a of the trigger lever 230 adjoins the front side of the flange 204b of the plunger 204, which will lead to the cocking of the trigger lever 230. This means that the cocking of the trigger lever 230 is achieved after the stopper B 284 of the holder locking component 280 rides over. To sum up, while the lancet assembly is inserted until the force required for launching the pricking component is stored in the plunger, the locking protrusion 116 rides over the stopper B 284 so that the locking protrusion 116 and the stopper B 284 can come into contact with each other, and also the cocking of the trigger lever 230 is achieved. In other words, the lancet holder is held in place when the loading of the lancet assembly into the injector is completed.

It is preferable that the locking protrusion 116 of the lancet holder 102 and the stopper A 282 of the holder-locking component 280 respectively have flat surfaces 116S and 282S which make it possible to make complementary contact with each other (see Fig. 1A and Fig. 8(e)). In this case, the locking protrusion 116 can smoothly ride over the stopper A 282. It is also preferable that the locking protrusion 116 of the lancet holder 102 and the stopper B 284 of the holder locking component 280 respectively have flat surfaces 116S and 282S which make it possible to make complementary contact with each other (see Fig. 1A and Fig. 8(e)). In this case, the locking protrusion 116 can smoothly ride over the stopper B 284.

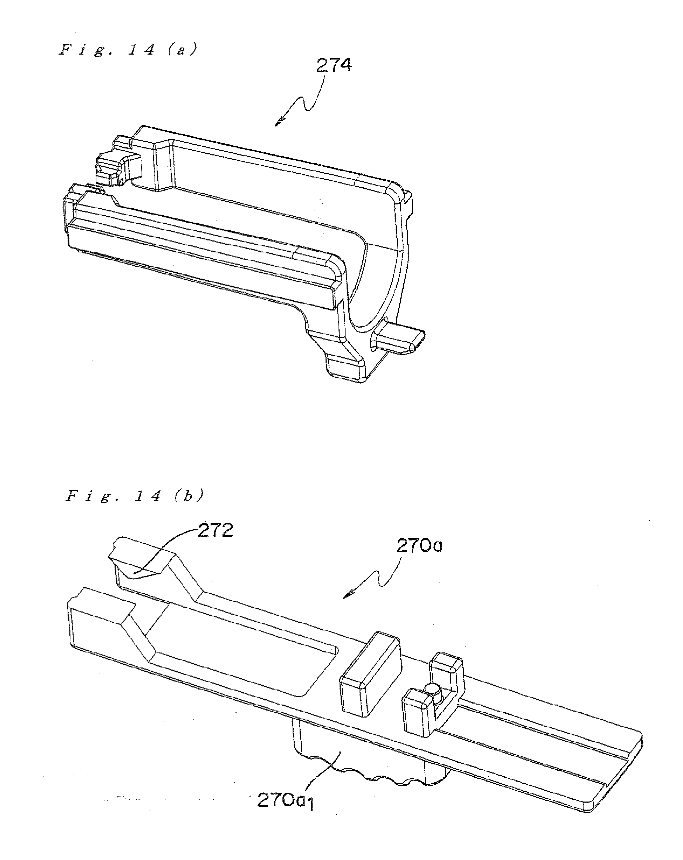

The pricking device of the present invention has "holder lock releasing function" in correspondence to the holder locking function. This function makes it possible to smoothly discharge the spent lancet assembly by canceling the holder locking function, upon discharging the spent lancet assembly from the injector after the pricking. For such function, there are mainly provided a release part A and/or a release part B provided in the holder locking component 280 and a releasing part provided in the ejector. As shown in Fig. 8(e), the release part A 286 and/or the release part B 288 are provided on the side of the holder locking component 280. As illustrated, it is preferable that the release part A 286 is located adjacent to the stopper A 282 in the holder locking component 280 (that is, the release part A 286 and the stopper A 282 are preferably disposed to adjoin each other in the lateral direction/transverse direction of the holder locking component 280). It is also preferable that the release part B 288 is located adjacent to the stopper B 284 (that is, the release part B 288 and the stopper B 284 are preferably disposed to adjoin each other in the lateral direction/ transverse direction of the holder locking component 280). More specifically, it is preferable that the release part A 286 is provided so as to protrude in the transverse direction of the holder locking component from the side face of the holder locking component, the side face being is adjacent to the position where the stopper A 282 is provided. It is also preferable that the release part B 288 is provided to as to protrude in the transverse direction of the holder locking component from the side face of the holder locking component, the side face being adjacent to the position where the stopper B 264 is provided. The release part A desirably serves to release the stopper A from the locked state, and also the release part B desirably serves to release the stopper B from the locked state. The releasing part 272 is provided on the front portion of the ejector 270, as shown in Fig. 8(d) and Fig. 14(b).

The release part and the releasing part work properly when the used lancet assembly is discharged from the injector by sliding the ejector after pricking. Specifically, upon pressing the ejector two slide it forward, the releasing part 272 moves sliding on the release part A 286 and/or the release part B 288 so that the holder locking component 280 is caused to move outwardly in a circular movement with a center located at the front end 280b. As a result, the contact-enabled state between the locking protrusion 116 of the lancet holder and the stopper A 282 and/or the stopper B 284 of the holder locking component 280 is canceled. Such cancellation leads to an achievement of a smooth discharge of the lancet holder 102 from the injector 200. In case where both of the stopper A 282 and the stopper B 284 are provided on the holder locking component 280, the releasing part 272 of the ejector 270 first moves sliding on the stopper B 288, and then moves sliding on the stopper A 286. As a result, the contact-enabled state between the locking protrusion 116 and the stopper B 284 is canceled first, and then the contact-enabled state between the locking protrusion 116 and the stopper A 282 is canceled.

The release part B 288 of the holder locking component 280 and the releasing part 272 of the ejector 270 are designed in shapes that coordinate with each other, so that the releasing part 272 can move smoothly in contact with the release part B 288. Similarly, the release part A 286 of the holder locking component 280 and the releasing part 272 of the ejector 270 are designed in shapes that coordinate with each other, so that the releasing part 272 can move smoothly in contact with the release part A 286.

It is preferable that the releasing part 272 of the ejector is formed in a shape of inverted triangle as shown in Fig. 8(d). In this case, it is preferable that the release part B 288 of the holder locking component 280 is formed in a semi-circular shape, and the release part A 286 of the holder locking component 280 is formed in a continuous shape as shown in Fig. 8(e). In case where they are in such forms, the riding of the releasing part 272 over the release part B 288 causes the holder locking component 280 to move toward the outside and then return to its original position, and also the sliding of the releasing part 272 on the release part A 286 causes the holder locking component 280 to move toward the outside and keep such moved position due to the continuous form of the release part A 286.

According to the present invention, the ejector 270 provided in the injector of the pricking device serves to press not only the lancet holder 102 but also the lancet body 151. Namely, upon the discharge of the lancet assembly, the ejector 270 presses both of the lancet holder 102 and the lancet body 151. This makes it possible to discharge the spent lancet assembly smoothly. As shown in Fig. 8(d), the ejector 270 has the pair of pressing wing parts 274 that extend forward from the middle of the ejector main body 270a. Since the tip 275 of the pressing wing parts 274 can make contact with not only the edge of the opening end 103 of the lancet holder 102 but also the rear end portion 165a of the lancet body 151, both of the lancet holder 102 and the lancet body 151 can be pushed.

The ejector 270 shown in Fig. 8(d) can be constructed by combining the wing part 274 having the configuration of a pair of tweezers shown in Fig. 14(a) and the ejector main body 270a shown in Fig. 14(b). However, when possible, it is preferable to mold the ejector 270 shown in Fig. 8(d) in a single piece.

As shown in Fig. 8(d), it is preferable that each wing of the pair of pressing wing parts 274 is formed in a squarely bent shape such that it extends upward from the ejector main body 270a and then extends forward. It is also preferable that the tip 275 of each wing part is bent inwardly, so that the distal end portion of each wing part can push not only the edge of the opening end 103 of the lancet holder 102 but also the rear end 165a of the lancet body 151. As shown in Fig. 10, it is particularly preferable that an inside tip 275a of each wing part makes contact with the rear end 165a of the lancet body whereas an outside tip 275b of each wing part makes contact with the edge of the opening end 103 of the lancet holder (each wing preferably has such a recess in the contact portion, as shown in Fig. 8(d) in order to ensure the reliable contact). Such contact makes it possible for the pair of pressing wing parts 274 to push both of the lancet holder 102 and the lancet body 151 upon the forward sliding of the ejector.

In order to make the forward sliding motion of the ejector 270 stable, or in order to enable the tip 275 of the pair of pressing wing parts 274 to reliably make contact with the rear end 165a of the lancet body and the edge of the opening end of the lancet holder, the plunger of the injector preferably has a groove 204f on a side thereof (see Fig. 8(a)). More specifically, as shown in Fig. 8(a), the groove 204f is preferably formed on a part of the end portion of the plunger. It is also preferable that a pair of the grooves 204f is provided, one groove being opposed to the other groove in the plunger. In a case where the plunger 204 has the grooves 204f, the tips 275 of the pressing wing parts 274 of the ejector 270 is guided along the groove 204f during the sliding motion, and thereby the sliding motion becomes stable. In a case where the tips 275 of the pressing wing parts 274 are guided by the grooves 204f, the movement of the tips 275 of the pressing wing parts 274 can be kept within the predetermined pathway, so that the tips 275 of the pressing wing parts 274 can reliably make contact with the rear end 165a of the lancet body 151 and the edge of the opening end 103 of the lancet holder 102.

As described above, the ejector 270 has the holder lock releasing function wherein the releasing part 272 of the main body 270a engages with the release part A 286 and/or the release part B 288 of the holder locking component 280. This means that, when the ejector 270 is moved sliding forward, not only the pair of pressing wing parts 274 pushes the lancet holder 102 and the lancet body 151 simultaneously, but also the contact-enabled state of the protrusion 116 of the lancet holder 102 with the stopper A 282 and/or the stopper B 284 of the holder locking component 280 is canceled by releasing part 272 of the ejector main body 270a. In other words, the pressing of the assembly and the releasing of the holder lock operate simultaneously with each other, which will lead to an achievement of the smooth discharge the used lancet assembly from the injector.

The pricking device and the lancet assembly of the present invention are provided with various safety features. One of the safety features is a function of preventing the spent lancet from coming off the lancet holder. For such feature, there are mainly provided the securing bosses of the lancet holder (particularly a boss A 121a provided at a forward position among the two securing bosses 121a, 121b) and the securing protrusion 158 of the lancet body. As shown in Fig. 1B and Fig. 1C, the front face 121a₁ of the securing boss A 121a (which is located forward with respect to the other boss) forms a flat surface that faces forward. More specifically, the front-sided face 121a₁ of the securing boss A 121a is the flat surface that extends toward the inside of the holder in the direction perpendicular to the pricking direction. As shown in Fig. 5D, the securing protrusion 158 of the spent lancet body is located forward with respect to the securing boss 121a. Accordingly, even when the spent lancet body 151 moves backward within the lancet holder 102, the movement of the securing protrusion 158 of the lancet body 151 is surely stopped by the front side 121a of the securing boss 121a, and thereby the spent lancet body 151 does not come off the lancet holder 102. In other words, in a case where the front side of the securing boss 121a is in a form of a smooth slope, there is provided a undesirable possibility when the spent lancet body 151 moves backward within the lancet holder 102 and then the securing protrusion 158 makes contact with the front side of the securing boss 121a. Namely, in this case, the securing protrusion 158 may ride over the securing boss 121a since the securing protrusion 158 of the lancet body 151 does not receive strong resistance from the front side of the securing boss 121a. This means that the spent lancet body 151 may come off the lancet holder 102 in the case where the front side of the securing boss 121a is in a form of a smooth slope. According to the present invention, in contrast, since the front side 121a₁ of the securing boss 121a of the lancet holder forms the flat surface that faces forward, the securing protrusion 158 of the lancet body 151 receives stronger resistance from the front side of the securing protrusion 121a so that the securing protrusion 158 does not ride over the boss 121a. Thus there is no possibility that the spent lancet body 151 comes off the lancet holder 102.

As will be understood from the foregoing description, the present invention provides the lancet assembly that avoids the danger of human body touching the spent lancet with the pricking component exposed, and that makes it possible to safely dispose of the spent lancet assembly in a hygienic manner.

Another safety feature is an indicator function provided on the lancet assembly and the injector. For such function, there are mainly provided "indicating part" of the lancet cap 152, the indicating part protruding in a transverse direction of the lancet cap, and "slit" formed on the wall of the lancet holder. As shown in Fig. 2A and Fig. 2C, the indicating part 152a of the lancet cap 152 is provided on the side of the lancet cap. The indicating part 152a is preferably provided on the lancet cap such that it protrudes in a transverse direction of the lancet cap and located adjacent to the forward end of the lancet cap, as illustrated. Also as shown in Fig. 1A, the slit 118 of the lancet holder is preferably provided such that it extends in the longitudinal direction of the lancet holder 102. In the state of the lancet and the lancet holder being combined together, the indicating part 152a can be seen from the outside through the slit 118 of the lancet holder 102, as shown in Fig. 20A. Since the indicating part 152a is provided on the lancet cap 152, the position of the lancet cap 152 can be known from the position of the indicating part 152a, and thereby it can be determined whether or not the lancet cap 152 has been separated from the lancet body 151. When the indicating part 152a is seen at a rearward position of the slit 118, it is indicated that the lancet cap 152 has been not yet separated from the lancet body 151. When the indicating part 152a is seen at a forward position of the slit 118, it is indicated that the lancet cap 152 has been separated from the lancet body 151. This means that it can be determined whether or not the lancet assembly now in hand has been used by the position of the indicating part. As a result, there is reduced possibility of the spent lancet assembly being used again inadvertently by the user. And also, because it can be known indirectly from the outside that the lancet cap 152 has been separated from the lancet body 151, the user is given the means for reliably identifying the stage of operation, which will lead to a smooth handling of the device.

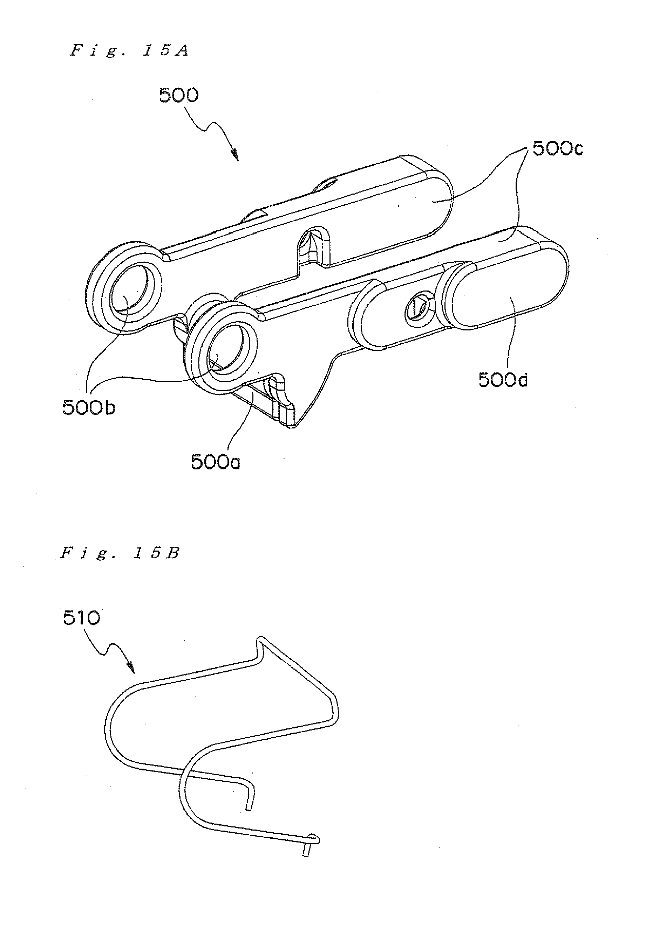

Another indicator function provided on the injector will be now described. For such indicator function, there are mainly provided "indicator component 500 pivoted on the trigger lever 230", "edge of the opening end 103 of the lancet holder 102" and "window provided in the injector housing". The indicator component 500 is shown in Fig. 15A. The window of the injector housing is shown by reference numeral 216 in Fig. 7A and Fig. 7B. As shown in Fig. 15A, the indicator component 500 is pivoted at the front end 500b thereof on the trigger lever 230. The side of a rear end 500c of the indicator component 500 is provided with the indicating portion 500d. The indicator component 500 is provided along with a spring 510 (see Fig. 15B) on the injector 200 (see Fig. 7D). As a result, as long as there is no force exerted from the outside, the indicator component 500 is forced by the spring 510 to move in a circular movement clockwise (or inwardly) around a center located at the front end 500b. In this state, the indicating part 500d provided on the side of the rear end 500c of the indicator component 500 is not seen through the injector window 216. In contrast, when the lancet assembly 100 has been inserted to the pricking-enabled position, the edge of the opening end 103 of the lancet holder 102 (more particularly the peripheral region B shown in Fig. 3) makes contact with and pushes the holder contact region (portion indicated by reference numeral 500a in Fig. 15A) of the indicator component 500. As a result, the indicator component 500 moves in a circular movement counterclockwise (or outwardly) around the center located at the front end 500b, so that the indicating part 500d provided on the side of the rear end moves to a position where it can be seen through the injector window 216.

Thus it can be determined whether or not the lancet assembly 100 is ready for pricking, by checking to see if the indicating part 500d can be seen through the injector window 216 or not. This enables the user to operate the device by reliably identifying the stage of operation and to handle the device smoothly, while preventing erroneous operation.

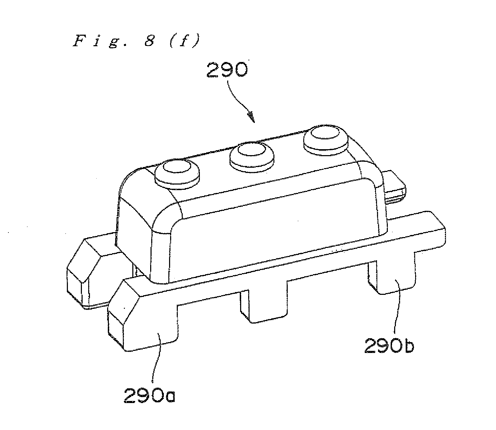

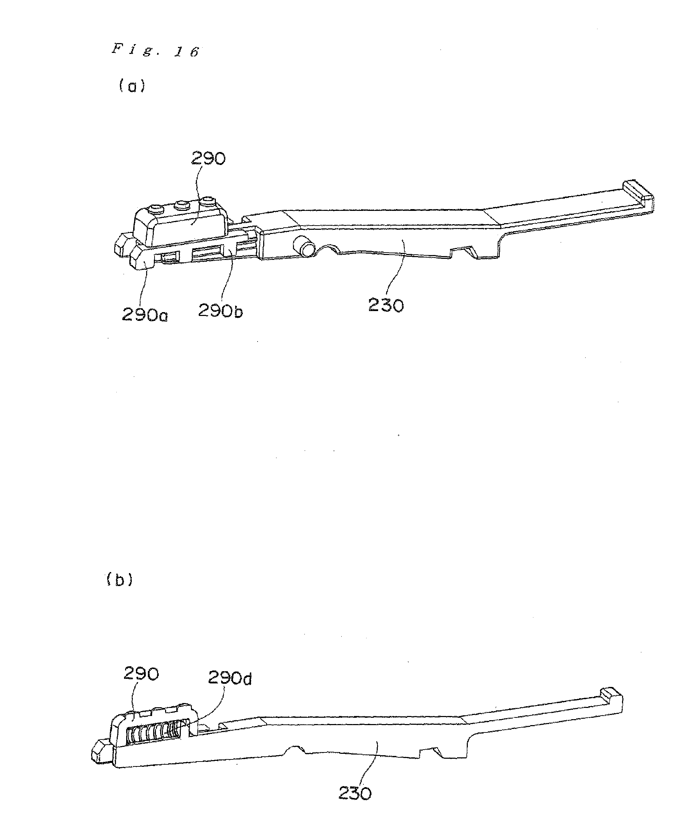

Another safety feature of the pricking device is a trigger lever locking mechanism. This mechanism serves to prevent the depression of the trigger lever during the loading operation. For such mechanism, there are mainly provided "trigger locking component 290". The trigger locking component is provided on the front end of the trigger lever 230. The trigger locking component 290 is shown in Fig. 8(f). As shown in Fig. 16(a), the trigger locking component 290 is provided so as to be capable of sliding back and forth on the front end of the trigger lever 230. A spring 290d is provided within the trigger locking component 290 as shown in Fig. 16(b), so that the trigger locking component 290 is subjected to a force acting forward. A part of the top side of the trigger locking component 290 projects through the opening 214 of the injector housing as shown in Fig. 7A. The trigger locking component 290 is allowed to move back and forth in the opening 214.

The trigger locking component 290 is kept at the foremost position in opening 214 of the injector housing as long as no extraneous force is exerted (see Fig. 6A and Fig. 6B). In this state, locking parts (indicated by 290a and 290b in Fig. 16(a)) provided at the bottom of the trigger locking component 290 can make contact with lock part (indicated by 211a and 211b in Fig. 9A and Fig. 9B) provided on the inner wall 210 of the injector housing, and thereby the trigger locking component 290 cannot be pressed into the injector. Specifically, the locking part 290a of the trigger locking component can make contact with the lock part 211a of the inner wall 210 of the housing, and also the locking part 290b of the trigger locking component can make contact with the lock part 211b of the inner wall 210 of the injector housing, and thereby the trigger locking component 290 cannot be pressed into the injector (Fig. 12(b) shows the state in which the locking part 290a and the lock part 211a are in contact with each other). Accordingly, when there is no extraneous force exerted and thus the trigger locking component 290 is located at a front position in the housing opening 214 of the injector 200, the trigger lever 230 cannot be depressed into the injector.

In contrast, when the extraneous force is exerted against the force of moving forward, and thereby the trigger locking component 290 moves backward in the housing opening 214 of the injector 200, the contact-enabled state between the locking parts 290a and 290b provided at the bottom of the trigger locking component 290 and the lock parts 211a and 211b provided on the inner wall 210 of the injector housing is canceled. As a result, the trigger locking component 290 can be pressed into the injector. Specifically, when the trigger locking component 290 is caused to move backward, the contact-enabled state between the locking part 290a of the trigger locking component 290 and the lock part 211a of the inner wall 210 of the housing is canceled and also the contact-enabled state between the locking part 290b of the trigger locking component 290 and the lock part 211b of the inner wall 210 of the housing is canceled, and thereby the trigger locking component 290 can be pressed into the injector.

Since the trigger lever 230 cannot work unless the trigger locking component 290 is moved backward as described above, there is eliminated such a danger that the pricking component is inadvertently launched by carrying out the loading operation with the trigger lever 230 depressed.

In the lancet assembly of the present invention, the stability of pricking motion of the pricking component is improved. For such improved stability, there are mainly provided the combination of the slit 118 of the lancet holder and the securing protrusion 158 of the lancet body. The pair of wing parts 159 extending toward the back of the lancet body also contributes to the improved stability.

The combination of the slit of the lancet holder and securing protrusion of the lancet body will now be described. According to the present invention, the pricking component is ready to be launched after the front-sided boss 121a of the lancet holder 102 rides over the securing protrusion 158 of the lancet body 151 (when viewed differently, the pricking component is ready to be launched after the securing protrusion 158 of the lancet body 151 rides over the front-sided boss 121a of the lancet holder 102), and therefore the shape of the securing protrusion 158 may be deformed during the riding process. Also when the lancet 150 is inserted into the lancet holder 102 to obtain the lancet assembly 100 before use, the securing protrusion 158 of the lancet body 151 rides over the rear-sided boss 121b of the lancet holder 102, thus giving rise to the possibility of the shape of the securing protrusion 158 to be deformed. According to the present invention, however, since the securing protrusion 158 moves along the slit 118 of the lancet holder 102 upon the pricking, the securing protrusion 158, even if its shape is deformed, does not make frictional contact with the wall of the holder. As a result, there is eliminated the resistance against the sliding motion (i.e. the resistance due to the contact), which leads to an achievement of the stability of pricking motion of the pricking component.

The lancet assembly of the present invention has the protrusion 158 for securing the lancet body 151 to the lancet holder 102 and the protrusions 157a, 157b for guiding the lancet body 151 within the lancet holder 102. The protrusion 158 and the protrusions 157a, 157b are provided separately from each other. This also contributes to the improvement of stability of the pricking motion of the pricking component 153. Specifically, even when the shape of the securing protrusion 158 is deformed, what guide the pricking motion of the pricking component (namely what guide the lancet body) in the present invention are the guided protrusions 157a and 157b shown in Fig. 2A, not the securing protrusion 158. Therefore, even when the shape of the securing protrusion 158 is deformed, there is no influence on the guiding of the pricking component and thus the stable pricking is achieved. In other words, the securing protrusion 158 extends in the transverse direction of the lancet body along the flat surface A that includes the center axis of the lancet, while on the other hand the guided protrusion 157 extends in the transverse direction of the lancet body along the flat surface B (excluding the flat surface A) that is parallel to the flat surface A (the flat surface A and the flat surface B are schematically shown in Fig. 2B(g)), and thereby, the guiding of the pricking component is not affected even when the shape of the securing protrusion 158 is deformed, and thus the stable pricking motion is enabled. Much the same is true on a case where the guided protrusion 157 extends in the transverse direction of the lancet body along the flat surface A that includes the center axis of the lancet whereas the securing protrusion 158 extends in the transverse direction of the lancet body along the flat surface B that is parallel to the flat surface A. Namely, in this case, the guiding of the pricking component is not affected even when the shape of the securing protrusion 158 is deformed, and thus the stable pricking motion is enabled.

The pair of wing parts 159 extending backward of the lancet body is also related to the stability of pricking motion of the pricking component 153. As will be described in more detail later, upon the pricking, the wing parts 159 accommodated in the bow-shaped groove 206d of the lancet cap removing part 206 moves forward in the pricking direction along the bow-shaped groove 206d. Since the wing part 159 extends backward, the moving wing part 159 receives less resistance from the wall of the bow-shaped groove 206d. Accordingly, the stability of pricking motion of the pricking component 153 is not impaired.

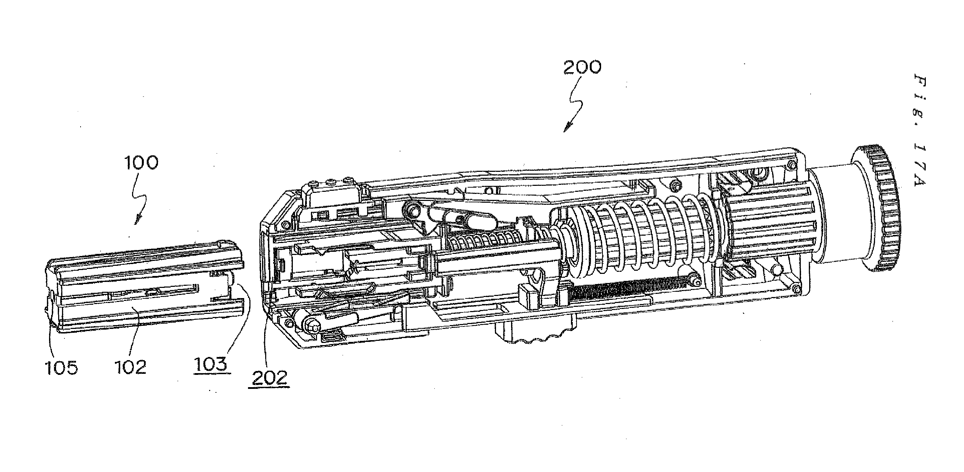

With reference to Figs. 17 to 27, the procedure of operation will now be described serially from the loading of the lancet assembly 100 through the completion of loading and the pricking until the ejection of the spent lancet assembly 100 after pricking. Figs. 17 to 27 show the time changes of the lancet assembly 100 and the injector 200 in the sequential order of the figure numbers. Figs. 17A to 27A and Figs. 17B to 27B show the same states, respectively, when the figure number is the same.

First, as shown in Figs. 17A and 17B, the lancet assembly 100 and the injector 200 are prepared. In this case, the prepared lancet assembly 100 is one in which the lancet is housed in the lancet holder 102 and the lancet body 151 is secured to the lancet holder 102. Then, the lancet holder 102 is inserted into the injector 200 through the front end opening 202 of the injector 200 to commence the loading of the lancet assembly 100. Upon loading, the lancet holder 102 is inserted in such an orientation that the pricking opening 105 of the lancet holder 102 faces forward and the opening end 103 of the lancet holder 102 faces backward, as shown in Fig. 17A. In other words, the opening end 103 of the lancet holder 102 firstly passes through the front end opening of the injector 200.

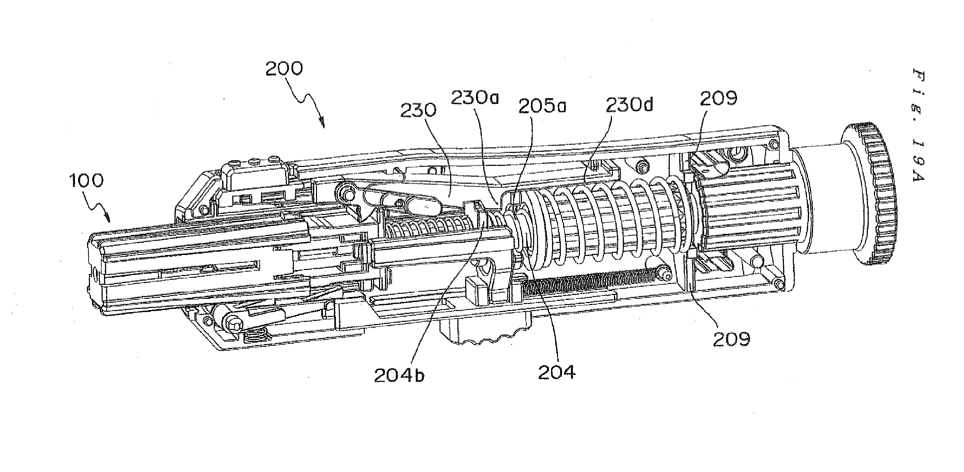

Upon the loading the lancet assembly 100, the lancet cap removing part 206 enters the lancet holder 102 through the opening end of the lancet holder 102 as shown in Fig. 18A and Fig. 18B (particularly see Fig. 18B). Since the rear portion of the guided protrusion of the lancet body 151 outwardly expands the free end of the plate spring component, the notched portion of the lancet holder does not engage with the hook-shaped portion of the plate spring component of the injector, and therefore the lancet assembly can be inserted into the injector. As the insertion is continued, the locking protrusion of the lancet holder rides over the stopper A of the holder locking component. As the insertion is continued further, the distal end 156a of the lancet cap engage integrally with the edge portions 206a₁, 206b₁ of the lancet cap removing part 206, as shown in Fig. 19A and Fig. 19B (particularly see Fig. 19B). Substantially simultaneously as this engagement takes place, the engagement portion 165 of the rear end of the lancet body makes contact with (i.e. abuts on) the front end 204a of the plunger 204 of the injector (see Fig. 19B). The engagement portion 165 and the front end 204a are finally interconnected integrally. When the lancet holder 102 is further inserted after the lancet body 151 and the plunger 204 make contact with each other, the lancet body 151 secured to the lancet holder 102 acts to press the plunger 204, which results in the retracting of the plunger 204.

As shown in Fig. 19A and Fig. 19B (particularly in Fig. 19B), the plunger 204 has a flange 204b formed at the intermediate portion thereof. A release spring 205a is provided around the plunger 204 and between the flange 204b and a partition 209 provided within the injector housing 201. The spring 205a functions to move the plunger 204 forward (namely in the pricking direction) when there is no external force acting on the plunger 204. In the initial state where no external force is acting, the flange 204b of the plunger 204 and the engagement portion 230c (or the front stepped portion 230c) of the trigger lever 230 are in engagement, as shown in Fig. 18A and Fig. 18B. Such engagement between the flange 204b and portion 230c makes it possible to restrict the forward movement of the plunger 204. The rear end portion 165 of the lancet body 151 can press the plunger 204 backward against the force of the spring 205a urging the plunger 204 to return forward. The retracting of the plunger eventually causes the spring 205a of the plunger 204 to be compressed and the force required for launching the pricking component 153 is stored in the plunger 204.

Whereas the lancet body 151 is secured to the lancet holder 102, the lancet cap 152 is not secured to the lancet holder 102. The further insertion of the lancet holder from the state shown in Fig. 19B causes the lancet cap 152 to be pressed by the lancet cap removing part 206 in the pricking direction. That is, there is generated the force causing the lancet cap 152 and the lancet body 151 to depart from each other, and thereby the bridging component 154 for interconnecting the lancet cap 152 and the lancet body 151 is broken in two so that the lancet cap 152 is separated from the lancet body 151. Since the lancet cap 152 is separated while leaving the pricking component 153 in the lancet body 151, the breaking of the bridging component 154 provides the lancet body 151 with the tip of the pricking component 153 exposed.

When the lancet holder 102 is inserted further into the injector 200 following the separation, the separated lancet cap 152 is further pressed forward by the lancet cap removing part 206. Thus, the separated lancet cap 152 moves forward within the lancet holder 102 toward the position that is off the pricking pathway while keeping contact with and sliding on the slope component 170. The slope component 170 provided within the lancet holder 102 is clearly shown in Fig. 17B, for example. It is shown in Fig. 20B and Fig. 5C that the separated lancet cap 152 has moved to the position that is off the pricking pathway.

The injector 200 has the trigger lever 230 (see Fig. 19A) that is pressed to launch the pricking component. The trigger lever 230 is configured to be subject to the force acting inwardly. For example, the trigger lever 230 is configured to be subject to the inward force toward the inside of the injector 200 by a leaf spring thereof (the leaf spring being a portion indicated by 230d in Fig. 19A). After the flange 204b of the plunger 204 moves backward while pressing the rear stepped portion 230a of the trigger lever 230 toward the outside, the front portion of the flange 204b of the plunger 204 is located adjacent to the rear stepped portion 230a of the trigger lever 230, which will lead to the cocking of the trigger lever 230 as shown in Fig. 20A and Fig. 20B. Substantially in parallel to the trigger lever 230 being cocked, the locking protrusion 116 of the lancet holder 102 rides over the stopper B 284 of the holder locking component 280. Also substantially in parallel to the trigger lever 230 being cocked, the edge of the opening end of the lancet holder push the holder contact portion 500a provided on the indicator component 500, and thereby the indicator component 500 is turned counterclockwise to a position where the indicating portion 500d can be seen through the indicator window provided in the injector housing. In this state of the cocking, the distance between the rear end 204d of the plunger 204 and the inner wall surface 203 on the rear end of the injector 200 (specifically, "inner wall surface 203" is the inner wall surface of an outer drum 350 of the pricking depth adjusting mechanism 300 to be described later) may be very small, or may be zero meaning that the rear end 204d and the inner wall surface 203 are in contact with each other, as shown in Fig. 20B.

The rear end 204d of the plunger 204 and inner wall surface 203 on the rear end of the injector 200 finally make full contact with each other (i.e. abut against each other), which prevents the plunger 204 from being retracting further. Therefore, when the lancet holder 102 is forced to move further in the direction of insertion, the lancet holder 102 receives the force causing it to move in the direction of insertion, whereas the lancet body 151 receives the force resisting it. In this state, an attempt to move the lancet body 151 in the direction of insertion by inserting the lancet holder 102 is prevented since the lancet body 151 is in engagement with the plunger 204 (which cannot be retracted further), and thereby the force causing the lancet cap 152 and the lancet body 151 to depart from each other is generated. In this case, when the force exceeds a predetermined threshold, the lancet body 151 is no longer secured to the lancet holder 102. More specifically, when the lancet holder 102 is forced to move further in the inserting direction, the securing boss 121a slotted in the securing groove 120 of the lancet holder 102 rides over the securing protrusion 158 of the lancet body 151 (when viewed differently, the securing protrusion 158 of the lancet body 151 rides over the securing boss 121a provided in the securing groove 120 of the lancet holder 102), and thereby the contact between the securing bosses 121a, 121b and the securing protrusion 158 ceases, which results in the pricking-enabled state. In the pricking-enabled state, the pair of guided protrusions 157a and the pair of guided protrusions 157b of the lancet body 151 are guided along the guide channels 107 of the lancet holder 102, so that the lancet body 151 with the tip of the pricking component 153 exposed can move in the pricking direction or in the opposite direction along the guide channels 107.

Upon inserting the lancet holder 102 into the injector 200 to the point where the lancet body 151 can move in the pricking direction and in the opposite direction, the lancet cap 152 is pressed by the lancet cap removing part 206 so that the lancet cap is separated from the lancet body 151 and finally the separated lancet cap reaches the position off the pricking pathway. Fig. 20B shows that the separated lancet cap 152 has moved to the position that is off the pricking pathway.

Upon loading the lancet assembly, the lancet cap removing part 206 enters the lancet holder 102 so that the pair of second wing parts 159 is accommodated in the bow-shaped groove 206d of the lancet cap removing part 206. The second wing parts 159 lie in the bow-shaped groove 206d without being in a deformed state.

The Loading of the lancet assembly by inserting the lancet holder 102 into the injector 200 is completed through the following steps (i) to (ix):
(i) The locking protrusion of the lancet holder rides over the stopper A of the holder locking component;
(ii) The rear portion of the guided protrusion of the lancet body outwardly expands the free end of the plate spring component;
(iii) The lancet cap is separated from the lancet body, so that the tip of the pricking component is exposed whereas the pricking component remains situated in the lancet body;
(iv) The separated lancet body moves to the position that is off the pricking pathway;
(v) The plunger is retracted;
(vi) The flange of the retracted plunger and the trigger lever becomes in a cocked state;
(vii) The locking protrusion of the lancet holder rides over the stopper B of the holder locking component;
(viii) The indicator component moves to a position where the indicating portion can be seen through the indicator window from the outside; and
(ix) The lancet body is no longer secured to the lancet holder, and thus completing the loading operation.
   It should be noted that "steps (i) and (vii)", "step (ii)", "steps (iii) and (iv)", "steps (v) and (vi)" and "step (viii)" proceed substantially concurrently. It should be also noted that the steps (vi) and (vii) and
(viii) may take place substantially simultaneously.

The state after the completion of the loading is shown in Fig. 20A and Fig. 20B. In this state, the pricking device 400 of the present invention composed of the lancet assembly 100 and the injector 200 is ready for pricking. That is to say, the lancet body 151 (specifically the lancet body with the tip of the pricking component exposed) is ready to be launched. In this state, the locking protrusion of the lancet holder and the stopper B of the holder locking component can make contact with each other, and thereby the lancet holder is securely held in the injector.

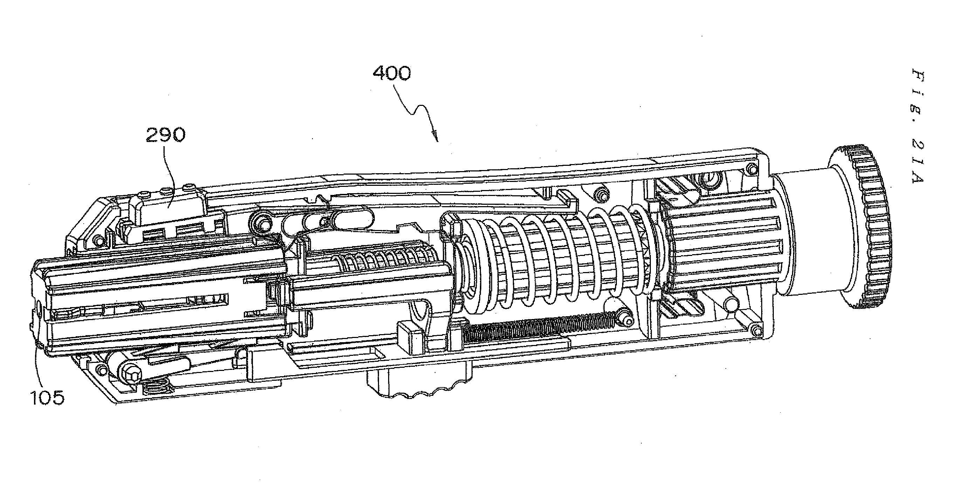

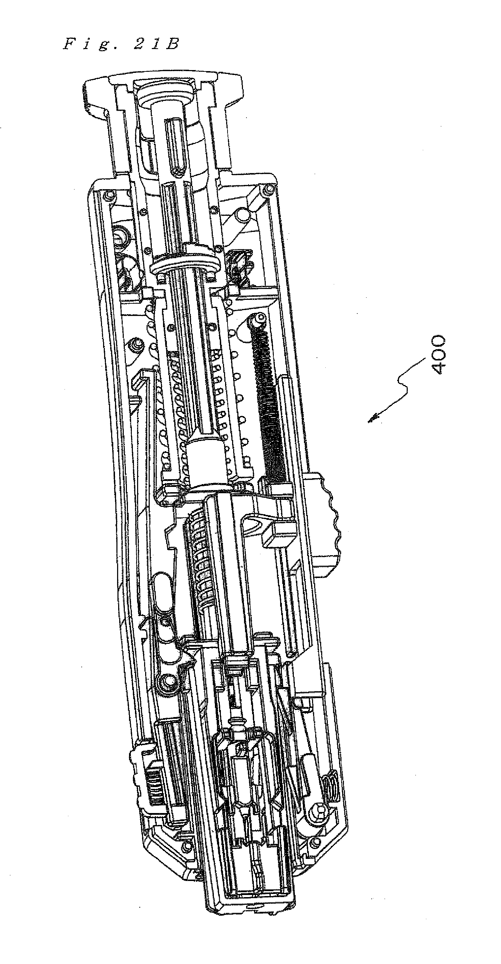

The pricking is performed as follows: the pricking opening 105 is applied to a predetermined region to be pricked (for example, a finger tip). Subsequently, the trigger locking component 290 is pressed to slide backward, and then the trigger locking component 290 is pressed toward the inside of the injector 200 so as to cease the engagement between the rear stepped portion 230a of the trigger lever and the flange 204b of the plunger 204. This results in an instantaneous expansion of the compressed spring 205a, and thereby the lancet body 151 is launched in the pricking direction. Fig. 21A and Fig. 21B show the state immediately after the front portion 230b of the trigger lever 230 is pressed toward the inside of the injector 200.

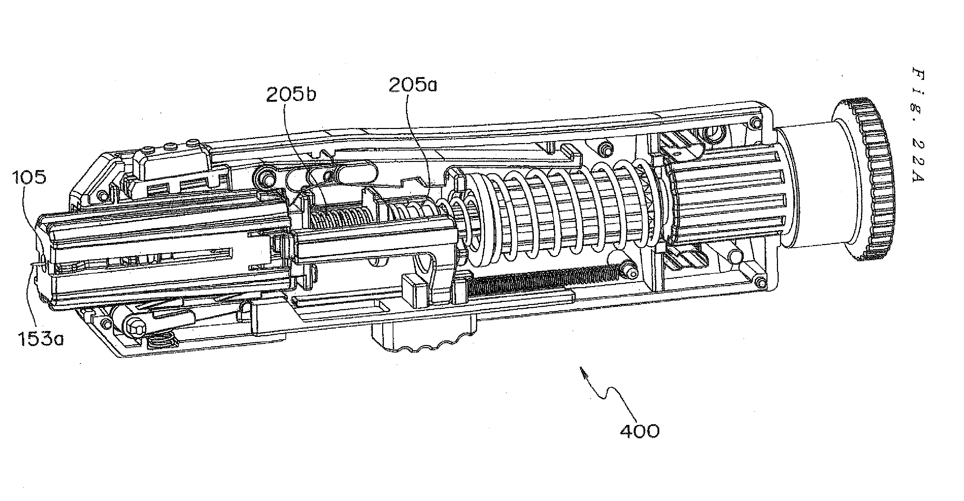

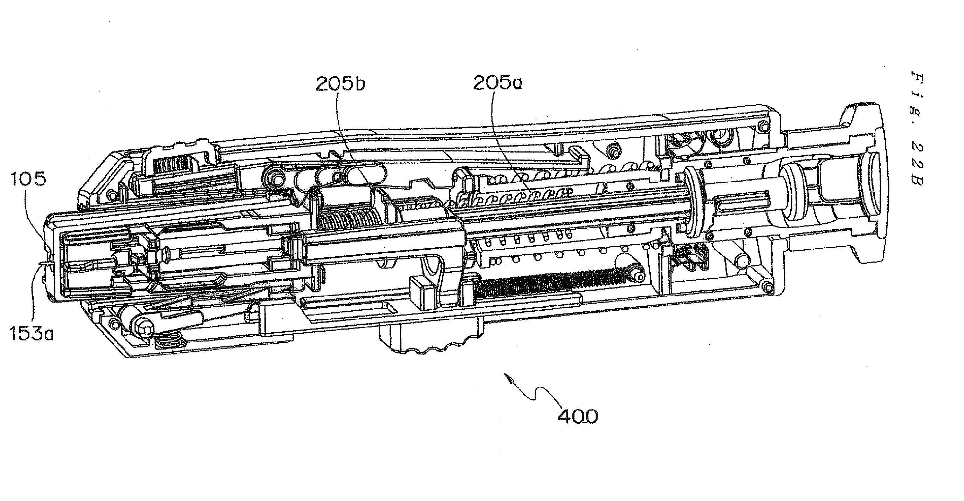

In the course of the launching of the lancet body 151, the pair of second wing parts 159, which has been accommodated in the bow-shaped groove 206d of the lancet cap removing part 206, moves forward along the bow-shaped groove 206d in the pricking direction. Due to the groove 206d, the pair of second wing parts 159 can move forward without being expanded toward the outside, which enables the pair of second wing parts 159 to move forward without touching or hitting the stopper surface 180. In other words, the lancet cap removing part 206, which has been inserted into the lancet holder 102, serves to block the stopper surface 180 (more particularly, the edge of the lancet cap removing part 206 is in contact with the stopper surface 180), and thereby the pair of second wing parts 159, upon pricking, can move forward without touching or hitting the stopper surface 180. Therefore, the lancet body 151 can move forward without being obstructed by the second wing parts 159. This means that the tip 153a of the pricking component can protrude from the pricking opening 105 to prick the predetermined region, as shown in Fig. 22A and Fig. 22B.

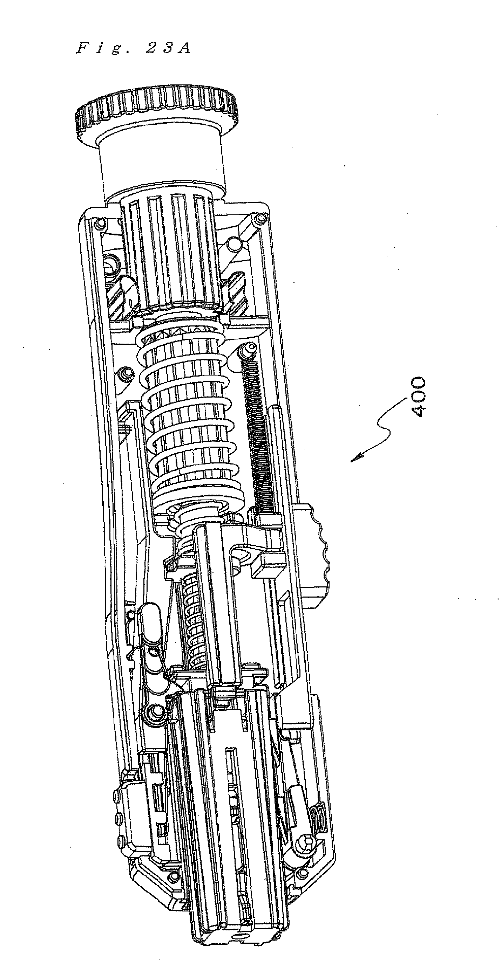

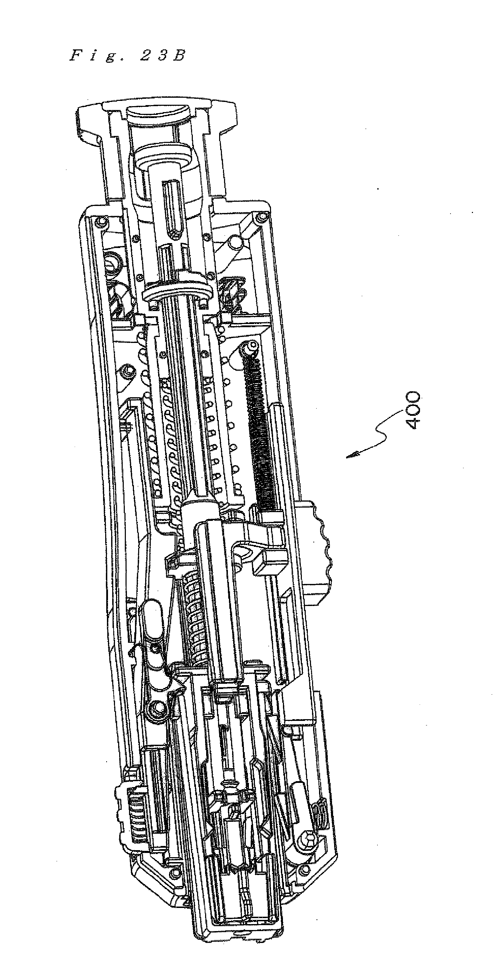

Upon pricking, the release spring 205a expands to the maximum whereas the return spring 205b is compressed, as shown in Fig. 22A. After pricking, therefore, the release spring 205a attempts to returns to its original shape whereas the compressed return spring 205b serves to press the flange 204b of the plunger 204 to returns to its original shape. This causes the lancet body 151 (hence the pricking component 153) to be retracted quickly. The state of the pricking device 400 after the pricking is shown in Fig. 23A and Fig. 23B.

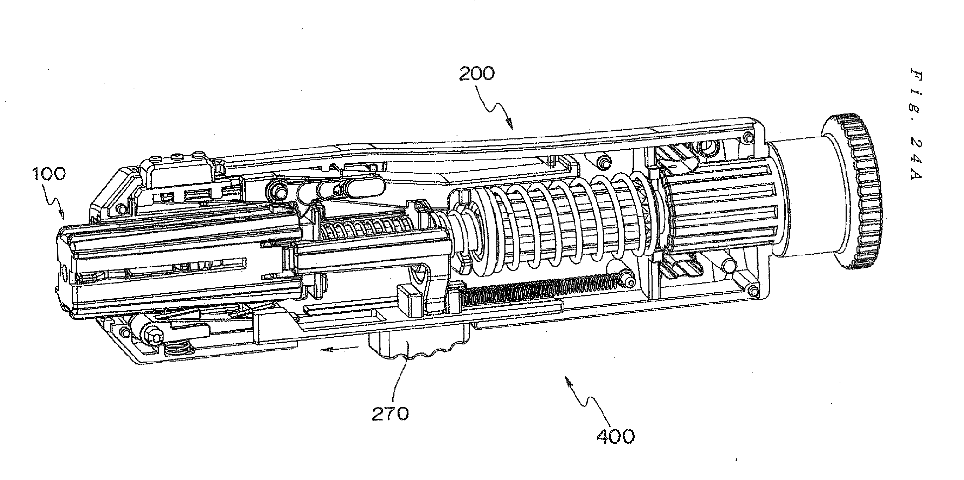

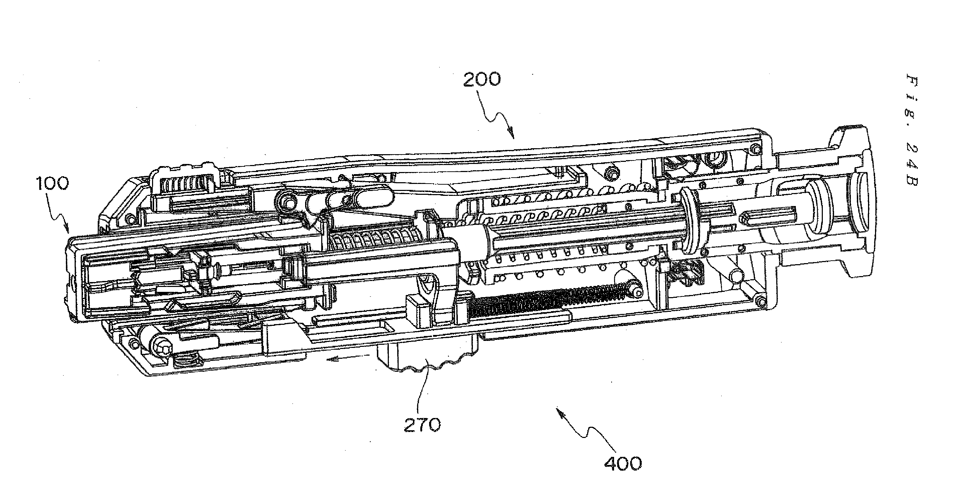

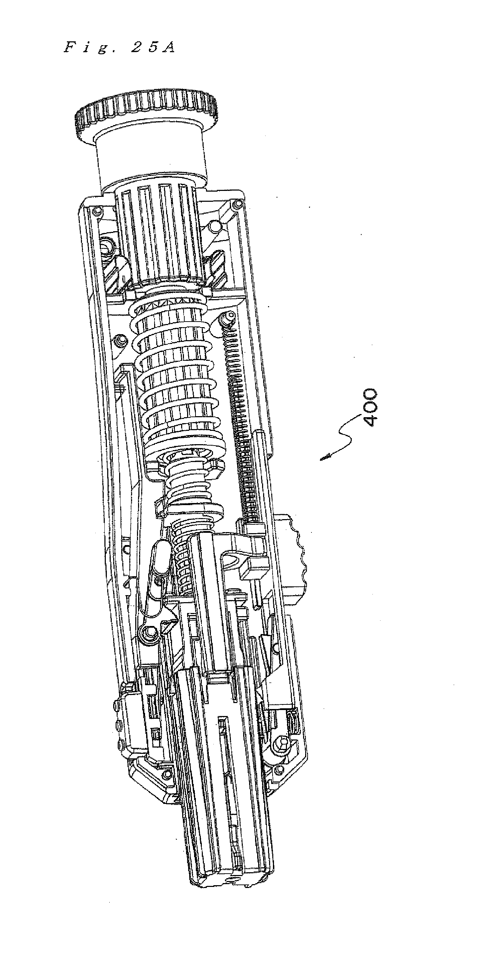

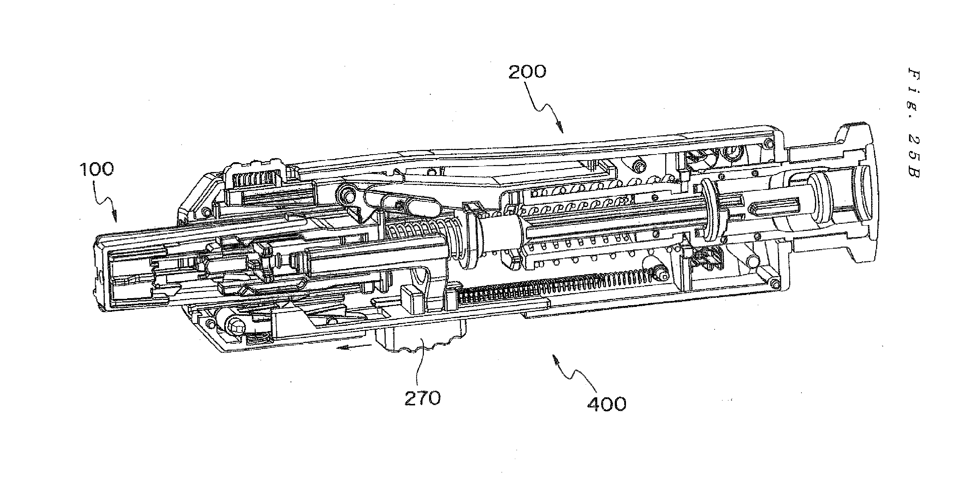

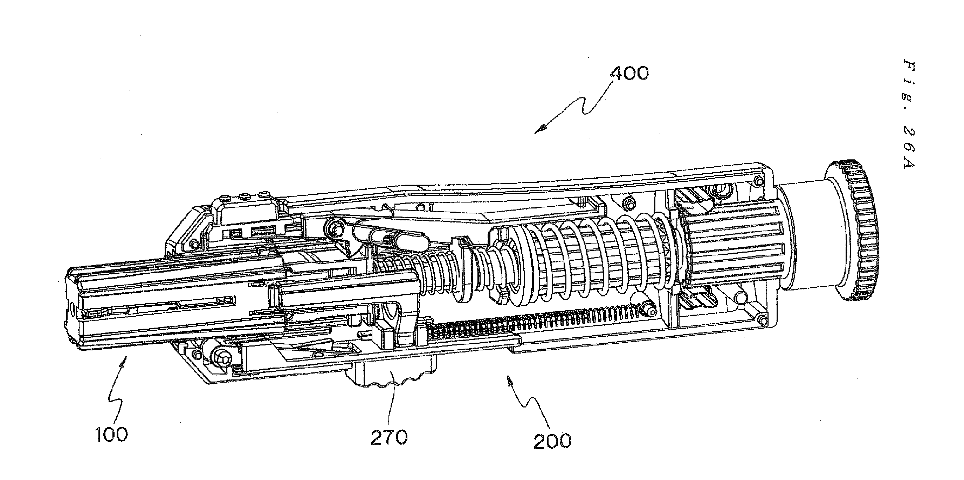

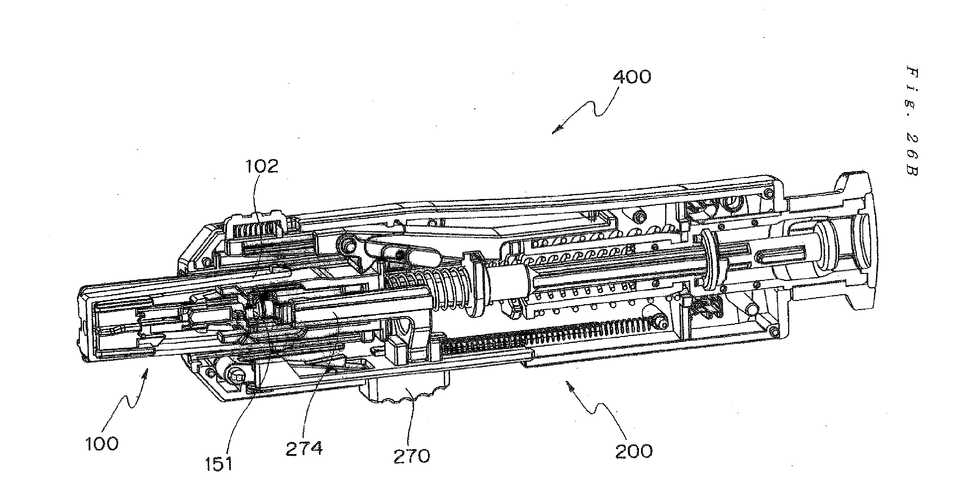

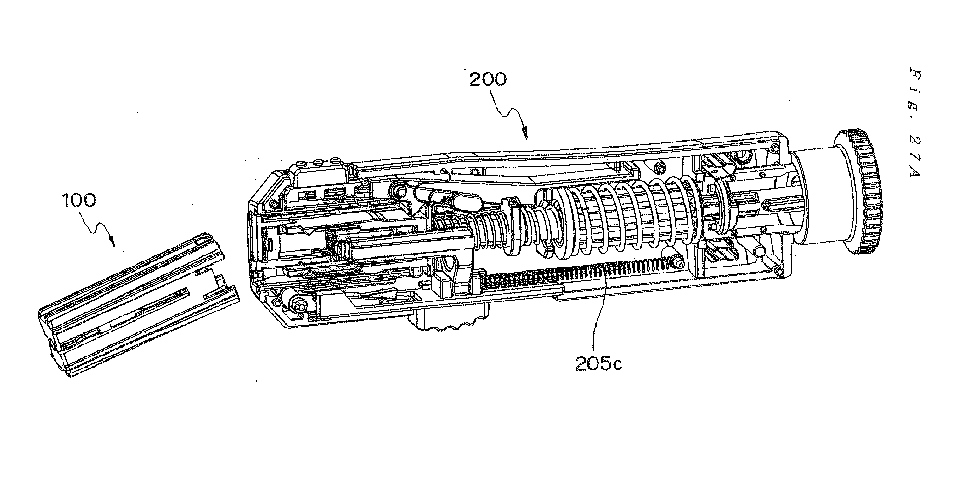

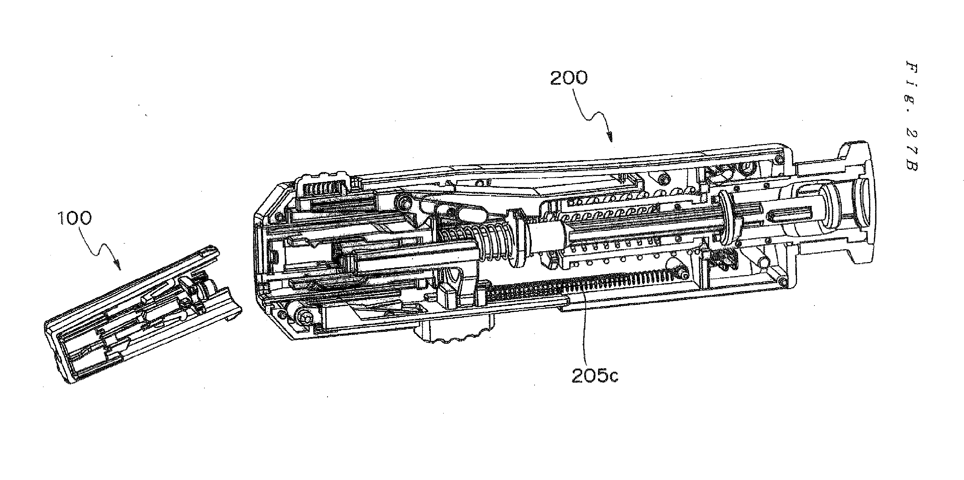

To remove (discharge) the lancet holder 102 (more particularly the spent lancet assembly 100) from the injector 200 after the pricking, the ejector 270 of the injector 200 is used. Specifically, a part 270a₁ of the main body 270a of the ejector 270 is pressed to slide with a finger or the like so that the pressing wing parts 274 of the ejector 270 pushes the edge of the opening end 103 of the lancet holder 102 and the rear end 165a of the lancet body 151, and thereby the spent lancet assembly 100 is discharged from the injector 200. The removing operation is shown sequentially in Figs. 24 to 27. Fig. 24A and Fig. 24B show the early state in which the lancet ejector 270 begins to slide. Fig. 25A and Fig. 25B show a mid stage of the sliding operation of the ejector 270. Fig. 26A and Fig. 26B show a latter stage of the sliding operation of the ejector 270. Fig. 27A and Fig. 27B show a state in which the lancet assembly 100 has been completely discharged from the injector 200.

Upon the discharge of the lancet assembly, the assembly pressing function and the holder lock releasing function work concurrently. Namely, the sliding of the ejector forward not only causes the pair of pressing wing parts 274 of the ejector 270 to push the lancet holder 102 and the lancet body 151 simultaneously, but also causes the releasing part 272 of the ejector main body 272a to cancel the contact-enabled state between the protrusion 116 of the lancet holder 102 and the stopper A 282 and/or the stopper B 284 of the holder locking component 280.

As will be understood by making reference to Fig. 27B, the ejector 270 has an ejector spring 205c. Therefore, after the discharging of the lancet assembly 100 is completed, the ejector 270 can be returned to its original position by releasing the finger from the ejector 270.

In the lancet holder 102 which has been removed, the separated lancet cap 152 and the lancet body 151 with the tip of the pricking component 153 exposed are contained. After the lancet holder 102 is discharged from the injector 200, the pair of second wing parts 159 is able to make contact with or hit the stopper surface 180 provided in the lancet holder 102 (see Fig. 5E). Therefore, the tip of the pricking component 153 will not protrude from the pricking opening 105 due to the restriction of the movement of the lancet body 153 in the pricking direction. As will be seen from Fig. 5E, the rear end face of the slope component 170 provided in the lancet holder 102 serves as the stopper surface 180 (most clearly shown in Fig. 1B). In this way, the protruding of the tip of the pricking component 153 from the pricking opening 105 is prevented, and thus the spent lancet assembly 100 can be safely disposed of.

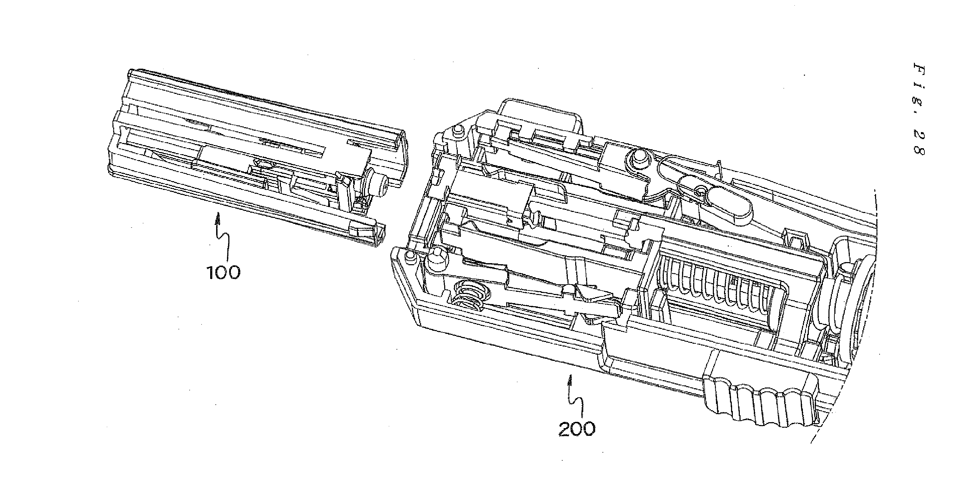

Now the recharging prevention function and the holder locking function, both of which are related to the feature of the present invention, will be described further with reference to Figs. 28 to 42. Figs. 28 to 42 show the time change of the lancet assembly 100 and the injector 200 (particularly the front portion of the injector) in the sequential order of the figure numbers. To make it easier to understand, the drawings show the state of the injector housing with the foreground removed.

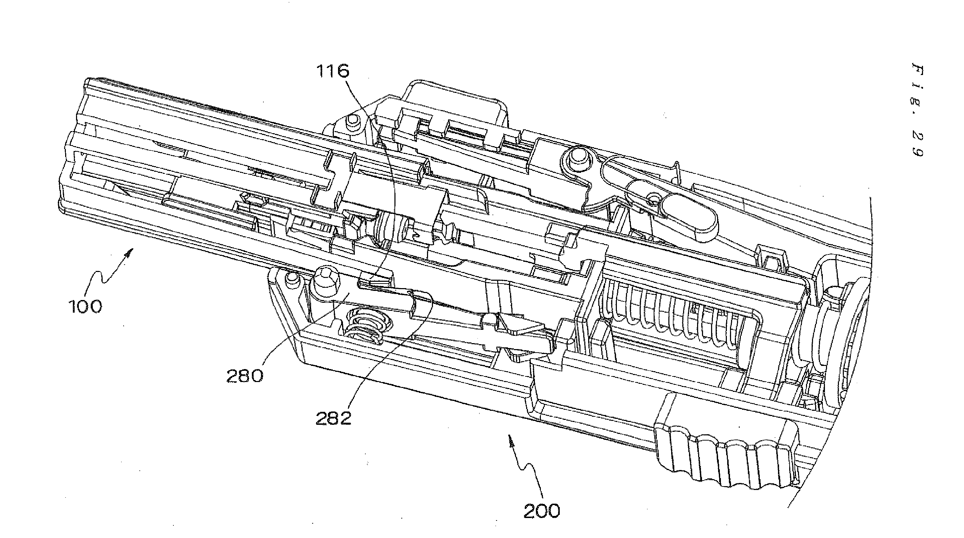

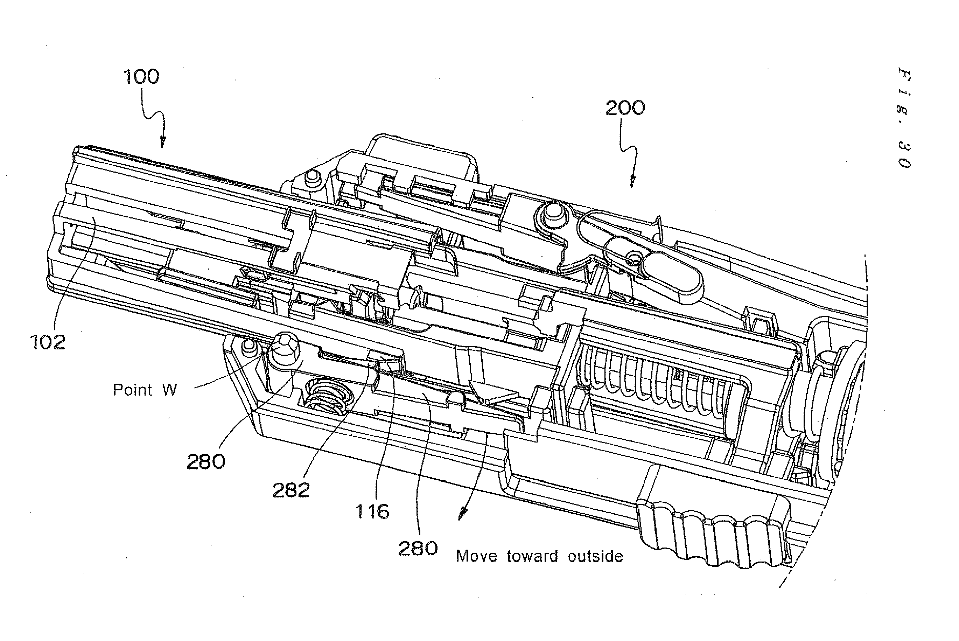

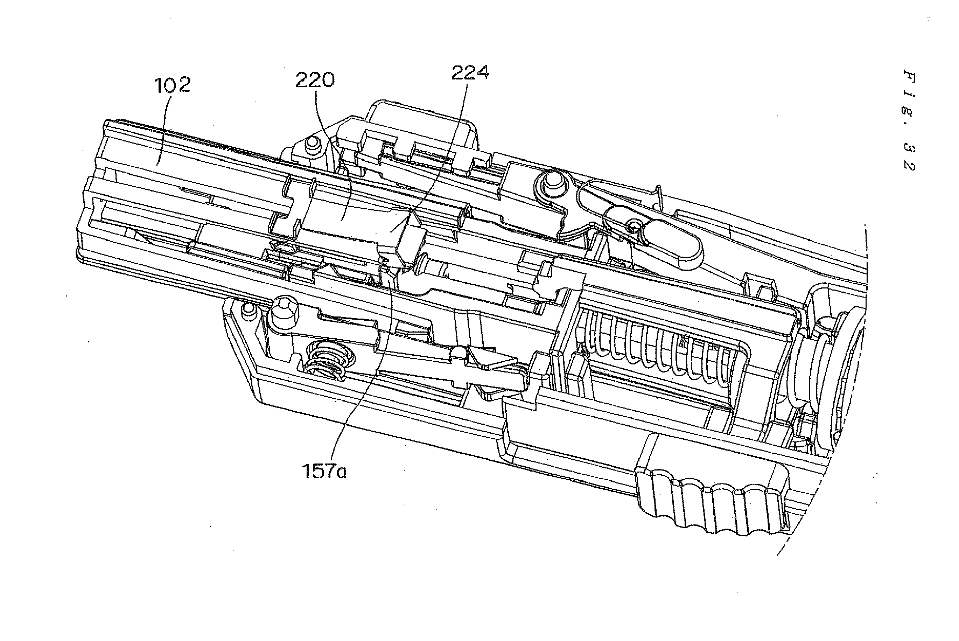

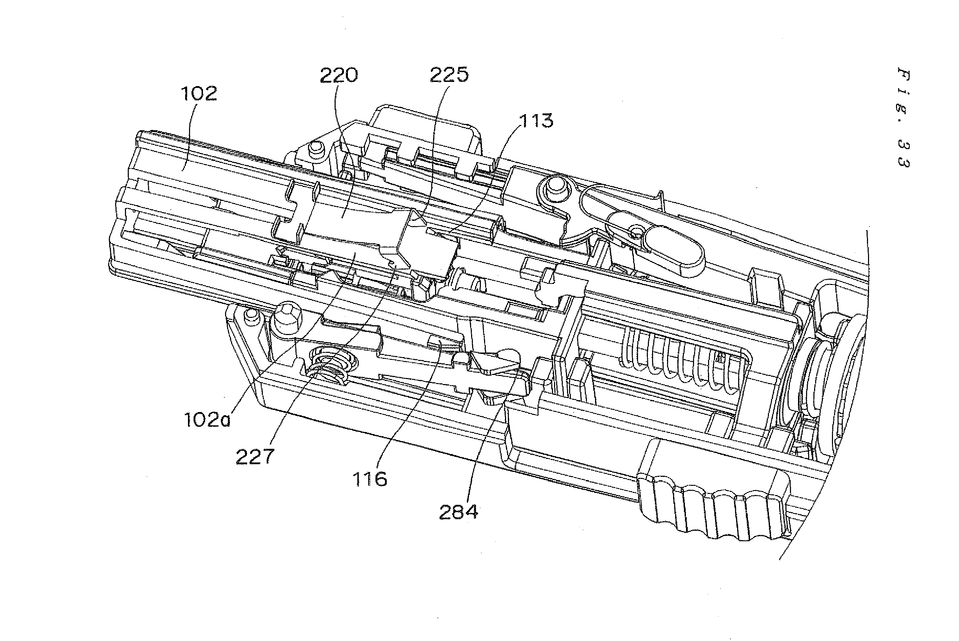

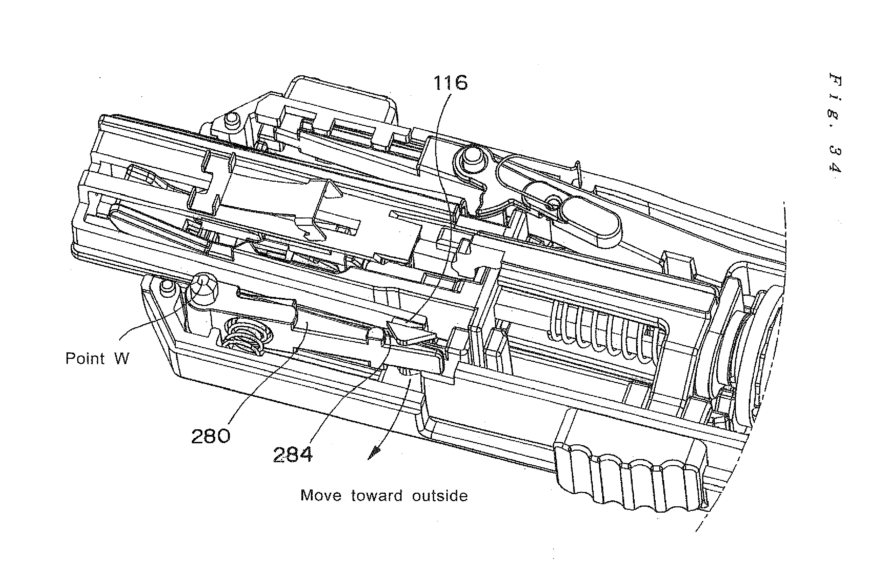

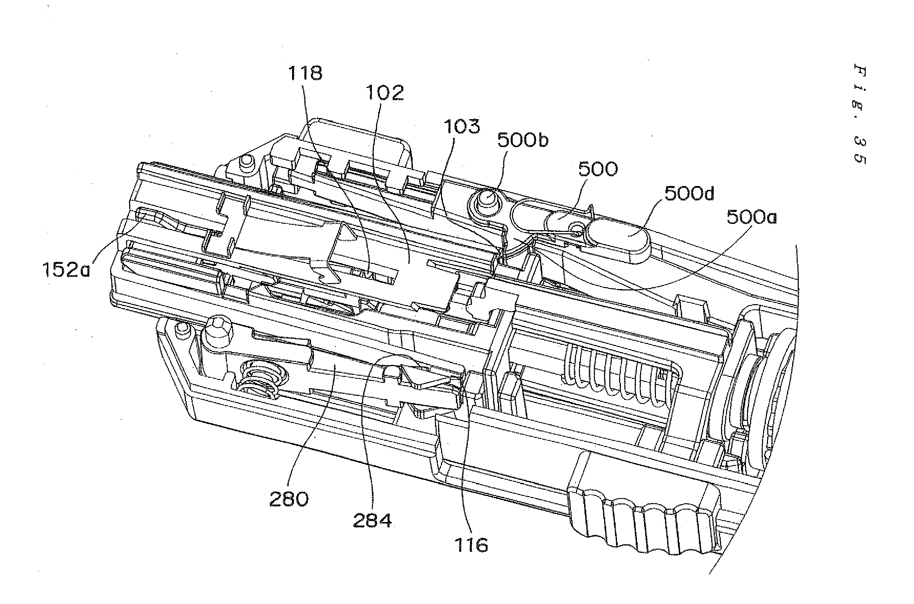

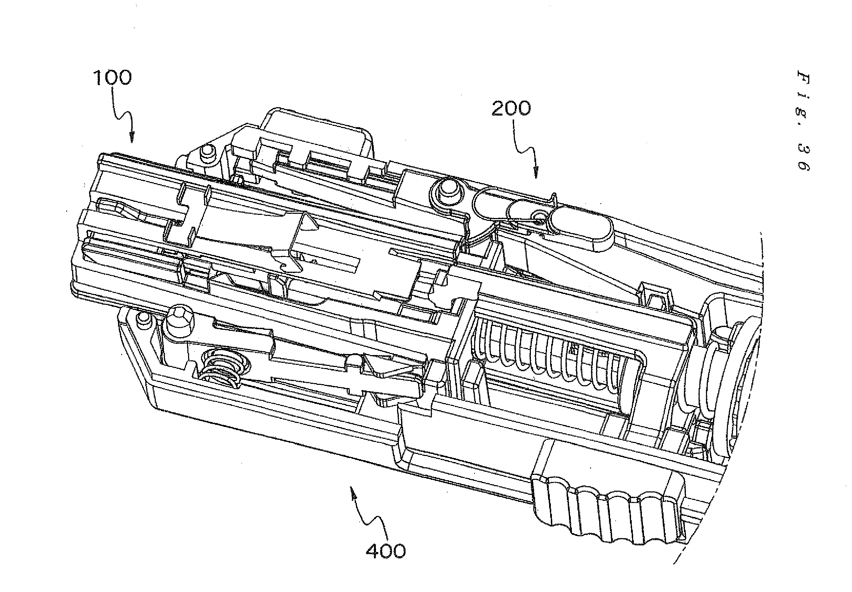

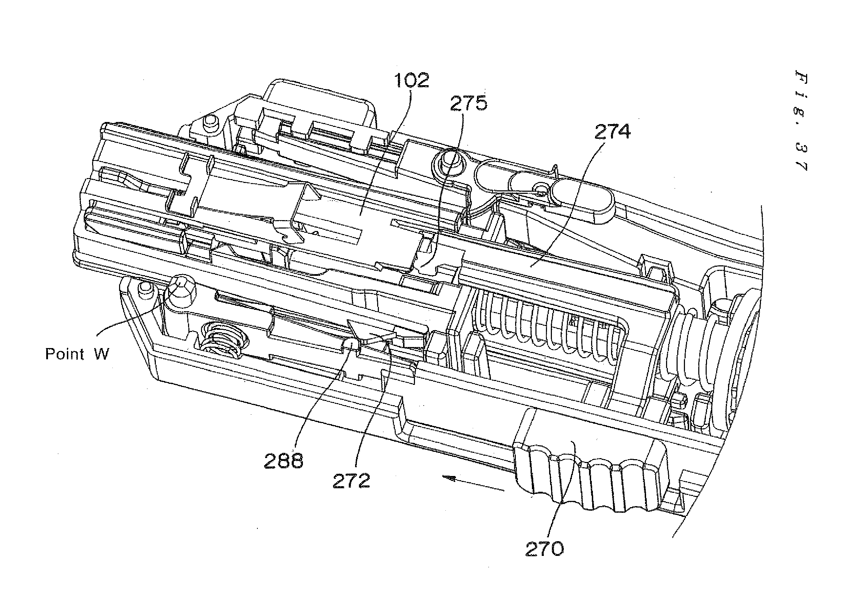

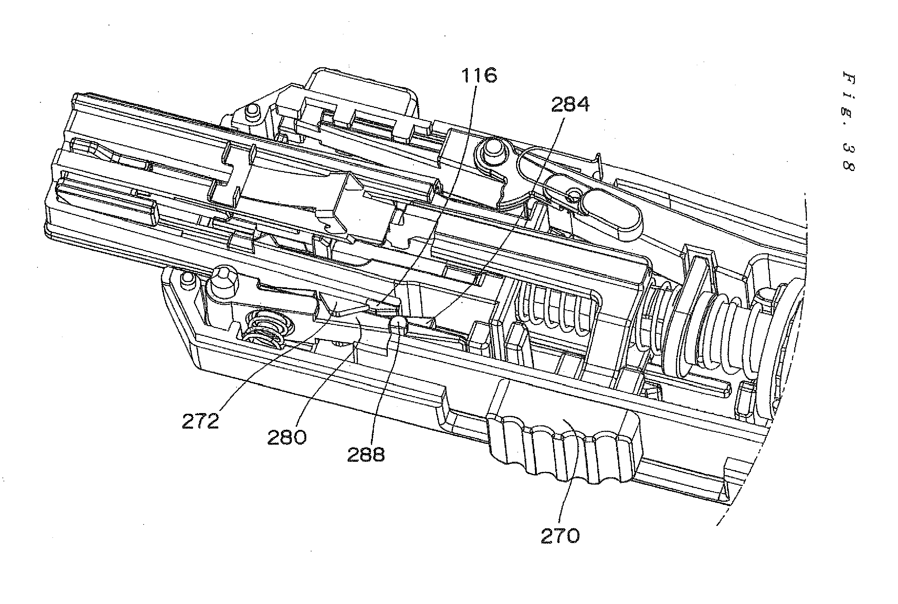

Fig. 28 shows a state before loading of the unspent lancet assembly 100 into the injector 200. Fig. 29 shows the early stage of the loading operation of the lancet assembly 100. As shown in Fig. 29, the locking protrusion 116 of the lancet holder is sliding on the holder locking component 280, and is particularly starting to slide on the stopper A 282. Fig. 30 shows that the holder locking component 280 is forced slightly outward with point W as the center, because the locking protrusion 116 of the lancet holder 102 is sliding on the holder locking component 280. Fig. 30 also shows a state immediately before the locking protrusion 116 rides over the stopper A 282. Fig. 31 shows the state where the rear portion 157a₁ of the guided protrusion 157a of the lancet body is in contact with the tip end portion 227 of the free end 224 of the plate spring component 220. Fig. 31 also shows the state after the locking protrusion 116 has ridden over the stopper A 282. After the locking protrusion 116 has ridden over the stopper A 282, the locking protrusion 116 and the stopper A 282 can make contact with each other and the holder locking component 280 that has been pressed outwardly returns to its original position. At this time point, the rear end 165a of the lancet body and the front end 204a of the plunger engage with each other so that the lancet and the plunger are integrated. In the state shown in Fig. 32, the guided protrusion 157a of the lancet body forces the free end 224 of the plate spring component 220 to deflect outwardly (toward the foreground of the drawing). The notched portion 113 of the lancet holder 102 and the hook-shaped portion 225 of the plate spring component 220 do not engage with each other because the plate spring component 220 deflects (see Fig. 33), and therefore it is made possible to insert the lancet assembly. Fig. 33 shows the state of the tip end portion 227 of the plate spring component 220 beginning to slide on the outer wall 102a of the lancet holder 102 as the lancet assembly (hence the lancet holder 102) is inserted. In Fig. 33, the locking protrusion 116 begins to ridden over the stopper B 284 (the stopper B is shown only partially in this drawing, and Fig. 38 best shows the stopper B 284). Fig. 34 shows the state immediately before the locking protrusion 116 has ridden over the stopper B 284 (the stopper B is shown only partially in this drawing), with the holder locking component 280 being forced slightly outward with point W as the center. Fig. 35 shows a state after the locking protrusion 116 has ridden over the stopper B. After the locking protrusion 116 has ridden over the stopper B, the locking protrusion 116 and the stopper B 284 can make contact with each other and the holder locking component 280 that has been pressed outwardly returns to its original position. Along with this movement, in the state shown in Fig. 35, the edge of the opening end 103 of the lancet holder 102 pushes the holder contact portion 500a of the indicator component 500, and thereby causing the indicator component 500 to move in a circular movement counterclockwise around the center located at the front end 500b, so that the indicating portion 500d provided on the side of the rear end moves to the position where it can be seen through the injector window 216. In the state shown in Fig. 35, the indicating part 152a of the lancet cap can be seen at the forward position of the slit 118 of the lancet holder so that the separation of the lancet cap from the lancet body can be confirmed. Fig. 36 shows the state after the pricking. Fig. 37 shows the state of the ejector 270 beginning to slide forward. In the state shown in Fig. 37, as the ejector 270 is pressed to slide forward, the lancet holder 102 and the lancet body are pushed by the tip 275 of the pressing wing parts 274 of the ejector 270, and thereby they start to move forward. While pressing in this way, the releasing part 272 of the ejector 270 begins to slide on the release part B 288, so that the holder locking component 280 is forced to move slightly outward with the point W as the center, and thereby avoiding the contact between the locking protrusion 116 and the stopper B 288.

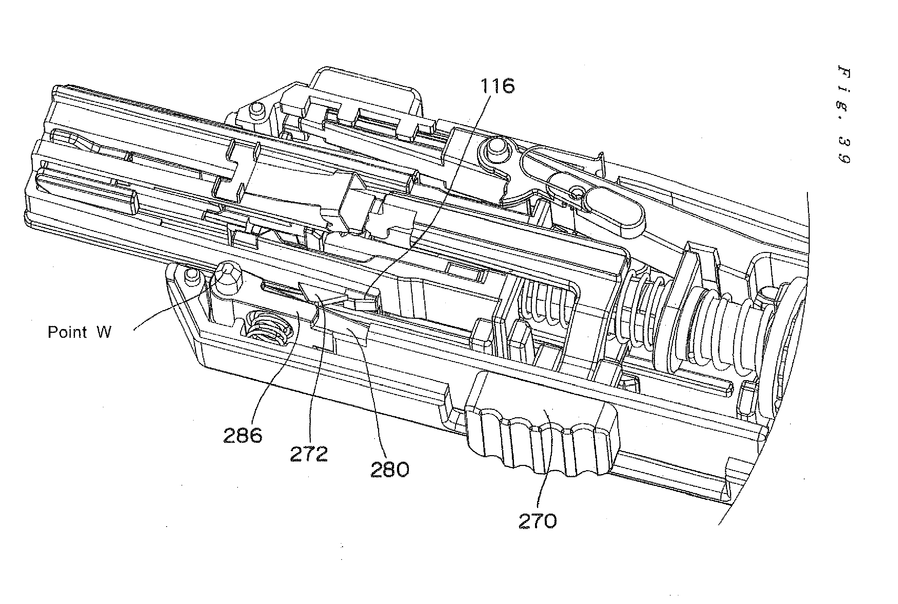

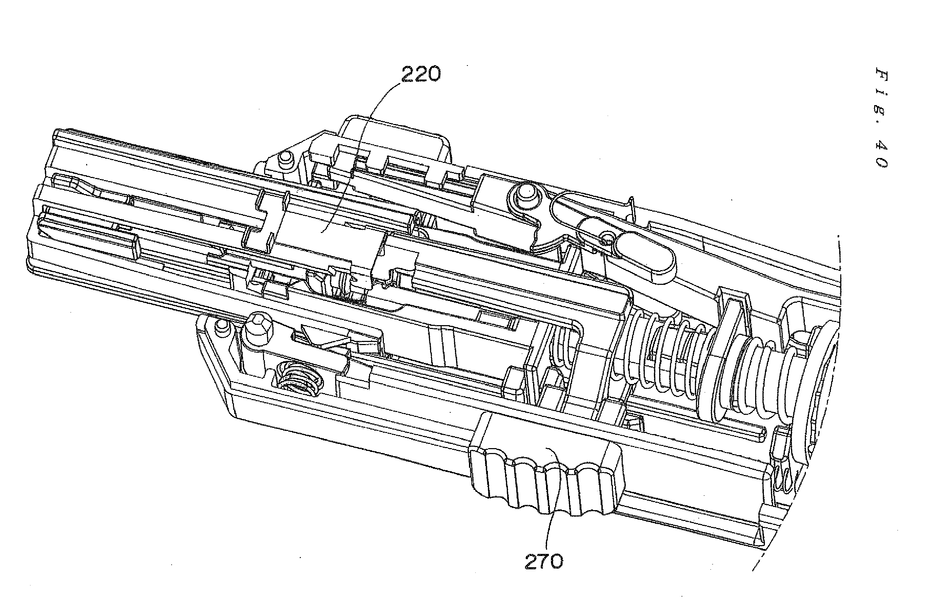

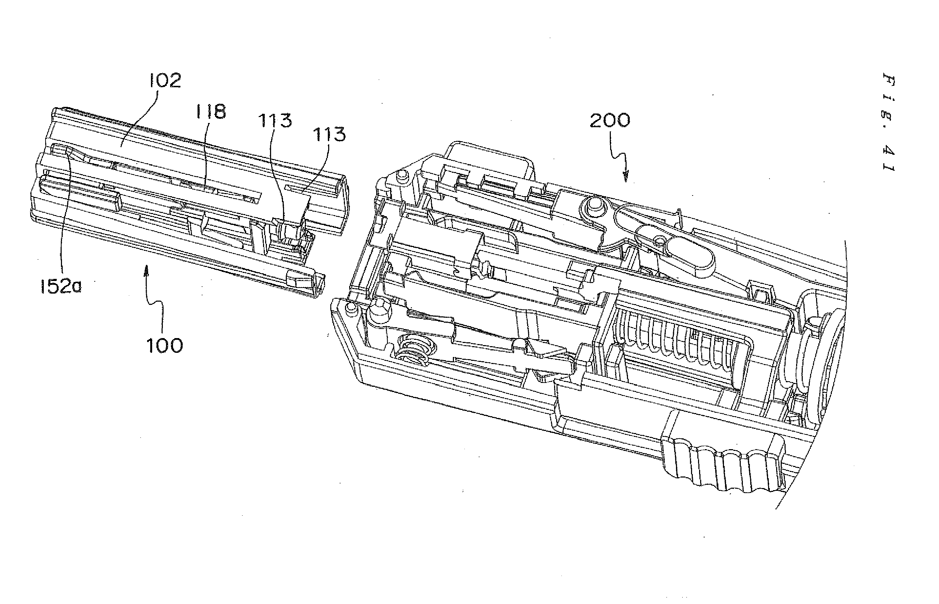

Fig. 38 shows the state of the ejector 270 sliding further forward. The releasing part 272 of the ejector 270 has ridden over the release part B 288, and thus the holder locking component 280 that has been pressed outwardly has returned to its original position. Fig. 39 shows the state of the ejector 270 sliding further forward. The releasing part 272 of the ejector 270 begins to slide on the release part A 286, so that the holder locking component 280 is forced to move slightly outward with the point W as the center, and thereby avoiding the contact between the locking protrusion 116 and the stopper A. Fig. 40 shows the state of the ejector 270 sliding further forward. Fig. 40 especially shows that the plate spring component 220 that has been deflected outwardly (toward foreground in the drawing) returns to its original state. Fig. 41 shows the state of the spent lancet assembly 100 being completely discharged from the injector 200. As shown in Fig. 41, the indicating part 152a that can be seen through the slit 118 of the lancet holder 102 is located at the forward position, thus giving visual indication that the discharged lancet assembly has been once used.

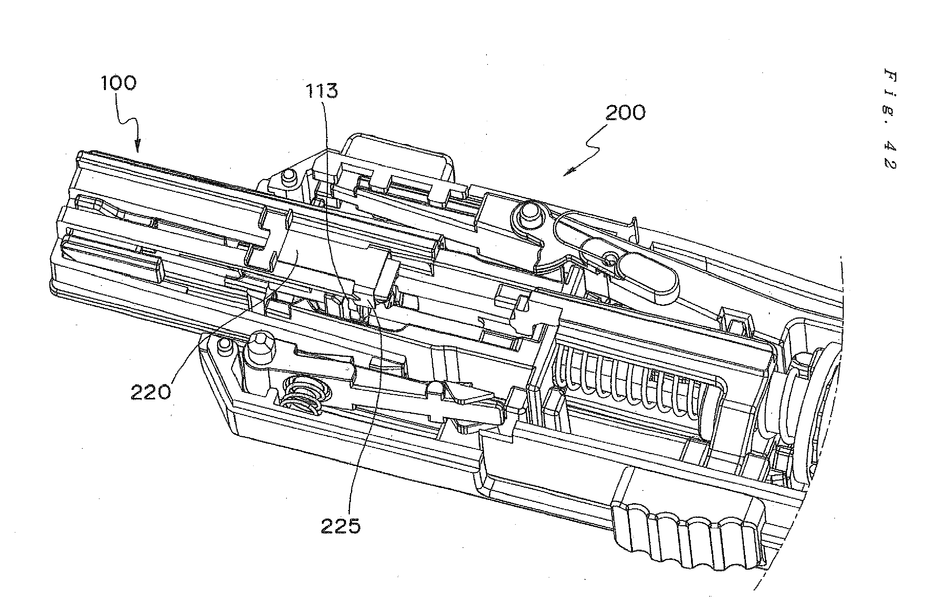

Fig. 42 shows the state in which it is attempted to insert the spent lancet assembly 100 into the injector 200. As illustrated, when it is attempted to insert the spent lancet assembly 100, the notched portion 113 of the lancet holder 102 (the notched portion is not clearly shown in Fig. 42, but is best shown in Fig. 41) engages with the hook-shaped portion 225 of the plate spring component 220 of the injector 200, and therefore the lancet assembly 100 cannot be inserted further.

Although the present invention have been described above, those skilled in the art will understand that the present invention is not limited to the above, and various modifications may be made.

### INDUSTRIAL APPLICABILITY

The pricking device composed of the lancet assembly and the injector described above makes it easier, safer and more hygienic to take a sample of the blood. The pricking device is not limited to taking the blood sample of diabetic, and can be used in various applications requiring blood samples.

### CROSS REFERENCE TO RELATED PATENT APPLICATION

The present application claims the rights of priority of Japanese Patent Application No.2006-163499 (filed on June 13, 2006, the title of the invention: "LANCET ASSEMBLY AND PRICKING DEVICE"), the disclosures of which are all incorporated herein by reference.

## Claims

1. A pricking device comprising a lancet assembly and an injector;
wherein the lancet assembly comprising a lancet and a lancet holder that houses the lancet, wherein the lancet comprises a lancet body, a lancet cap and a pricking component in which the lancet body and the lancet cap are made of resin and the pricking component is made of metal, the pricking component is situated in both of the lancet body and the lancet cap, the tip of the pricking component is covered with the lancet cap, and the lancet cap and the lancet body are integrally connected together by a bridging component;
wherein the injector used in combination with the lancet assembly is capable of launching the pricking component, the injector comprising a plunger that is capable of engaging with the rear end portion of the lancet body, and thus capable of launching the lancet body in the pricking direction; a lancet cap removing part that is capable of making contact with the lancet cap; and a trigger lever that is pressed to launch the pricking component, wherein the lancet assembly can be loaded into the injector by inserting the lancet holder into the injector through a front end opening of the injector;
wherein the lancet holder has a notched portion formed on an edge of the opening end thereof, whereas a plate spring component made of metal is provided on the inner wall of an injector housing at the front side of the injector,
the one end of the plate spring component is attached to the inner wall of the injector housing, whereas the other end of the plate spring component is a free end with a hook-shaped portion formed in the tip region thereof, and
when the spent lancet assembly whose lancet body has been launched in the pricking direction is inserted through the front end opening of the injector, the notched portion of the lancet holder engages with the hook-shaped portion of the plate spring component, and thereby a further insertion of the lancet holder into the injector is prevented.

2. The pricking device according to claim 1,
wherein
a rear portion of the lancet body is provided with a protrusion that extends transversely;
when an unspent lancet assembly wherein the lancet body is secured to the lancet holder such that the protrusion of the lancet body is located rearward with respect to the notched portion of the lancet holder, is inserted through the front end opening of the injector, the protrusion of the lancet body outwardly presses and expands the free end of the plate spring component so that the notched portion of the lancet holder does not engage with the hook-shaped portion of the plate spring component, which enables the unspent lancet assembly to be inserted into the injector.

3. The pricking device according to claim 2,
wherein
a rear side of the protrusion of the lancet body is chamfered; and
the surface of the chamfered rear side is capable of making contact with the tip end portion of the free end of the plate spring component, and thereby the free end of the plate spring component can expand outwardly upon being pressed.

4. The pricking device according to any one of claims 1 to 3, wherein
the main body of the plate spring component extends parallel to the inner wall surface of the injector housing, whereas the hook-shaped portion of the plate spring component extends in the direction perpendicular to the inner wall surface of the injector housing.

5. The pricking device according to any one of claims 1 to 4, wherein
a pair of the plate spring components are provided on opposing inner walls of the injector housing, whereas a pair of the notched portions are provided on opposing edges of the opening end of the lancet holder.

6. The pricking device according to any one of claims 1 to 5, wherein
two hook-shaped portions are provided on opposing peripheral portions at a tip end region of the free end of the plate spring component, whereas two notched portions are formed in the edges of the opening end of the lancet holder such that the two notched portions correspond to the two hook-shaped portions.

7. The pricking device according to any one of claims 2 to 6, wherein
the lancet holder has, on its inner wall, a guide for guiding the lancet body along the pricking direction;
the protrusion of the lancet body is guided by the guide of the lancet holder upon the launching the lancet body in the pricking direction.

8. A pricking device comprising a lancet assembly and an injector;
wherein the lancet assembly comprising a lancet and a lancet holder that houses the lancet wherein the lancet holder has a locking protrusion on its outer wall surface such that it is located adjacent to an opening end thereof, the lancet comprises a lancet body, a lancet cap and a pricking component in which the lancet body and the lancet cap are made of resin and the pricking component is made of metal, the pricking component is situated in both of the lancet body and the lancet cap, the tip of the pricking component is covered with the lancet cap, and the lancet cap and the lancet body are integrally connected together by a bridging component;
wherein the injector used in combination with the lancet assembly is capable of launching the pricking component, wherein the injector comprises a plunger that is capable of engaging with the rear end portion of the lancet body, and thus capable of launching the lancet body with the pricking component in the pricking direction; a lancet cap removing part that is capable of making contact with the lancet cap; and a trigger lever that is pressed to launch the pricking component, a holder locking component provided on the inner wall of an injector housing at the front side of the injector, and provided with a stopper A which protrudes from a surface S thereof toward the inside of the injector, wherein the lancet assembly can be loaded into the injector by inserting the lancet holder into the injector through a front end opening of the injector;
wherein a front end of the holder locking component is pivoted on the inner wall surface of the injector housing whereas a rear end of the holder locking component is a free end;
a spring is provided between the holder locking component and a side wall surface of the injector housing so that a inward force toward the inside of the injector is exerted on the holder locking component;
when the lancet assembly is inserted through the front end opening of the injector, the locking protrusion of the lancet holder slides on the holder locking component and then the locking protrusion rides over the stopper A, and thereby the locking protrusion and the stopper A can make contact with each other so that the inserted lancet holder does not come off the injector.

9. The pricking device according to claim 8,
wherein
a stopper B protruding toward the inside of the injector is provided rearward with respect to the stopper A on the surface S of the holder locking component;
after the riding of the locking protrusion over the stopper A upon the further insertion of the lancet holder into the injector, the locking protrusion rides over the stopper B so that the lancet holder moves to a position where the lancet body is ready to be launched in the pricking direction, and thereby the locking protrusion and the stopper B can make contact with each other.

10. The pricking device according to claim 9,
wherein
the rear end portion of the lancet body has an engagement portion that is capable of engaging with a plunger provided in the injector;
the rear end portion of the lancet body and the front end portion of the plunger engage with each other when the lancet assembly is loaded into the injector through the front end opening of the injector, and thereby the plunger is retracted so that the force required for launching the pricking component is stored in the plunger; and
while the lancet assembly is inserted until the force required for launching the pricking component is stored in the plunger, the locking protrusion rides over the stopper B of the holder locking component and thereby the locking protrusion and the stopper B can make contact with each other.

11. The pricking device according to claim 9 or 10,
wherein
the locking protrusion of the lancet holder and the stopper A and/or the stopper B of the holder locking component respectively have flat surfaces through which the locking protrusion makes complementary contact with the stopper A and/or the stopper B upon the sliding of the locking protrusion on the stopper A and/or the stopper B.

12. The pricking device according to any one of claims 8 to 11, wherein
the injector further comprises an ejector;
the ejector presses the lancet holder and the lancet body forward when the ejector is caused to slide forward, and thereby the lancet assembly is ejected from the injector.

13. The pricking device according to claim 12,
wherein
the ejector has a pair of pressing wing parts extending forward;
each tip of the pressing wing parts makes contact with both of the edge of the opening end of the lancet holder and the rear end portion of the lancet body, and thereby both of the lancet holder and the lancet body are simultaneously pushed forward.

14. The pricking device according to claim 13,
wherein the tips of the pair of pressing wing parts are respectively bent inwardly.

15. The pricking device according to claim 14,
wherein the plunger of the injector has a groove on its side;
an edge portion of the pressing wing part of the ejector is guided along the groove when the ejector is caused to slide forward.

16. The pricking device according to any one of claims 9 to 15, wherein
the holder locking component further has a release part A and/or a release part B on its side;
the ejector further has a releasing part capable of making contact with the release part A and/or the release part B; and
when the ejector is caused to slide forward, the releasing part slides on the release part A and/or the release part B, causing the holder locking component to move outwardly while rotating around the front end thereof, and thereby a contact-enabled state between the locking protrusion of the lancet holder and the stopper A and/or B of the holder locking component ceases.

17. The pricking device according to claim 16,
wherein the release part A and the stopper A are located adjacent to each other in the holder locking component; and/or
the release part B and the stopper B are located adjacent to each other in the holder locking component.

18. The pricking device according to claim 16 or 17,
wherein
when the ejector is caused to slide forward, both of the lancet holder and the lancet body are simultaneously pushed forward and the contact-enabled state between the locking protrusion of the lancet holder and the stoppers A and/or B of the holder locking component ceases, and thereby the lancet assembly can be discharged from the injector.

19. The pricking device according to any one of claims 8 or 18, wherein
the injector further comprises an indicator component in which the front end of the indicator component is pivoted on the trigger lever whereas the rear end of the indicator component is provided with an indicating portion; and
when the lancet assembly is inserted to a position where the lancet body is ready to be launched in the pricking direction, the edge of the opening end of the lancet holder pushes a holder-contact region of the indicator component so that the indicator component swivels around the front end thereof, causing the indicating portion to move to a position where a window is formed in the injector housing.

20. The pricking device according to any one of claims 8 to 19, wherein
the injector has a trigger locking component provided at the front end of the trigger lever;
a part of the trigger locking component extends through an opening of the injector housing, and the trigger locking component is capable of sliding on the trigger lever forward or backward within the opening of the injector housing; and
the trigger locking component is provided with a spring for forcing the trigger locking component to move forward so that the trigger locking component is located at the front position of the opening of the injector housing, as long as the force is not applied to the trigger locking component, in which case a locking part provided at the bottom of the trigger locking component can make contact with a lock part provided on the inner wall of the injector housing so that the trigger locking component cannot be pressed into the injector, and thereby the trigger lever cannot be pressed into the injector;
in a case where the force is applied to the trigger locking component against the forward force, causing the trigger locking component to move backward within the opening of the injector housing, a contact-enabled state between the locking part of the trigger locking component and the lock part provided on the inner wall of the injector housing ceases, enabling the trigger locking component to be pressed into the injector, and thereby the trigger lever can be pressed into the injector.

21. A lancet assembly comprising a lancet and a lancet holder that houses the lancet, wherein
the lancet comprises a lancet body, a lancet cap and a pricking component in which the lancet body and the lancet cap are made of resin and the pricking component is made of metal, the pricking component is situated in both of the lancet body and the lancet cap, the tip of the pricking component is covered with the lancet cap, and the lancet cap and the lancet body are integrally connected together by a bridging component;
when the lancet cap is pressed in the pricking direction with the lancet body attached to the lancet holder, the bridging component is broken so that the lancet cap is separated from the lancet body, and thereby the tip of the pricking component is exposed while the pricking component remains situated in the lancet body;
when the separated lancet cap is pressed further in the pricking direction, the separated lancet cap moves to a position that is off the pricking pathway of the pricking component;
wherein the lancet holder has two securing bosses formed within a securing groove formed on the inner wall thereof whereas the lancet body has a securing protrusion that slots into the securing grooves;
the lancet body is secured to the lancet holder by locating one securing protrusion between the two securing bosses;
wherein the lancet holder has a guide on its inner wall, and the lancet body has a guided protrusion; and
when a contact between the bosses and the securing protrusion ceases by pressing the lancet body , the guided protrusion can move along the guide so that the lancet body is guided along the pricking direction.

22. The lancet assembly according to claim 21,
wherein
the securing protrusion extends in the transverse direction of the lancet body along flat surface A that includes the center axis of the lancet; and
the guided protrusion extends in the transverse direction of the lancet body along flat surface B that is parallel to the flat surface A.

23. The lancet assembly according to claim 21 or 22,
wherein
the lancet cap is capable of making contact with a lancet cap removing part that is provided in an injector for launching the pricking component;
the lancet cap removing part makes contact with the lancet cap when the lancet holder with the lancet body secured thereto is inserted into the injector;
when the lancet holder is further inserted while the lancet cap removing part and the lancet cap are still in contact with each other, the force for pressing the lancet cap in the pricking direction is generated so that the bridging component is broken and the lancet cap is separated from the lancet body, and thereby the tip of the pricking component is exposed while the pricking component remains situated in the lancet body; and,
when the lancet holder is furthermore inserted, the separated lancet cap moves to the position that is off the pricking pathway within the lancet holder.

24. The lancet assembly according to claim 23,
wherein
the lancet body has a pair of wing parts extending backward, each tip of the wing parts being provided with a contact portion;
in the spent lancet assembly that has been ejected from the injector after pricking, the contact portion of each wing part can make contact with a stopper surface provided within the lancet holder so that the tip of the pricking component does not protrude from the pricking opening.

25. The lancet assembly according to claim 23 or 24,
wherein
as to the two securing bosses of the lancet holder, a front side of boss A located forward with respect to the other boss forms a flat surface facing forward;
after the lancet cap is separated from the lancet body upon the loading of the lancet assembly into the injector, the plunger is prevented from being retracted further, so that the lancet body being in engagement with the plunger is prevented from being further inserted; and thereafter, when the lancet holder is forced to move further in the direction of insertion, the contact between the securing boss of the lancet holder and the securing protrusion ceases, and thereby the lancet body can move in the pricking direction; and
when the lancet body is ready to be launched, the securing protrusion of the lancet body can make contact with the front side of the boss A of the lancet holder, and thereby the lancet body does not come off the lancet holder.

26. The lancet assembly according to any one of claims 21 to 25, wherein
the lancet cap has an indicating part that protrudes in the transverse direction thereof, and the lancet holder has, in its wall, a slit extending in the longitudinal direction thereof;
when the separated lancet cap moves to a position that is off the pricking pathway within the lancet holder, the indicating part moves along the slit.

27. The lancet assembly according to claim 26,
wherein,
when the separated lancet cap moves to a position that is off the pricking pathway within the lancet holder, the securing protrusion of the lancet body moves along the slit of the lancet holder.
